# EUROPEAN PATENT SPECIFICATION

(11) **EP 3 156 004 B1**
(45) Date of publication and mention of the grant of the patent: **11.04.2018**
(21) Application number: 16196958.9
(22) Date of filing: 03.02.2012
(51) Int. Cl.: A61F 2/01, A61B 17/221

(54) **VASCULAR AND BODILY DUCT TREATMENT DEVICES**
GEFÄSS- UND KÖRPERKANALBEHANDLUNGSVORRICHTUNGEN
DISPOSITIFS DE TRAITEMENT DE VAISSEAU SANGUIN ET CORPOREL

(30) Priority: 04.02.2011 US 201113021364; 23.11.2011 US 201113303890
(43) Date of publication of application: 19.04.2017
(62) Divisional of application: 12742286.3
(73) Proprietor: Concentric Medical, Inc., Mountain View, CA 94041 (US)
(72) Inventor: Grandfield, Ryan M., Livermore, CA 94550 (US); Wilson, Scott D., Fremont, CA 94538 (US); Sanders, Elliot H., Palo Alto, CA 94303 (US)
(74) Representative: ZBM Patents - Zea, Barlocci & Markvardsen

(56) References cited:
- US-A1- 2001 021 873
- US-A1- 2007 198 028
- US-A1- 2007 260 266
- US-A1- 2011 009 950

## Description

### TECHNICAL FIELD

This application relates to devices and methods for treating the vasculature and other ducts within the body.

### BACKGROUND

Self-expanding prostheses, such as stents, covered stents, vascular grafts, flow diverters, and the like have been developed to treat ducts within the body. Many of the prostheses have been developed to treat blockages within the vasculature and also aneurysms that occur in the brain. What are needed are improved treatment methods and devices for treating the vasculature and other body ducts, such as, for example, aneurysms, stenoses, embolic obstructions, and the like.

### SUMMARY OF THE DISCLOSURE

In accordance with the invention, an embolic obstruction retrieval device according to claim 1 is provided. Various embodiments are set out in the dependent claims.

### BRIEF DESCRIPTION OF THE DRAWINGS

Alternative implementations of the present disclosure are described herein with reference to the drawings wherein:
Figure 1A illustrates a two-dimensional plane view of an example of an expandable member of a treatment device.
Figure 1B is an isometric view of the expandable member illustrated in Figure 1A
Figure 2 illustrates a distal wire segment that extends distally from an example of an expandable member.
Figure 3 illustrates the distal end of an expandable member having an atraumatic tip.
Figure 4A illustrates a two-dimensional plane view of an expandable member of an example of a treatment device.
Figure 4B is an enlarged view of the proximal-most segment of the expandable member illustrated in Figure 4A.
Figure 5 illustrates a distal end of an example of an expandable member.
Figure 6A illustrates a two-dimensional plane view of an example of an expandable member of a treatment device.
Figure 6B is an isometric view of the expandable member illustrated in Figure 6A.
Figure 7A illustrates a two-dimensional plane view of an expandable member of an example of a treatment device.
Figure 7B is an isometric view of the expandable member illustrated in Figure 7A.
Figure 7C illustrates a two-dimensional plane view of an example of an expandable member of a treatment device.
Figure 8 illustrates a two-dimensional plane view of an example of an expandable member of a treatment device.
Figure 9 illustrates an expandable member in an expanded position having a bulge or increased diameter portion.
Figure 10 illustrates a two-dimensional plane view of an example of an expandable member of a treatment device.
Figure 11A illustrates a two-dimensional plane view of an example of an expandable member of a treatment device.
Figure 11B is an isometric view of the expandable member illustrated in Figure 11A.
Figure 12 illustrates a two-dimensional plane view of an example of an expandable member of a treatment device.
Figures 13A through 13C illustrate an example of a method for retrieving an embolic obstruction.
Figure 14 illustrates a two-dimensional plane view of an example of an expandable member of a treatment device.
Figure 15 illustrates a two-dimensional plane view of yet another example of an expandable member of a treatment device.
Figure 16 illustrates an isometric view of another example of an expandable member having an internal wire segment.
Figure 17 illustrates an isometric view of an example of an expandable member having an external wire segment.
Figure 18 illustrates an isometric view of an example of an expandable member in yet another example having a distal emboli capture device.
Figure 19 illustrates a two-dimensional plane view of another example of an expandable member of a treatment device.
Figure 20 illustrates the expandable member of Figure 19 having a longitudinal slit.
Figure 21 illustrates the expandable member of Figure 19 having a spiral slit.
Figure 22 illustrates the expandable member of Figure 19 having a partial spiral slit.
Figure 23 illustrates a two-dimensional plane view of another example of an expandable member of a treatment device.
Figure 24A illustrates a two-dimensional plane view of another example of an expandable member of a treatment device.
Figure 24B is an isometric view of the expandable member illustrated in Figure 24A.
Figure 25 illustrates a manner in which the proximal extending wire segment of an expandable device is attached to a delivery wire in one embodiment.
Figure 26 illustrates a two-dimensional plane view of an example of an expandable member of a treatment device.
Figures 27A and 27B illustrate isometric side and top views, respectively, of the expandable member depicted in Figure 26.
Figures 28A and 28B illustrate a proximal wire segment and a distal wire segment, respectively, of an example of an expandable member.
Figure 29 is a graph representing a radial force curve of an example of an expandable member.
Figure 30 illustrates a two-dimensional plane view of an example of a clot retrieval device.
Figure 31 illustrates a two-dimensional plane view of an example of a clot retrieval device.
Figures 32A-C illustrate cell structures according to some of the implementations of Figure 31.
Figure 33A illustrates a two-dimensional plane view of an example of a clot retrieval device.
Figures 33B and 33C illustrate top and side isometric views of the device illustrated in Figure 33A.
Figure 34A illustrates a two-dimensional plane view of an example of a clot retrieval device.
Figures 34B and 34C illustrate top and side isometric views of the device illustrated in Figure 34A.
Figure 35A illustrates a two-dimensional plane view of an example of a clot retrieval device.
Figures 35B and 35C illustrate top and side isometric views of the device illustrated in Figure 35A.
Figure 36 illustrates a two-dimensional plane view of an example of a clot retrieval device.
Figure 37 illustrates a two-dimensional plane view of an example of a clot retrieval device.
Figure 38 illustrates a two-dimensional plane view of an example of a clot retrieval device.
Figure 39 illustrates a two-dimensional plane view of an example of a clot retrieval device.
Figure 40A illustrates a two-dimensional plane view of an example of a clot retrieval device.
Figure 40B illustrates a three-dimensional view of the clot retrieval device of Figure 40A.
Figure 41 illustrates a two-dimensional plane view of an example of a clot retrieval device.
Figure 42A illustrates a two-dimensional plane view of an example of a clot retrieval device.
Figure 42B illustrates an enlarged two-dimensional plane view of the proximal tapered end portion of the retriever device depicted in Figure 45A.
Figure 43 illustrates a two-dimensional plane view of a proximal-most cell structure according to some examples.
Figure 44 illustrates a two-dimensional plane view of a proximal-most cell structure according to some examples.
Figures 45A-C illustrate two-dimensional plane views of clot retrieval devices according to some examples.
Figure 46 illustrates a two-dimensional plane view of an example of a clot retrieval device.
Figure 47A illustrates a two-dimensional plane view of a distal end of clot retrieval devices according to some examples.
Figure 47B illustrates a three-dimensional view of the distal end depicted in Figure 47A.
Figure 48A illustrates a two-dimensional plane view of an example of a clot retrieval device.
Figure 48B illustrates a three-dimensional view of the clot retrieval device depicted in Figure 48A.
Figure 49 illustrates a two-dimensional plane view of an example of a clot retrieval device.
Figure 50 illustrates a two-dimensional plane view of distal segment of an example of a retrieval device.
Figures 51A-D illustrate two-dimensional plane views of retrieval devices according to some examples.
Figures 52A and 52B illustrate two-dimensional plane views of retrieval devices according to some examples.
Figure 53 illustrates a two-dimensional plane view of an example of a retrieval device.
Figure 54 illustrates a two-dimensional plane view of an example of a retrieval device.
Figure 55 illustrates a two-dimensional plane view of an example of a retrieval device.
Figures 56A and 56B illustrates a two-dimensional plane view of an example of a retrieval device.
Figure 57 illustrates a two-dimensional plane view of an example of a retrieval device.
Figure 58A illustrates a two-dimensional plane view of a retrieval device according to some implementations.
Figure 58B illustrates an enlarged view of cell structures depicted in Figure 58A.
Figure 59A illustrates a side view of an example of a joint member.
Figure 59B illustrates a cross-sectional view of the joint member depicted in Figure 59A.
Figures 60A and 60B show wire attachment configurations according to some implementations.

### DETAILED DESCRIPTION

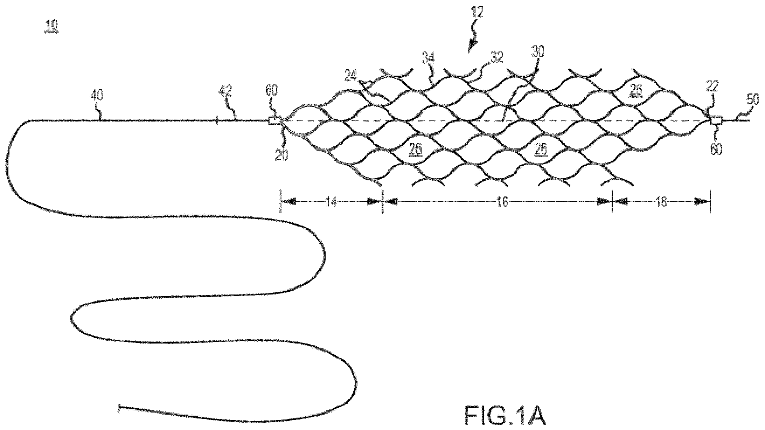

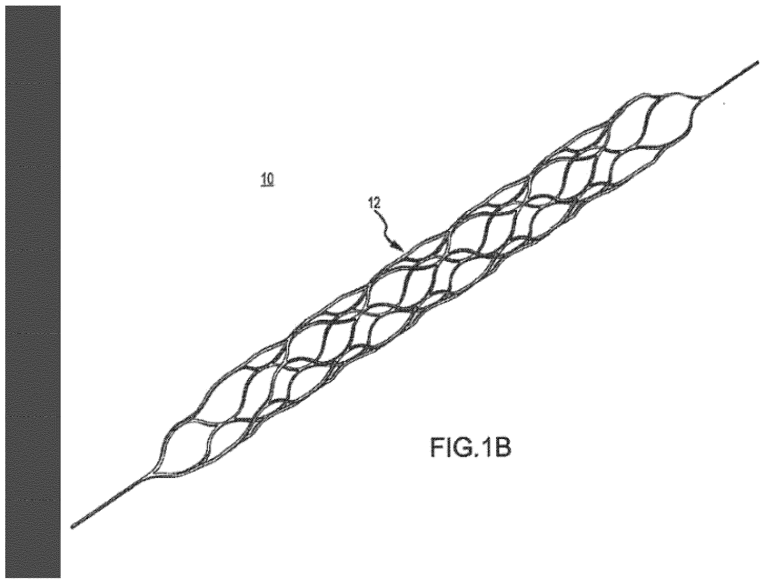

Figures 1A and 1B illustrate a vascular or bodily duct treatment device 10. Device 10 is particularly suited for accessing and treating the intracranial vascular of a patient, such as for example treating aneurysms or capturing and removing embolic obstructions, it is appreciated however, that device 10 may be used for accessing and treating other locations within the vasculature and also other bodily ducts. Other uses include, for example, treating stenoses and other types of vascular diseases and abnormalities. Figure 1A depicts device 10 in a two-dimensional plane view as if the device were cut and laid flat on a surface. Figure 1B depicts the device in its manufactured and/or expanded tubular configuration. Device 10 includes a self-expandable member 12 that is attached or otherwise coupled to an elongate flexible wire 40 that extends proximally from the expandable member 12. In one embodiment, the expandable member 12 is made of shape memory material, such as Nitinol, and is preferably laser cut from a tube. In one example, the expandable member 12 has an integrally formed proximally extending wire segment 42 that is used to join the elongate flexible wire 40 to the expandable member 12. In such an example, flexible wire 40 may be joined to wire segment 42 by the use of solder, a weld, an adhesive, or other known attachment method. In an alternative example, the distal end of flexible wire 40 is attached directly to a proximal end 20 of the expandable member 12. In one embodiment, the distal end of wire 40 has a flat, profile with a width of about 0.127 mm (0.005 inches) with the width and thickness of the wire segment 42 being about 0.16 mm (0.0063 inches) and about 0.0889 mm (0.0035 inches), respectively.

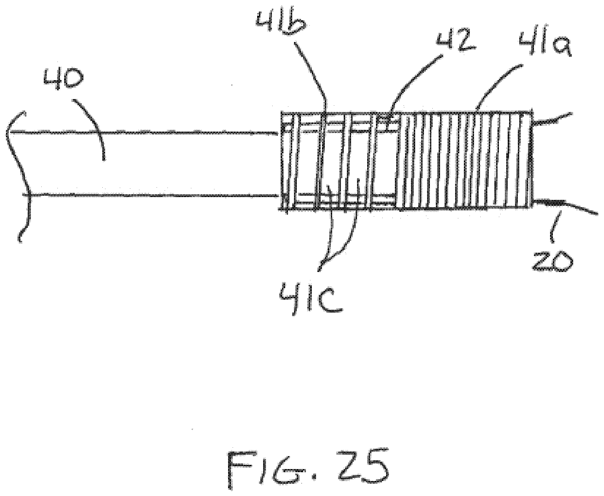

In one example, the distal end of wire 40 is attached to the proximally extending wire segment 42 by the following method, resulting in the joint illustrated in Figure 25. In one example, a coil 41 is positioned over wire segment 42, the coil having a closely wrapped segment 41a abutting the proximal end of expandable member 12, and a loosely wrapped segment 41b that includes one or more gaps 41c. The size of the one or more gaps 41c being sufficient to introduce a bonding agent into at least the inner cavity of coil segment, 41b. In one example, the length of wire segment 42 and the coil 41 are equal. In one example the length of the wire segment 42 is 4,0 millimeters with the coil 41 being of equal length. Once the coil 41 has been placed over the wire segment 42, the distal end of wire 40 is placed within coil segment 41b so that it makes contact with and overlaps the proximal end portion of wire segment 42. A bonding agent is then applied through the gaps 41c of coil 41 to bond the wire 40 with wire segment 41. The bonding agent may be an adhesive, solder, or any other suitable bonding agent. When the bonding agent is a solder, a preceding step in the process involves coating the distal end portion of wire 40 and the proximal end portion of wire segment 42 with tin or another suitable wetting agent. In one example the solder is gold and is used to enhance the radiopacity of the joint so that the joint may serve as a proximal radiopaque marker, in addition to the use of gold, all or portions of the coil may be made of a radiopaque material to further enhance the radiopacity of the joint. According to one example, the length of overlap between the wire 40 and wire segment 42 is between 0.75 and 1.0 millimeters. In the same implementation or in other implementations, the length of coil segment 41b is equal, or substantially equal, to the overlap length of the wire 40 and wire segment 42. In an alternative example, in lieu of the use of a single coil 41, two or more coils in abutting relationship are used with, for example, a first closely wound coil abutting the proximal end 20 of the expandable member 12 and a second loosely wound coil with gaps situated proximal to the closely wound coil. Although not shown in the figures, in one example a distal end length of wire 40 is tapers in the distal direction from a nominal diameter to a reduced profile. Along this length is provided a distal wire coil of a constant outer diameter with no taper. In accordance with one implementation, the diameter of coil 41 has the same outer diameter as the distal wire coil.

One advantage of the joint, construction is that it is resistant to buckling while the device is being pushed through a delivery catheter while at the same time being sufficiently flexible to enable the device to be delivered through the tortuous anatomy of a patient. In addition, the joint is able to withstand high tensile and torque loads without breaking. Load test have shown the joint of the previously described embodiment can withstand in excess of 2 pounds of tensile stress. In one embodiment, coil 41 is made of a radiopaque material to also function as a proximal radiopaque marker.

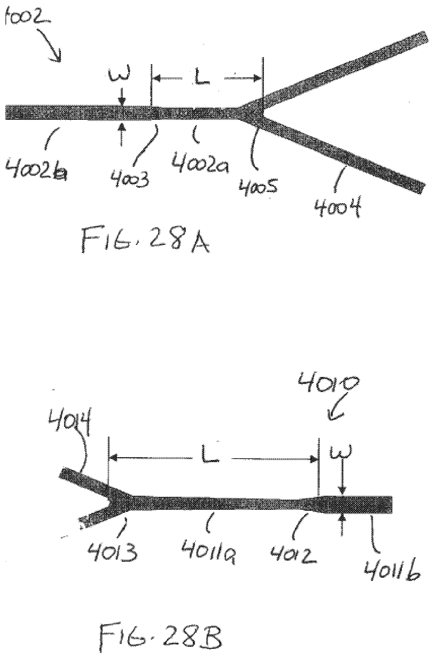

Figure 28A depicts an alternative proximal wire segment construction. As shown, the proximal wire segment 4002 comprises a first section 4002a and a second section 4002b, with the second section 4002b having a width W greater than the width of the first section. In one implementation a tapered transition section 4003 joins the first and second sections 4002a and 4002b. In one implementation the width of the first section 4002a is about 0.16 mm (0.0063 inches) while the width W of the second section is between about 0.2159 mm (0.0085 inches) and about 0.2677 mm (0.0105 inches). In one implementation the length L between the proximal end 4005 of the expandable member 4004 and second section 4002b of the wire segment 4002 is between about 0.4318 mm (0.017 inches) and about 0.5588 mm(0.022 inches). An advantage of the inclusion of the second section 4002b is that the greater width dimension provides a larger surface area for bonding the wire segment 4002 to the elongate wire 40 used in the delivery and retraction of the elongate member from a duct of a patient. In one implementation the first section 4002a has a circular or substantially circular construction and the second section 4002b has a flat profile formed by a pressing/coining operation.

In the example of Figures 1A and 1B, expandable member 12 includes a plurality of generally longitudinal undulating elements 24 with adjacent undulating elements being out-of-phase with one another and connected in a manner to form a plurality of diagonally disposed cell structures 26. The expandable member 12 includes a proximal end portion 14, a cylindrical main body portion 16 and a distal end portion 18 with the cell structures 26 in the main body portion 16 extending continuously and circumferentially around a longitudinal axis 30 of the expandable member 12. The cell structures 26 in the proximal end portion 14 and distal end portion 18 extend less than circumferentially around the longitudinal axis 30 of the expandable member 12.

In one embodiment, expandable member 12 has an overall length of about 33.0 millimeters with the main body portion 16 measuring about 16.0 millimeters in length and the proximal and distal end portions 14 and 18 each measuring about 7.0 millimeters in length. In alternative embodiments, the length of the main body portion 16 is generally between about 2.5 to about 3.5 times greater than the length of the proximal and distal end portions 14 and 18.

In use, expandable member 12 is advanced through the tortuous vascular anatomy or bodily duct of a patient to a treatment site in an unexpanded or compressed state (not shown) of a first nominal diameter and is movable from the unexpanded state to a radially expanded state of a second nominal diameter greater than the first nominal diameter for deployment at the treatment site. In alternative examples the first nominal diameter (e.g., average diameter of main body portion 16) ranges between about 0.4318 mm (0.017 inches) to about 0.762 mm (0.030 inches), whereas the second nominal diameter (e.g., average diameter of main body portion 16) is between about 2.5 to about, 5.0 millimeters. In one implementation, the dimensional and material characteristics of the cell structures 26 residing in the main body portion 16 of the expandable material 12 are selected to produce sufficient radial force and contact interaction to cause the cell structures 26 to engage with an embolic obstruction residing in the vascular in a manner that permits partial or full removal of the embolic obstruction from the patient. In alternative examples the dimensional and material characteristics of the cell structures 26 in the main body portion 16 are selected to produce a radial force per unit length of between about 0.005 N/mm to about 0.050 N/mm, preferable between about 0.010 N/mm to about 0.050 N/mm, and more preferably between about 0.030 N/mm and about 0.050 N/mm. In one example, the diameter of the main body portion 16 in a fully expanded state is about 4.0 millimeters with the cell pattern, strut dimensions and material being selected to produce a radial force of between about 0.040 N/mm to about 0.050 N/mm when the diameter of the main body portion is reduced to between about 1.0 millimeters to about 1.5 millimeters. In the same or alternative example, the cell pattern, strut dimensions and material(s) are selected to produce a radial force of between about 0.010 N/mm to about 0.020 N/mm when the diameter of the main body portion is reduced to 3.0 millimeters.

In the examples of Figures 1A and 1B, each of the cell structures 26 are shown having the same dimensions with each cell structure including a pair of short struts 32 and a pair of long struts 34. In an exemplary embodiment, struts 32 have a length of between about 2.032 mm (0.080 inches) and about 2.54 mm (0.100 inches), struts 34 have a length of between about 3.302 mm (0.130 inches) and about 3.556 mm (0.140 inches), with each of struts 32 and 34 having an as-cut width and thickness of about 0.0762 mm (0.003 inches) and about 0.1143 mm (0.0045 inches), respectively, and a post-polishing width and thickness of between about 0.05588 mm (0.0022 inches) and about 0.09906 mm (0.0039 inches), respectively. An advantage of having a shut, thickness to width ratio of greater than one is that, it promotes integration of the strut into the embolic obstruction. In alternative embodiments, the post-polishing width and thickness dimensions varies between about 0.0508 mm (0.0020 inches) to about 0.889 mm (0.0035) and about, 0.0762 mm (0.0030 inches) to about 0.1016 mm (0,0040 inches), respectively, with the thickness to width ratio varying between about 1.0 to about 2.0, and preferably between about 1.25 to about 1.75.

In one embodiment, only the strut elements of the main body portion 16 have a thickness to width dimension ratio of greater than one. In another embodiment, only the strut elements of the main body portion 16 and distal end portion 18 have a thickness to width dimension ratio of greater than one. In another embodiment, only a portion of the strut, elements have a thickness to width dimension ratio of greater than one. In yet another embodiment, strut elements in different parts of the expandable member have different thickness to width dimension ratios, the ratios in each of the parts being greater than one. As an example, because the radial force exerted by the proximal end portion 14 and distal end portion 18 of the expandable member 12 may generally be less than the radial force exerted by the main body portion 16, the strut elements in the distal and/or proximal end portions can have a thickness to width ratio that is greater than the thickness to width ratio of the struts in the main body portion 16. An advantage of this construction is that the ability of the expandable member 12 to integrate into an embolic obstruction is made to be more uniform along the length of the expandable member.

In other embodiments, certain, or all of the strut elements have a tapered shape with the outer face of the strut having a width dimension less than the width dimension of the inner face of the strut. In other embodiments, the expandable member 12 may comprise strut elements having a generally rectangular cross-section and also strut elements having a tapered shape.

It is important to note that the present invention is not limited to expandable members 12 having uniform cell structures nor to any particular dimensional characteristics. As an example, in alternative embodiments the cell structures 26 in the proximal and/or distal end portions 14 and 18 are either larger or smaller in size than the cell structures 26 in the main body portion 16. In one embodiment, the cell structures 26 in the proximal and distal end portions 14 and 18 are sized larger than those in the main body portion 16 so that the radial forces exerted in the end portions 14 and 18 are lower than the radial forces exerted in the main body portion 16.

The radial strength along the length of the expandable member 12 may be varied in a variety of ways. One method is to vary the mass (e.g., width and/or thickness) of the struts along the length of the expandable member 12. Another method is to vary the size of the cell structures 26 along the length of the expandable member 12. The use of smaller cell structures will generally provide higher radial forces than those that are larger. Varying the radial force exerted along the length of the expandable member can be particularly advantageous for use in entrapping and retrieving embolic obstructions. For example, in one embodiment the radial force in the distal section of the main body portion 16 of the expandable member 12 in its expanded state is made to be greater than the radial force in the proximal section of the main body portion 16, Such a configuration promotes a larger radial expansion of the distal section of the main body portion 16 into the embolic obstruction as compared to the proximal section. Because the expandable member 12 is pulled proximally during the removal of the embolic obstruction from the patient, the aforementioned configuration will reduce the likelihood of particles dislodging from the embolic obstruction during its removal, in an alternative embodiment the radial force in the proximal section of the main body portion 16 of the expandable member 12 in its expanded state is made to be greater than the radial force in the distal section of the main body portion 16. In yet another embodiment, the main body portion 16 of the expandable member 12 includes a proximal section, a midsection and a distal section with the radial force in the proximal and distal sections being larger than the radial force in the midsection when the expandable member 12 is in an expanded state.

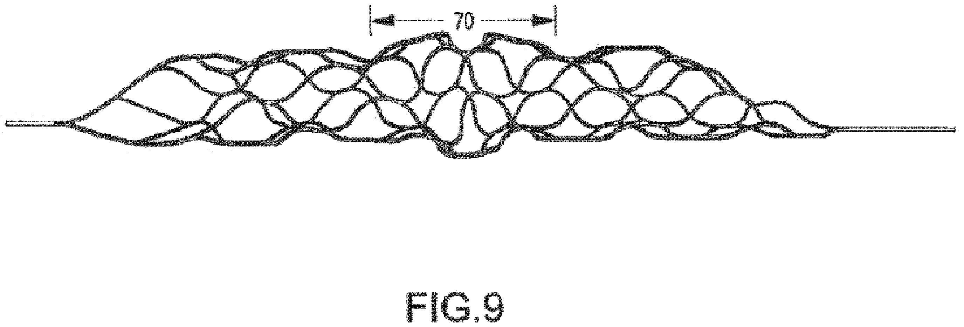

In alternative embodiments, as exemplified in Figure 9, the main body portion 16 may include an increased diameter portion or bulge 70 to enhance the expandable member's ability to entrap or otherwise engage with an embolic obstruction. In Figure 9, a single increased diameter portion 70 is provided within the midsection of main body portion 16. In alternative embodiments, the increased diameter portion 70 may be positioned proximally or distally to the midsection. In yet other embodiments, two or more increased diameter portions 70 may be provided along the length of the main body portion 16. In one implementation, the two or more increased diameter portions 70 have essentially the same manufactured nominal diameter. In another implementation, the distal-most increased diameter portion 70 has a greater manufactured nominal diameter than the proximally disposed increased diameter portions. In alternative exemplary embodiments the nominal diameter of the increased diameter portion 70 is between about 25.0 to about 45.0 percent greater than the nominal diameter of the main body portion. For example, in one embodiment, the nominal expanded diameter of main body portion 16 is about, 3.0 millimeters and the nominal diameter of the increased diameter portion 70 is about 4.0 millimeters. In another embodiment the nominal expanded diameter of main body portion 16 is about 3.50 millimeters and the nominal diameter of the increased diameter portion 70 is about 5.00 millimeters. In one embodiment, the one or more increased diameter portions 70 are formed by placing an expandable mandrel into the internal lumen of the main body portion 16 and expanding the mandrel to create the increased diameter portion 70 of a desired diameter. In another embodiment, one or more of the increased diameter portions 70 are formed by placing a mandrel of a given width and diameter into the main body portion 16 and then crimping the expandable member 12 in a manner to cause at least a portion of the main body portion 16 to be urged against the mandrel.

In one embodiment, the strut elements in the increased diameter portion or portions 70 have a thickness dimension to width dimension ratio that, is greater than the thickness to width ratio of the other struts in the main body portion 16. In yet another embodiment, the strut elements in the increased diameter portion or portions 70 have a thickness dimension to width dimension ratio that is less than the thickness to width ratio of the other struts in the main body portion 16.

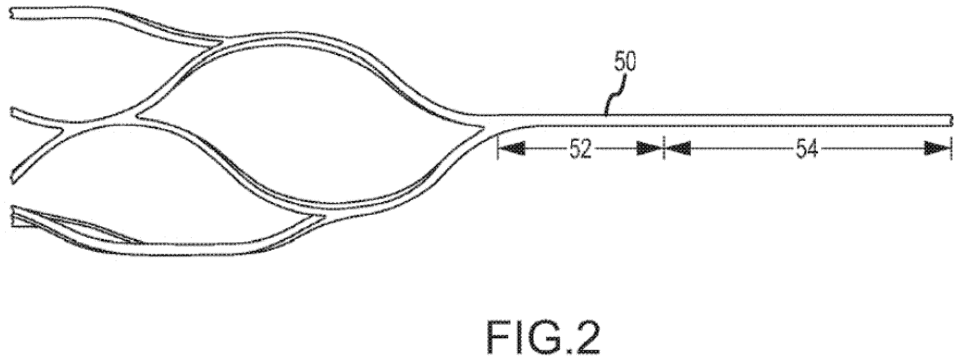

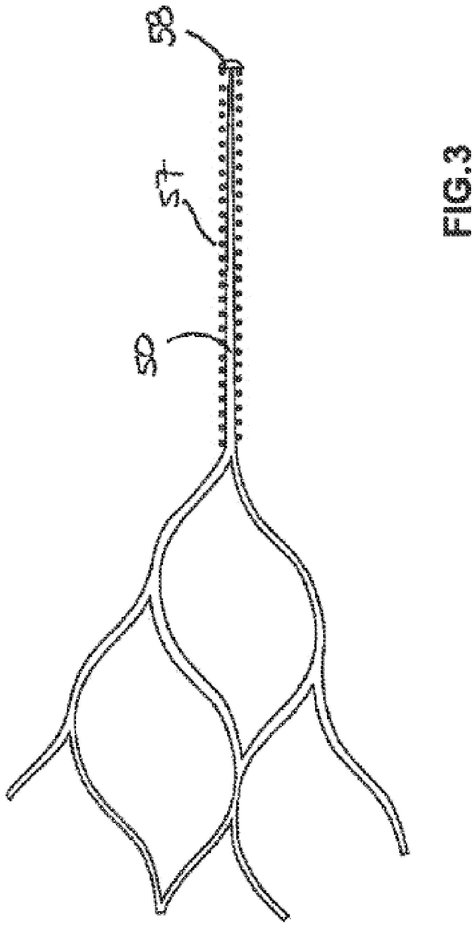

In one implementation, a distal wire segment 50, that is attached to or integrally formed with expandable member 12, extends distally from the distal end 22 of the expandable member 12 and is configured to assist in guiding the delivery of the expandable member to the treatment site of a patient. Figure 2 shows a distal wire segment 50 in one embodiment having a first section 52 of a uniform cross-section and a second section 54 having a distally tapering cross-section. In an exemplary embodiment, the first section 52 has a length of about 3.0 millimeters and an as-cut cross- sectional dimension of about 0.1143 mm (0.0045 inches) by about 0.0762 mm (0.003 inches), and whereas the second section 54 has a length of about 4.0 millimeters and tapers to a distal-most, as-cut, cross-sectional dimension of about 0.0508 mm (0.002 inches) by about 0.0762 mm (0.003 inches). Post-polishing of the device generally involves an etching process that typically results in a 40% to 50% reduction in the as-cut cross-sectional dimensions. In another embodiment, as depicted in Figure 3, the distal wire segment 50 is bound by a spring member 57 of a uniform diameter and is equipped with an atraumatic distal tip 58. In alternative embodiments, the spring element 57 and/or the atraumatic tip 58 are made or coated with of a radiopaque material, such as, for example, platinum.

Figure 28b illustrates an alternative distal wire segment construction. As depicted, the distal wire segment 4010 includes a first section 401 Ia and a second section 4011b, the second section 4011b having a width W greater than the width of the first, section 401 Ia. In one implementation a tapered transition section 4012 joins the first and second sections 4011a and 4011b. In one implementation the width W of the second section is between about 0.0762 mm (0.003 inches) and about 0.1016 mm (0.004 inches) with the length L between the distal end 4013 of the expandable member 4014 and the second section 4011b of the wire segment 4010 being between about 0.381 mm (0.015 inches) and about 0.508 mm (0.020 inches). An advantage of the inclusion of the second section 4011b is that the greater width dimension provides a larger surface area for bonding a coil/spring segment 57 to the wire segment 4010. In one implementation the first section 4011a has a circular or substantially circular construction and the second section 4011b has a flat profile formed by a pressing/coining operation.

In one example, as will be described in more detail below, the expandable member 12 is delivered to the treatment, site of a patient through the lumen of a delivery catheter that has been previously placed at the treatment site. In an alternative embodiment, the vascular treatment device 10 includes a sheath that restrains the expandable member 12 in a compressed state during delivery to the treatment site and which is proximally retractable to cause the expandable member 12 to assume an expanded state.

In one implementation, the expandable member 12 in the expanded state is able to engage an embolic obstruction residing at the treatment site, for example by embedding itself into the obstruction, and is removable from the patient by pulling on a portion of the elongate flexible wire 40 residing outside the patient until the expandable member 12 and at least a portion of the embolic obstruction are removed from the patient.

The use of interconnected and out-of-phase undulating elements 24 to create at least some of the cell structures 26 in alternative embodiments provides several advantages. First, the curvilinear nature of the cell structures 26 enhances the flexibility of the expandable member 12 during its delivery through the tortuous anatomy of the patient to the treatment site. In addition, the out-of- phase relationship between the undulating elements facilitates a more compact nesting of the expandable member elements permitting the expandable member 12 to achieve a very small compressed diameter. A particular advantage of the expandable member strut pattern shown in Figure 1A, and various other embodiments described herein, is that they enable sequential nesting of the expandable member elements which permit the expandable members to be partially or fully deployed and subsequently withdrawn into the lumen of a delivery catheter. The out-of-phase relationship also results in a diagonal orientation of the cell structures 26 which may induce a twisting action as the expandable member 12 transitions between the compressed state and the expanded state that helps the expandable member to better engage with the embolic obstruction. In alternative embodiments, the cell structures 26 of the expandable member 12 are specifically arranged to produce a desired twisting action during expansion of the expandable member 12. In this manner, different expandable members each having different degrees of twisting action may be made available to treat, for example, different types of embolic obstructions.

To enhance visibility of the device under fluoroscopy, the expandable member may be fully or partially coated with a radiopaque material, such as tungsten, platinum, platinum/iridium, tantalum and gold. Alternatively, or in conjunction with the use of a radiopaque coating, radiopaque markers 60 may be positioned at or near the proximal and distal ends 20 and 22 of the expandable device and/or along the proximal and distal wire segments 42 and 50 and/or on selected expandable member strut segments. In one embodiment, the radiopaque markers 60 are radiopaque coils, such as platinum coils.

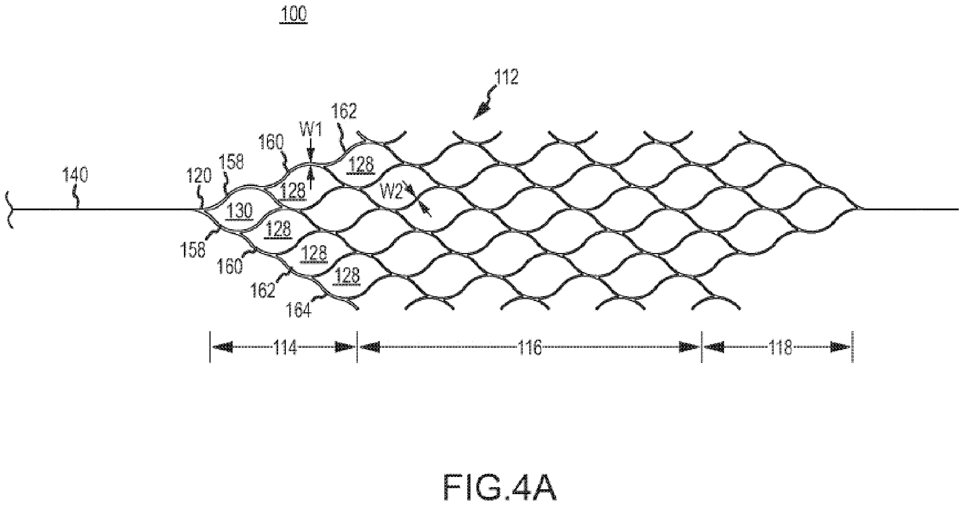

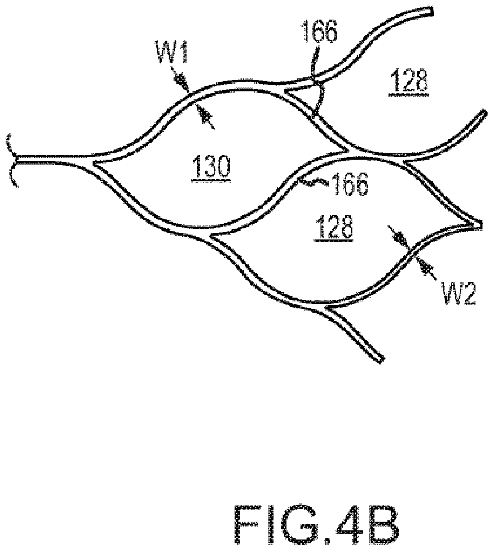

Figure 4A depicts a vascular treatment device 100 in a two-dimensional plane view in another example. In its manufactured and/or expanded tubular configuration, device 100 has a similar construction as device 10 shown in Figure 1B. Like device 10 described above in conjunction with Figures 1A and 1B, device 100 includes a self-expandable member 112 that is coupled to an elongate flexible wire 140. The expandable member 112 includes a proximal end portion 114, a cylindrical main body portion 116 and a distal end portion 118. As mentioned above, delivery of the expandable member 112 in its unexpanded state to the treatment site of a patient is accomplished in one manner by placing the expandable member 112 into the proximal end of a delivery catheter and pushing the expandable member 112 through the lumen of the delivery catheter until it reaches a distal end of the catheter that has been previously placed at or across the treatment site. The proximally extending elongate flexible wire 140 which is attached to or coupled to the proximal end 120 of the expandable member 112 is designed to transmit a pushing force applied to it to its connection point with the elongate flexible member 112. As shown in Figure 4A, and in more detail in Figure 4B, device 100 is distinguishable from the various embodiments of device 10 described above in that the proximal-most cell structures 128 and 130 in the proximal end portion 114 include strut elements having a width dimension W1 larger than the width dimension W2 of the other strut elements within the expandable member 112. As shown, the proximal-most wall sections 160, 162 and 164 of cell structures 128 are made of struts having width W1. Moreover, all the struts of the proximal-most cell structure 130 have an enhanced width W1. The inclusion and placement of the struts with width W1 provides several advantages. One advantage is that they permit the push force applied by the distal end of the elongate wire 140 to the proximal end 120 of elongate member 112 to be more evenly distributed about the circumference of the expandable member 112 as it is being advanced through the tortuous anatomy of a patient. The more evenly distributed push force minimizes the formation of localized high force components that would otherwise act on individual or multiple strut elements within the expandable member 112 to cause them to buckle. Also, by including the struts of width W1 in the peripheral regions of proximal end portion 114, they greatly inhibit the tendency of the proximal end portion 114 to buckle under the push force applied to it by elongate wire 140. In one exemplary embodiment the as-cut, width dimension W1 is about 0.1143 mm (0.0045 inches) and the as-cut width dimension W2 is about 0.0762 mm (0.003 inches). As discussed above, post-polishing of the device generally involves an etching process that typically results in a 40% to 50% reduction in the as-cut cross-sectional dimensions.

It is important to note that although the width dimension W1 is shown as being the same among all struts having an enhanced width, this is not required. For example, in one embodiment wall segments 158 may have an enhanced width dimension greater than the enhanced width dimension of wall segments 160, and wall segments 160 may have an enhanced width dimension greater than the enhanced width dimension of wall segments 162, and so on. Moreover, the inner strut elements 166 of the proximal-most cell structure 130 may have an enhanced width dimension less than the enhanced width dimensions of struts 158. Also, in alternative embodiments, the radial thickness dimension of struts 158, 160, 162, 164, etc. may be enhanced in lieu of the width dimension or in combination thereof.

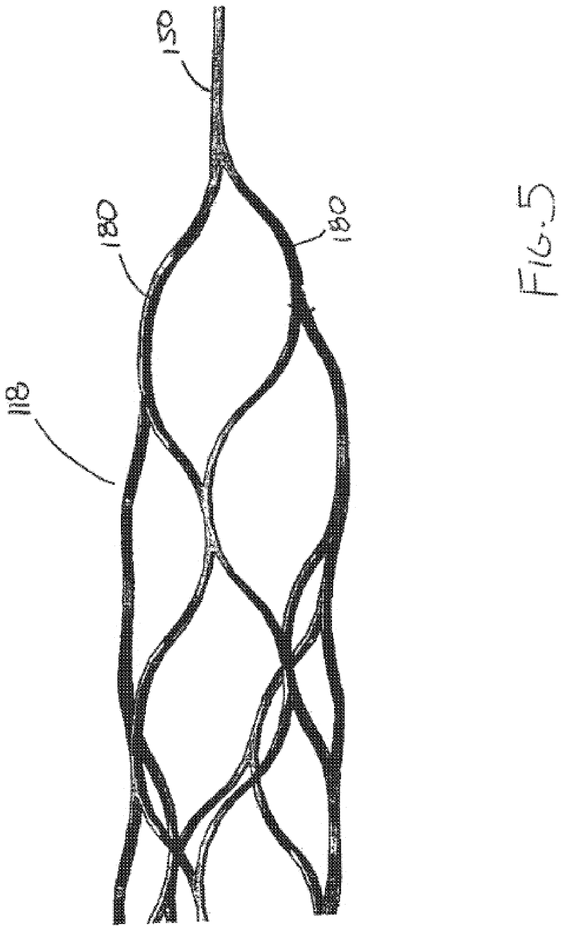

In yet another embodiment, as shown in Figure 5, some of the strut elements 180 in the distal end portion 118 of the expandable member 112 have a mass greater than that of the other struts to resist buckling and possible breaking of the struts as device 100 is advanced to a treatment site of a patient. In the embodiment shown, struts 180 are dimensioned to have the same width as distal wire segment 150. In alternative embodiments, the thickness dimension of struts 180 may be enhanced in lieu of the width dimension or in combination thereof.

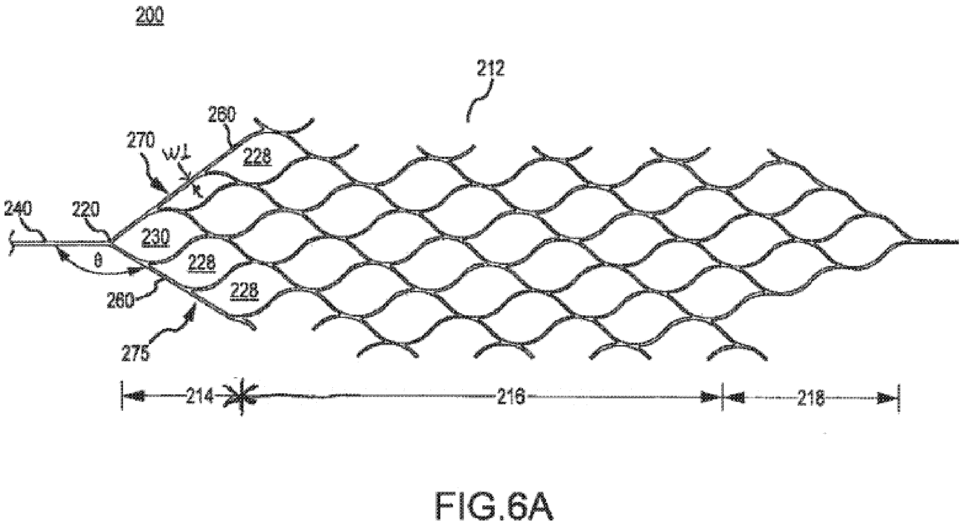

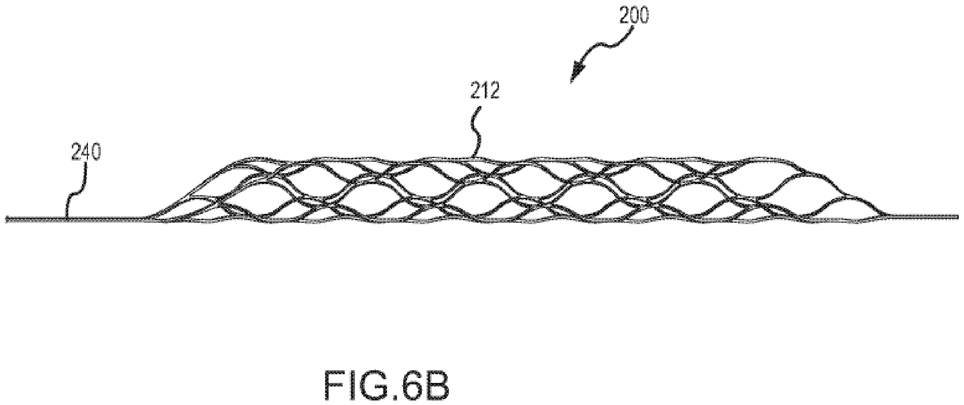

Figures 6A and 6B illustrate a vascular treatment device 200 in accordance with another example. Figure 6A depicts device 200 in a two-dimensional plane view as if the device were cut and laid flat on a surface. Figure 6B depicts the device in its manufactured and/or expanded tubular configuration. Device 200 includes an expandable member 212 having a proximal end portion 214, a cylindrical main body portion 216 and a distal end portion 218 with an elongate flexible wire 240 attached to or otherwise coupled to the proximal end 220 of the expandable member. The construction of device 200 is similar to device 100 described above in conjunction with Figures 4A except that the proximal wall segments 260 of cell structures 228 and 230 comprise linear or substantially linear strut, elements as viewed in the two dimension plane view of Figure 6A. In one embodiment, the linear strut elements 260 are aligned to form continuous and substantially linear rail segments 270 that, extend from the proximal end 220 of proximal end portion 214 to a proximal-most end of main body portion 216 (again, as viewed in the two dimension plane view of Figure 6A) and preferably are of the same length, but may be of different lengths. When the pattern of Figure 6A is applied to laser cutting a tubular structure, the resulting expandable member configuration is that as shown in Figure 6B. As shown in Figure 6B, rail segments 270 are not in fact linear but are of a curved and non-undulating shape. This configuration advantageously provides rail segments 270 devoid of undulations thereby enhancing the rail segments' ability to distribute forces and resist buckling when a push force is applied to them, in alternative preferred embodiments, the angle Θ between the wire segment 240 and rail segments 270 ranges between about 140 degrees to about 150 degrees. In one embodiment, one or both of the linear rail segments 270 have a width dimension W1 which is greater than the width dimension of the adjacent strut segments of cell structures 228 and 230. An enhanced width dimension W1 of one or both the linear rail segments 270 further enhances the rail segments' ability to distribute forces and resist buckling when a push force is applied to them. In another implementation, one or both of the linear rail segments 270 are provided with an enhanced thickness dimension, rather than an enhanced width dimension to achieve the same or similar result. In yet an alternative implementation, both the width and thickness dimensions of one or both of the linear rail segments 270 are enhanced to achieve the same or similar results. In yet another implementation, the width and/or thickness dimensions of each of the rail segments 270 differ in a manner that causes a more even compression of the proximal end portion 214 of the expandable member 212 when it is loaded or retrieved into a delivery catheter or sheath (not shown).

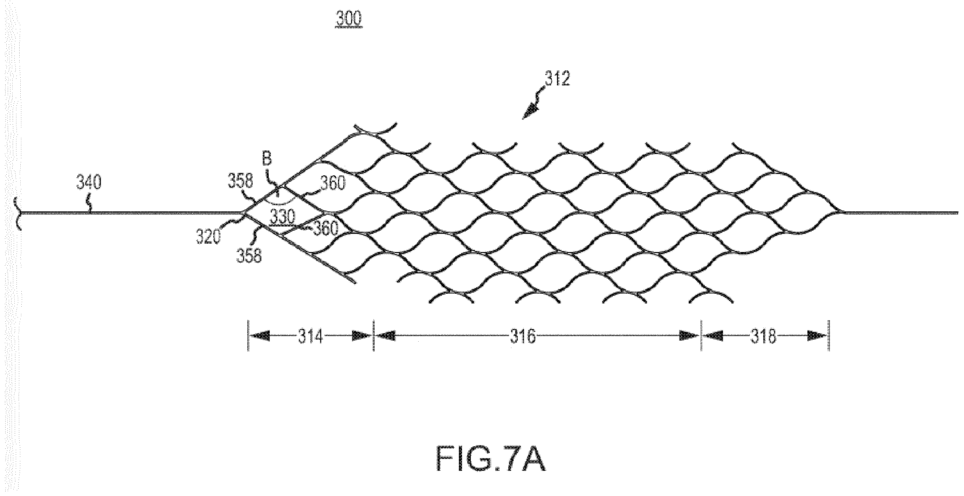

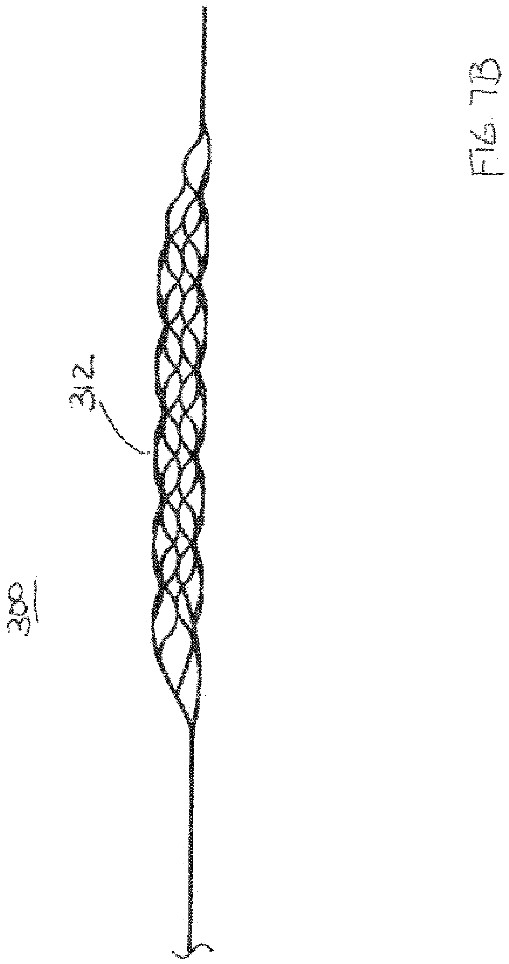

Figures 7A and 7B illustrate a vascular treatment device 300 in accordance with another example. Figure 7A depicts device 300 in a two-dimensional plane view as if the device were cut and laid flat on a surface. Figure 7B depicts the device in its manufactured and/or expanded tubular configuration. Device 300 includes an expandable member 312 having a proximal end portion 314, a cylindrical main body portion 316 and a distal end portion 318 with an elongate flexible wire 340 attached to or otherwise coupled to the proximal end 320 of the expandable member. The construction of device 300 is similar to device 200 described above in conjunction with Figures 6A and 6B except that the proximal-most cell structure 330 comprises a substantially diamond shape as viewed in the two-dimensional plane of Figure 7A. The substantially diamond-shaped cell structure includes a pair of outer strut elements 358 and a pair of inner strut elements 360, each having an enhanced width and/or enhanced thickness dimension as previously discussed in conjunction with the embodiments of Figures 4 and 6. In alternative preferred embodiments, the inner strut elements 360 intersect the outer strut elements 358 at an angle β between about 25.0 degrees to about 45,0 degrees as viewed in the two-dimensional plane view of Figure 7A. Maintaining the angular orientation between the inner and outer struts within in this range enhances the pushabilty of the expandable member 312 without the occurrence of buckling and without substantially affecting the expandable member's ability to assume a very small compressed diameter during delivery.

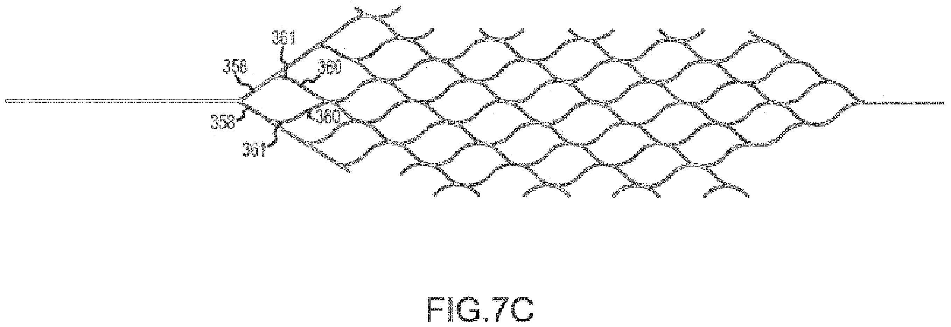

In one embodiment, the inner strut elements 360 have a mass less than that of the outer strut elements 358 that enables them to more easily bend as the expandable member 312 transitions from an expanded state to a compressed state. This assists in achieving a very small compressed diameter, in another embodiment, as shown in Figure 7C, the inner strut elements 360 are coupled to the outer strut elements 358 by curved elements 361 that enable the inner strut elements 360 to more easily flex when the expandable member 312 is compressed to its delivery position.

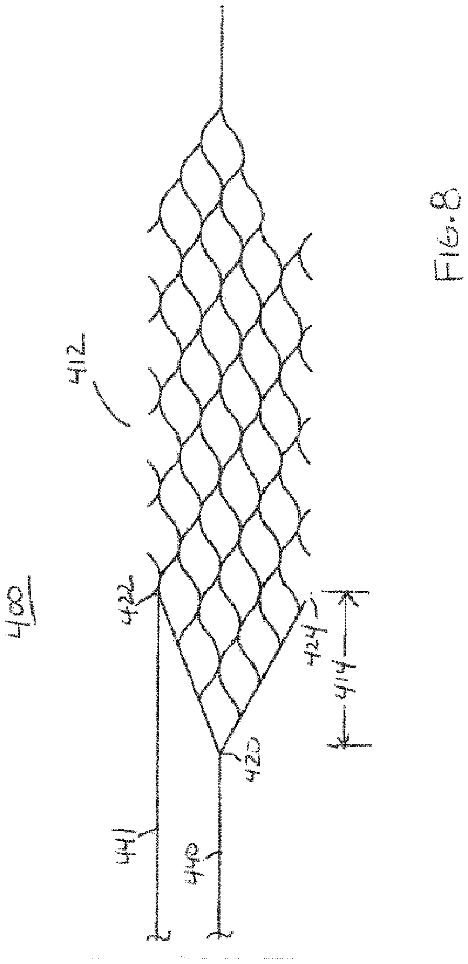

Figure 8 illustrates an alternative example of a vascular treatment device 400. Device 400 has a similar construction to that of device 200 depicted in Figures 6A and 6B with the exception that the expandable member 412 of device 400 is connected at its proximal end portion 414 with two distally extending elongate flexible wires 440 and 441, As illustrated, wire 440 is attached to or otherwise coupled to the proximal- most end 420 of proximal end portion 414, while wire 441 is attached to or otherwise coupled to the distal-most end 422 of the proximal end portion 414 at the junction with rail segment 470. In yet another embodiment, an additional elongate flexible wire (not shown) may be attached to the distal-most end 424. The use of two or more elongate flexible wires 440 and 441 to provide pushing forces to the proximal end portion 414 of elongate member 412 advantageously distributes the pushing force applied to the proximal end portion 414 to more than one attachment point.

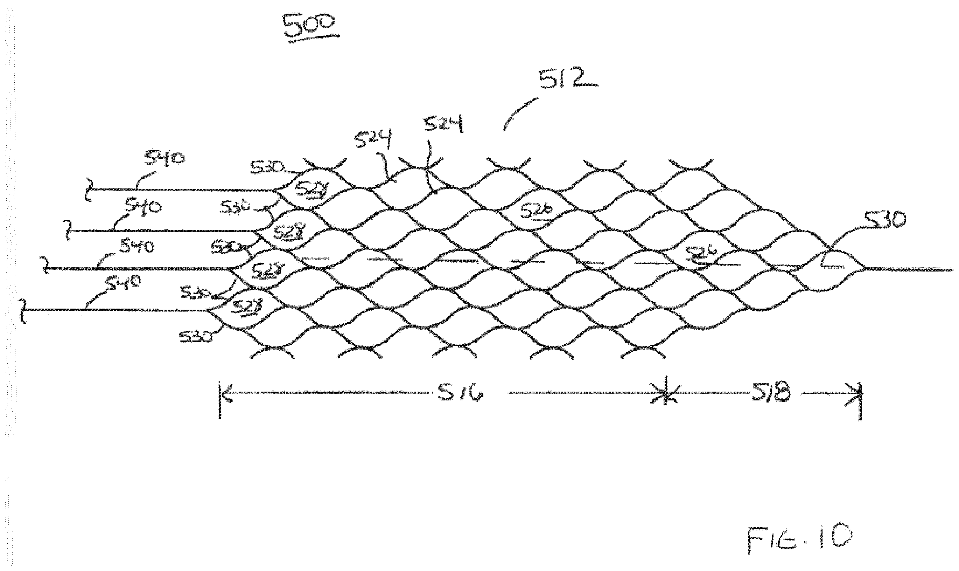

Figure 10 illustrates a two-dimensional plane view of a vascular treatment device 500 in another example. In the embodiment of Figure 10, expandable member 512 includes a plurality of generally longitudinal undulating elements 524 with adjacent undulating elements being out-of-phase with one another and connected in a manner to form a plurality of diagonally disposed cell structures 526. The expandable member 512 includes a cylindrical portion 516 and a distal end portion 518 with the cell structures 526 in the main body portion 516 extending continuously and circumferentially around a longitudinal axis 530 of the expandable member 512. The cell structures 526 in the distal end portion 518 extend less than circumferentially around the longitudinal axis 530 of the expandable member 512, Attached to or otherwise coupled to each of the proximal-most cell structures 528 are proximally extending elongate flexible wires 540. The use of multiple elongate flexible wires 540 enables the pushing force applied to the proximal end of the expandable member 512 to be more evenly distributed about its proximal circumference, in another embodiment, although not shown in Figure 10, the proximal-most strut elements 528 have a width and/or thickness greater than the struts in the other portions of the expandable member 512. Such a feature further contributes to the push force being evenly distributed about the circumference of the expandable member 512 and also inhibits the strut elements directly receiving the push force from buckling.

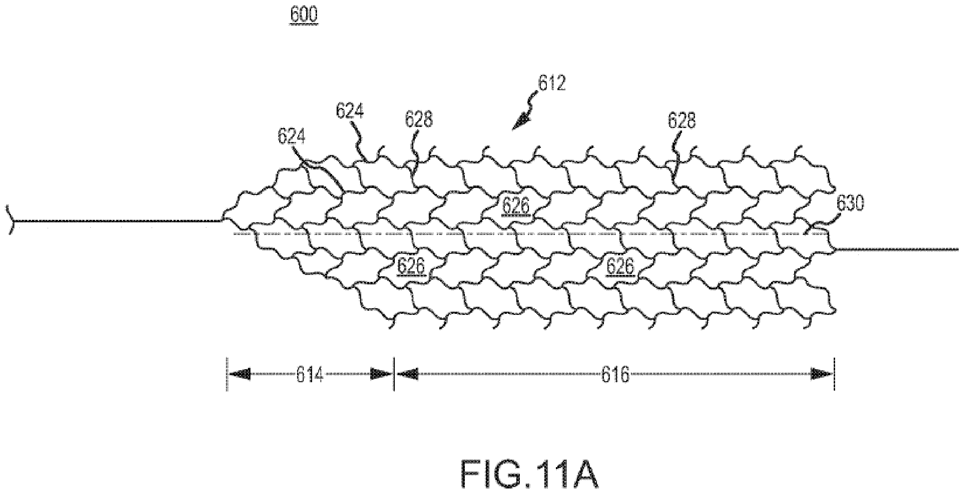

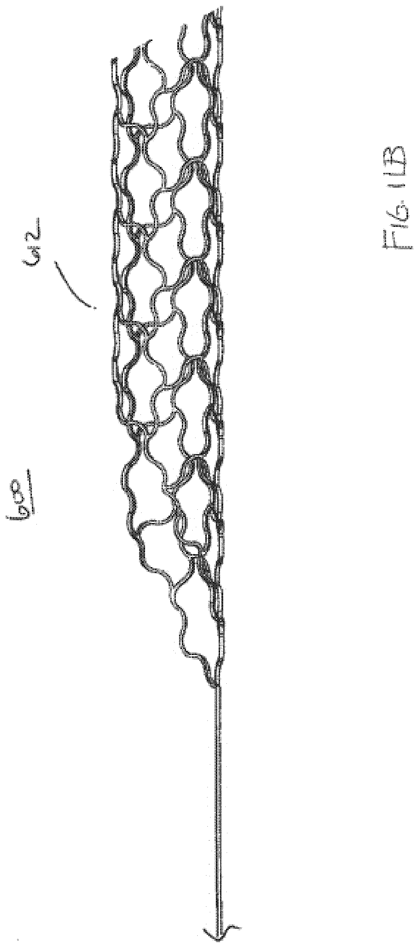

Figures 11A and 11B illustrate another example of a vascular treatment device 600. Figure 11A depicts device 600 in a two-dimensional plane view as if the device were cut and laid flat on a surface. Figure 11B depicts the device in its manufactured and/or expanded tubular configuration. In the embodiment of Figures 11A and 11B, expandable member 612 includes a plurality of generally longitudinal undulating elements 624 with adjacent undulating elements being interconnected by a plurality of curved connectors 628 to form a plurality of closed-cell structures 626 disposed about the length of the expandable member 612. In the example shown, the expandable member 612 includes a proximal end portion 614 and a cylindrical portion 616 with the cell structures 626 in the cylindrical portion 616 extending continuously and circumferentially around a longitudinal axis 630 of the expandable member 612. The cell structures 626 in the proximal end portion 614 extend less than circumferentially around the longitudinal axis 630 of the expandable member 612. In an alternative embodiment, the expandable member 612 includes a proximal end portion, a cylindrical main body portion and a distal end portion, much like the expandable member 12 depicted in Figures 1A and 1B, In such an embodiment, the cell structures 626 in the distal end portion of the expandable member would extend less than circumferentially around the longitudinal axis 630 of the expandable member 612 in a manner similar to the proximal end portion 614 shown in Figure 11A. Moreover, it is appreciated that the expandable members of Figures 1A, 4A, 6A, 7A, 7C, 10, 14, 15 and 19-24 may be modified in a way so as to eliminate the distal end portion (e.g., distal end portion 18 in Figure 1A) so that there exists only a proximal end portion and main body portion like that of Figure 11A.

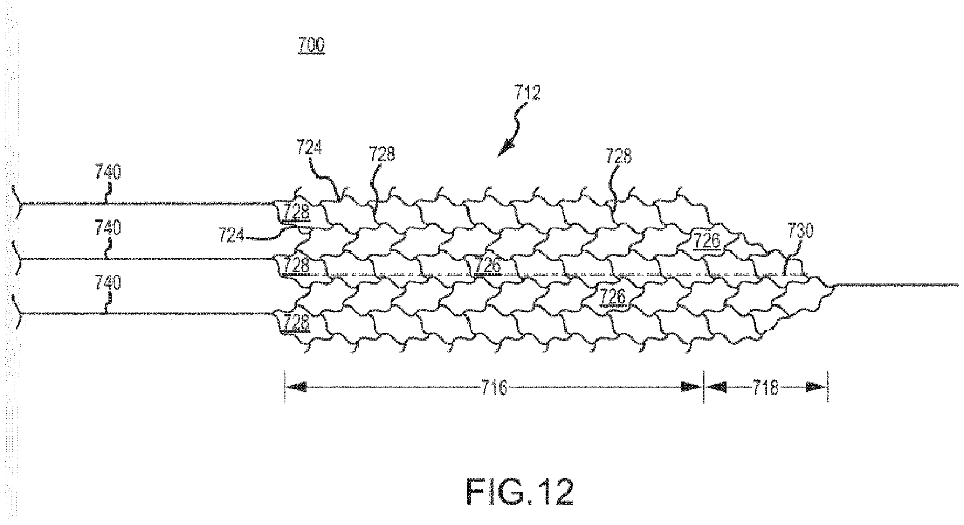

Figure 12 illustrates a vascular treatment device 700 in accordance with another example. Figure 12 depicts device 700 in a two-dimensional plane view as if the device were cut and laid flat on a surface. In the embodiment of Figure 12, expandable member 712 includes a plurality of generally longitudinal undulating elements 724 with adjacent undulating elements being interconnected by a plurality of curved connectors 728 to form a plurality of closed- cell structures 726 disposed about the length of the expandable member 712. In the embodiment shown, the expandable member 712 includes a cylindrical portion 716 and a distal end portion 718 with the cell structures 726 in the cylindrical portion 716 extending continuously and circumferentially around a longitudinal axis 730 of the expandable member 712. The cell structures 726 in the distal end portion 718 extend less than circumferentially around the longitudinal axis 730 of the expandable member 712. In a manner similar to that described in conjunction with the example of Figure 10, attached to or otherwise coupled to each of the proximal-most cell structures 728 are proximally extending elongate flexible wires 740. This arrangement enables the pushing force applied to the proximal end of the expandable member 712 to be more evenly distributed about its proximal circumference, in another embodiment, although not shown in Figure 12, the proximal-most strut elements 730 have a width and/or thickness greater than the struts in the other portions of the expandable member 712. Such a feature further contributes to the push force being evenly distributed about the circumference of the expandable member 712 and also inhibits the strut elements directly receiving the push force from buckling.

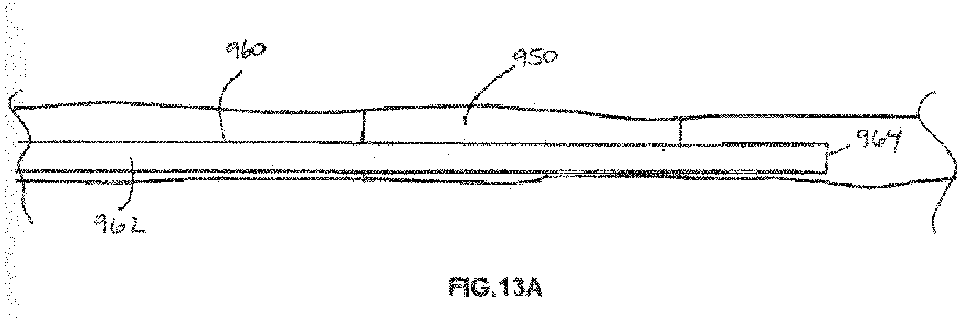

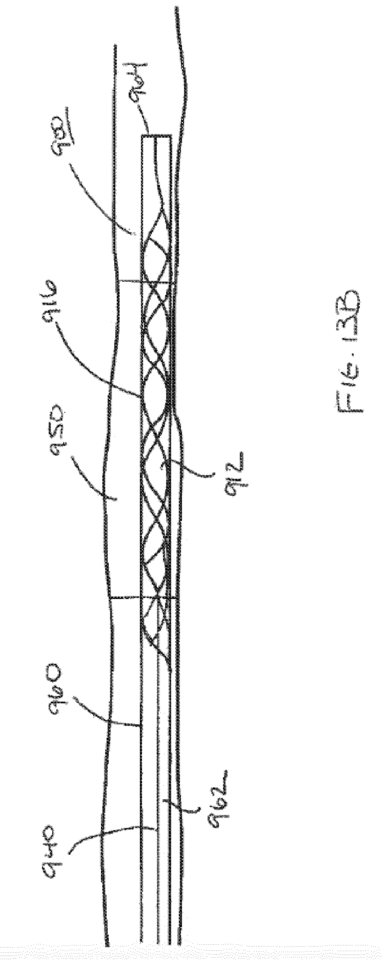

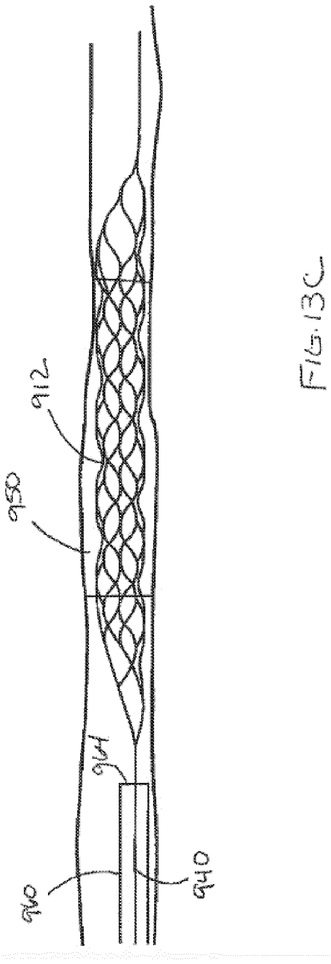

As previously discussed, in use, the expandable members of the present invention are advanced through the tortuous vascular anatomy of a patient to a treatment site, such as an embolic obstruction, in an unexpanded or compressed state of a first nominal diameter and are movable from the unexpanded state to a radially expanded state of a second nominal diameter greater than the first nominal diameter for deployment, at the treatment site. One manner of delivering and deploying expandable member 912 at the site of an embolic obstruction 950 is shown in Figures 13A through 13C. As shown in Figure 13A, a delivery catheter 960 having an inner lumen 962 is advanced to the site of the embolic obstruction 950 so that its distal end 964 is positioned distal to the obstruction. After the delivery catheter 960 is in position at the embolic obstruction 950, the retrieval device 900 is placed into the delivery catheter by introducing the expandable member 912 into a proximal end of the delivery catheter (not shown) and then advancing the expandable member 912 through the lumen 962 of the delivery catheter by applying a pushing force to elongate flexible wire 940, By the use of radiopaque markings and/or coatings positioned on the delivery catheter 960 and device 900, the expandable member 912 is positioned at the distal end of the delivery catheter 960 as shown in Figure 13B so that the main body portion 916 is longitudinally aligned with the obstruction 950. Deployment of the expandable member 912 is achieved by proximally withdrawing the delivery catheter 960 while holding the expandable member 912 in a fixed position as shown in Figure 13C. Once the expandable member 912 has been deployed to an expanded position within the obstruction 950, the expandable member 912 is retracted, along with the delivery catheter 960, to a position outside the patient. In one example, the expandable member 912 is first partially retracted to engage with the distal end 964 of the delivery catheter 960 prior to fully retracting the devices from the patient.

In one example, once the expandable member 912 is expanded at the obstruction 950, it is left to dwell there for a period of time in order to create a perfusion channel through the obstruction that causes the obstruction to be lysed by the resultant blood flow passing through the obstruction. In such an example, it is not necessary that the expandable member 912 capture a portion of the obstruction 950 for retrieval outside the patient. When a sufficient portion of the obstruction 950 has been lysed to create a desired flow channel through the obstruction, or outright removal of the obstruction is achieved by the resultant blood flow, the expandable member 912 may be withdrawn into the delivery catheter 960 and subsequently removed from the patient.

In another example, the expandable member 912 is expanded at the obstruction 950 and left to dwell there for a period of time in order to create a perfusion channel through the obstruction that causes the obstruction to be acted on by the resultant flow in a manner that makes the embolic obstruction more easily capturable by the expandable member and/or to make it more easily removable from the vessel wall of the patient. For example, the blood flow created through the embolic obstruction may be made to flow through the obstruction for a period of time sufficient to change the morphology of the obstruction that makes it more easily captured by the expandable member and/or makes it more easily detachable from the vessel wall. As in the preceding method, the creation of blood flow across the obstruction 950 also acts to preserve tissue. In one example, the blood flow through the obstruction may be used to lyse the obstruction. However, in this modified method, lysing of the obstruction is performed for the purpose of preparing the obstruction to be more easily captured by the expandable member 912. When the obstruction 950 has been properly prepared, for example by creating an obstruction 950 of a desired nominal inner diameter, the expandable member 912 is deployed from the distal end 964 of the delivery catheter 940 to cause it to engage with the obstruction. Removal of all, or a portion, of the obstruction 950 from the patient is then carried out, in a manner similar to that described above.

In yet another example, once the expandable member 912 has been delivered and expanded inside the obstruction 950, it may be detached from the elongate wire 940 for permanent placement within the patient. In such an example, the manner in which the elongate wire 940 is attached to the expandable member 932 allows the two components to be detached from one another. This may be achieved, for example, by the use of a mechanical interlock or an erodable electrolytic junction between the expandable member 912 and the elongate wire 940.

As described herein, the expandable members of the various embodiments may or may not include distal wire segments that are attached to their distal ends. In alternative preferred embodiments, vascular treatment devices that are configured to permanently place an expandable member at the site of an embolic obstruction do not include distal wire segments attached to the distal ends of the expandable members.

One advantage associated with the expandable member cell patterns of the present invention is that withdrawing the expandable members by the application of a pulling force on the proximal elongate wire flexible wire urges the expandable members to assume a smaller expanded diameter while being withdrawn from the patient, thus decreasing the likelihood of injury to the vessel wall. Also, during clot retrieval as the profile of the expandable members decrease, the cell structures collapse and pinch down on the clot to increase clot retrieval efficacy. Another advantage is that the cell patterns permit the expandable members to be retracted into the lumen of the delivery catheter after they have been partially or fully deployed. As such, if at any given time it is determined that the expandable member has been partially or fully deployed at an improper location, it may be retracted into the distal end of the delivery catheter and repositioned to the correct location.

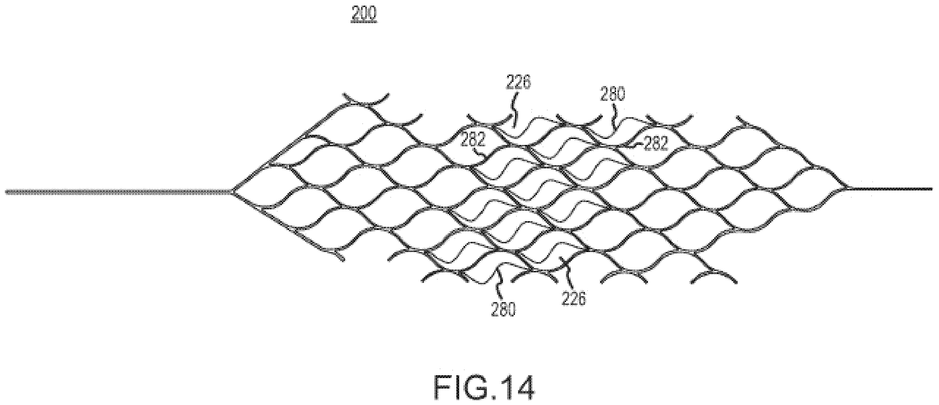

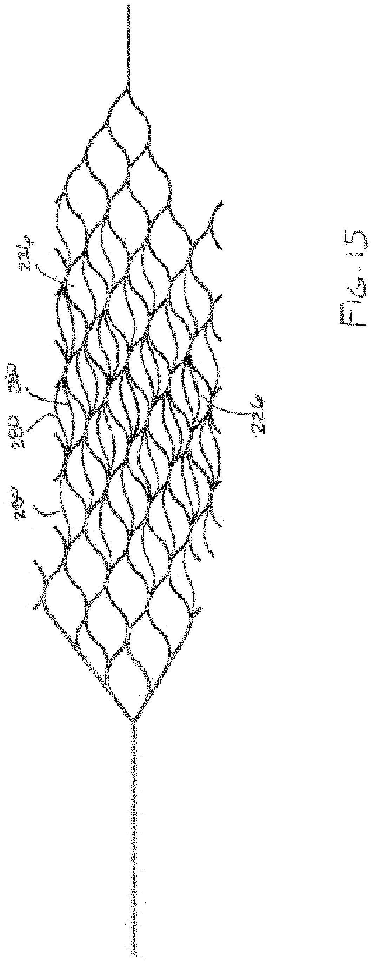

With reference to Figure 14, a modified version of the vascular treatment device 200 of Figure 6A is shown that includes thin strut elements 280 intersecting at least some of the cell structures 226 located in the cylindrical main body portion 216 of expandable member 212. The thin strut elements 280 are dimensioned to have a width of less than the strut elements 282 that form the cell structures 226. In alternative exemplary embodiments, strut elements 280 have an as-cut or polished width dimension that is between about 25% to about 50% smaller than the respective as-cut or polished width dimension of struts 262. When used for the purpose of clot retrieval, a purpose of the thin struts 280 is to enhance the expandable member's ability to engage with and capture an embolic obstruction. This is accomplished by virtue of several factors. First, the thinner width dimensions of the struts 280 make it easier for the struts to penetrate the obstruction. Second, they act to pinch portions of the entrapped obstruction against the outer and wider strut elements 282 as the expandable member is deployed within the obstruction. Third, they may be used to locally enhance radial forces acting on the obstruction. It is important to note that the use of thin strut elements 280 is not limited to use within cell structures 226 that reside within the cylindrical main body portion 216 of the expandable member 212. They may be strategically positioned in any or all of the cell structures of the expandable member. Moreover, it is important to note that the use of thin strut elements 280 is not limited to the embodiment of Figures 6, but are applicable to all the various embodiments disclosed herein. Lastly, in alternative exemplary embodiments, as shown in Figure 15, multiple thin strut elements 280 are provided within one or more of the cell structures 226, and may also be used in conjunction with cell structures that have a single thin strut element and/or cell structures altogether devoid of thin strut elements.

In the treatment of aneurysms when the treatment device is used for the purpose of diverting flow, the density of the cell structures 226 is sufficient to effectively divert flow away from the aneurysm sack. In alternative embodiments in lieu of, or in combination with adjusting the density of the cell structures 226, intermediate strut elements similar to the strut elements 280 of Figures 14 and 15 are used to increase the effective wall surface of the expandable member. In these embodiments, the intermediate strut elements may have the same, smaller, larger, or any combination thereof, dimensional characteristics of the cell structure struts. Conversely, in alternative embodiments for use in the treatment of aneurysms for the purpose placing coils or other like structures within the sack of the aneurysm, the size of the cell structures 226 is sufficient, to facilitate passage of the coils through the cell structures.

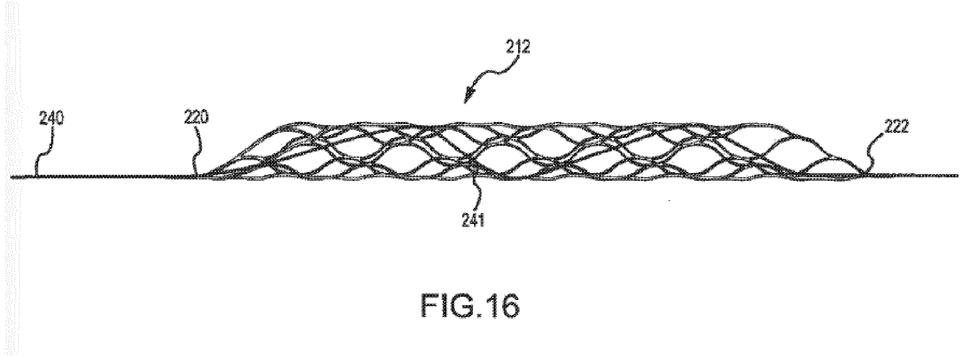

Figure 16 illustrates a treatment device according to the example of Figures 6A and 6B, wherein the pushability of the expandable member 212 during its advancement to the treatment site of a patient is enhanced by the inclusion of an internal wire segment 241 that extends between the proximal end 220 and distal end 222 of the expandable member 212. In this manner, the pushing force applied by elongate wire 240 is transmitted to both the proximal and distal ends of expandable device. The internal wire segment may be a discrete element that is attached to the proximal and distal ends of the expandable member, or may preferably be a co-extension of the elongate flexible wire 240. During delivery of the expandable member 212 to the treatment site in its compressed state, the internal wire segment 241 assumes a substantially straight or linear configuration so as to adequately distribute at least a part of the pushing force to the distal end 222 of the expandable member. When the expandable member 212 expands, it tends to foreshorten causing slack in the internal wire segment 241 that forms a long-pitched helix within the expandable member as shown in Figure 16. Art additional advantage associated with the use the internal wire segment 241 is that the formation of the internal helix upon expansion of the expandable member 212 assists in capturing the embolic obstruction.

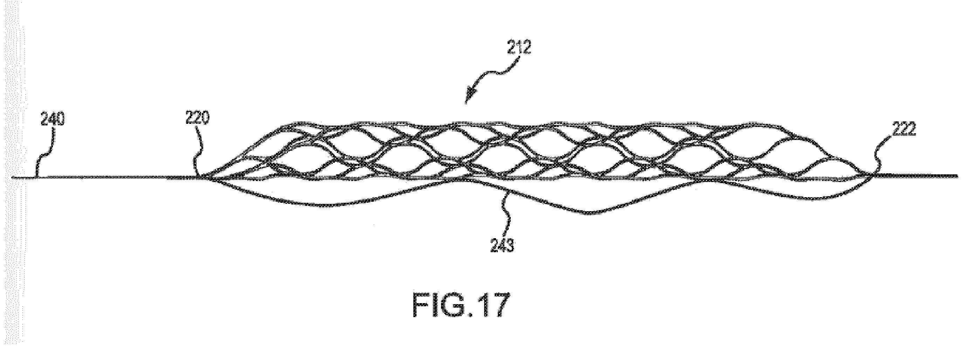

In an alternative embodiment, as shown in Figure 17, the pushability of the expandable member 212 during its advancement to the treatment site of a patient is enhanced by the inclusion of an external wire segment 243 that extend between the proximal end 220 and distal end 222 of the expandable member 212. In this manner, the pushing force applied by the elongate wire 240 is transmitted to both the proximal and distal ends of the expandable device. The external wire segment may be discrete element that is attached to the proximal and distal ends of the expandable member, or may preferably be a co-extension of the elongate flexible wire 240. During delivery of the expandable member 212 to the treatment site in its compressed state, the external wire segment 243 assumes a substantially straight or linear configuration so as to adequately distribute at least a part of the pushing force to the distal end 222 of the expandable member. When the expandable member 212 expands, it tends to foreshorten causing slack in the external wire segment 243 as shown in Figure 17. An additional advantage associated with the use of the external wire segment 243 is that it directly acts on the obstruction while the expandable member 212 is expanded to assist in engaging and capturing the embolic obstruction.

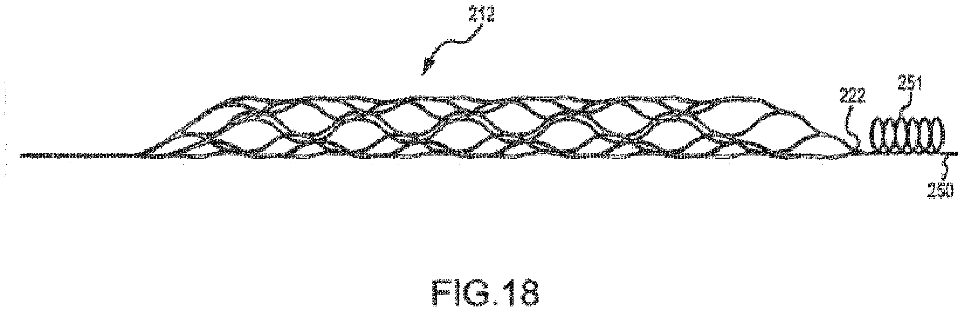

In yet another embodiment, a distal emboli capture device 251 is disposed on the distal wire segment 250, or otherwise attached to the distal end 222, of expandable member 212 as shown in Figure 18. The function of the distal emboli capture device 251 is to capture emboli that may be dislodged from the embolic obstruction during the expansion of the expandable member 212 or during its removal from the patient to prevent distal embolization. In Figure 18, the distal emboli capture device is shown as a coil. In alternative embodiments, baskets, embolic filters or other known emboli capture devices may be attached to the distal end 222 or distal wire segment 250 of expandable member 12.

Again, as with the embodiments of Figures 14 and 15, it is important to note that the features described in conjunction with Figures 16, 17 and 18 are not limited to the embodiment of Figures 6, but are applicable to all the various embodiments disclosed herein.

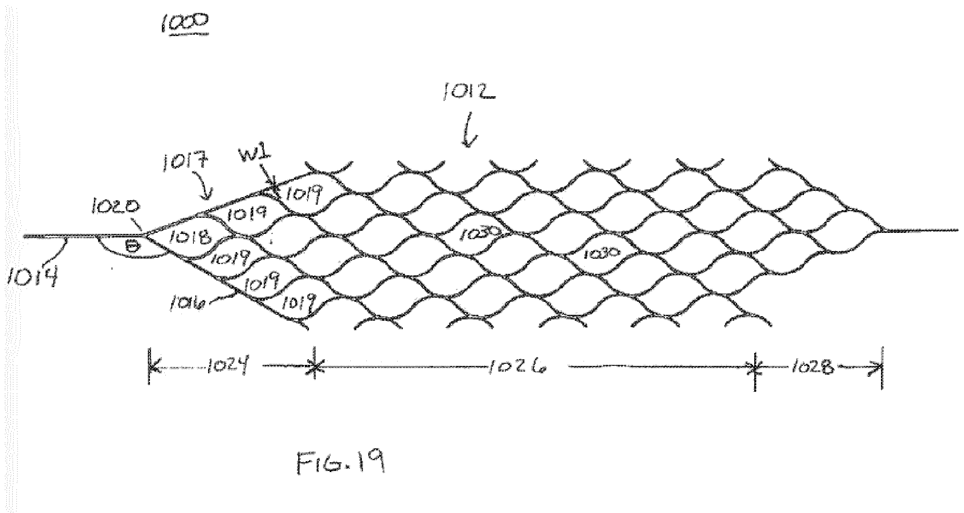

Figure 19 illustrates another example of a bodily duct or vascular treatment device 1000. Figure 19 depicts device 1000 in a two-dimensional plane view as if the device were cut and laid flat on a surface. Device 1000 includes an expandable member 3032 having a proximal end portion 1024, a cylindrical main body portion 1026 and a distal end portion 1028 with an elongate flexible wire 1014 attached to or otherwise coupled to the proximal end 1020 of the expandable member. The construction of device 1000 is similar to device 200 described above in conjunction with Figures 6A except that the cell structures 1018 and 1019 in the proximal end portion 1024 are more closely symmetrically arranged than the cell structures in the proximal end portion 214 of device 200. The more substantial symmetrical arrangement of the cell structures in the proximal end portion 1024 of device 1000 facilitates the loading or retrieval of the expandable member 1012 into a lumen of a delivery catheter or sheath (not shown) by causing the proximal end portion 1024 to collapse more evenly during compression. The proximal wall segments 1016 of cell structures 1018 and 1019 comprise linear or substantially linear strut elements as viewed in the two dimension plane view of Figure 19. In one embodiment, the linear strut elements 1016 are aligned to form continuous and substantially linear rail segments 1017 that extend from the proximal end 1020 of proximal end portion 1024 to a proximal- most end of main body portion 1026 (again, as viewed in the two dimension plane view of Figure 19) and preferably are of the same length. In alternative embodiments, the angle Θ between the wire segment 1014 and rail segments 1017 ranges between about 140 degrees to about 150 degrees. In one embodiment, one or both of the linear rail segments 1017 have a width dimension W1 which is greater than the width dimension of the adjacent strut segments of cell structures 1018 and/or 1019 and/or 1030. An enhanced width dimension W1 of one or both the linear rail segments 1017 further enhances the rail segments' ability to distribute forces and resist buckling when a push force is applied to them. In another implementation, one or both of the linear rail segments 1017 are provided with an enhanced thickness dimension, rather than an enhanced width dimension to achieve the same or similar result. In yet an alternative implementation, both the width and thickness dimensions of one or both of the linear rail segments 1017 are enhanced to achieve the same or similar results. In yet another implementation, the width and/or thickness dimensions of each of the rail segments 1017 differ in a manner that causes a more even compression of the proximal end portion 1024 of the expandable member 1012 when it is collapsed as it is loaded or retrieved into a delivery catheter or sheath.

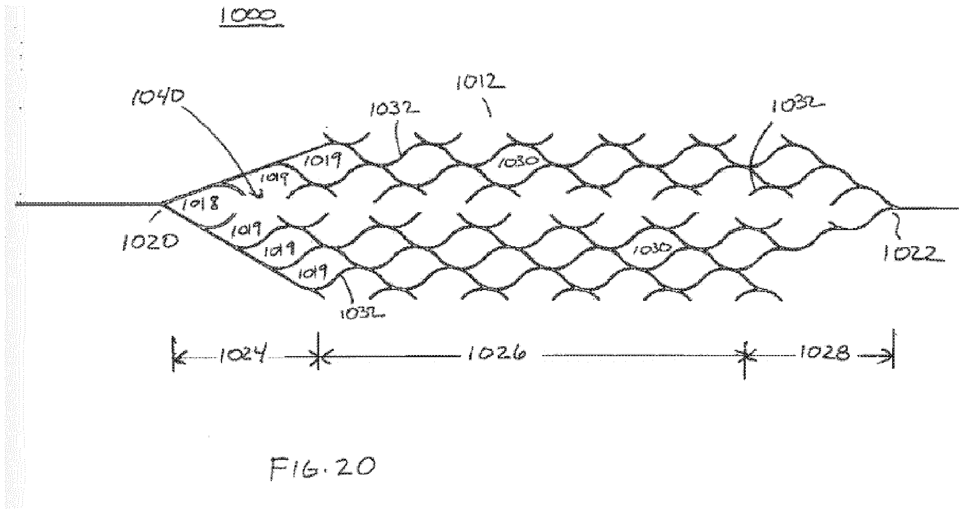

Although the description that follows is directed to the example of Figure 19, it is important to note that the provision of a slit as contemplated by the embodiments of Figures 20-22 are applicable to all the vascular treatment devices described herein, and their numerous embodiments and modifications thereof.

Turning now to Figure 20, the treatment device 3000 of Figure 19 is depicted having a longitudinal slit 1040 that extends from the proximal end 1020 to the distal end 1022 of the expandable member 1012. The slit 1040 permits the cell structures 1018, 1019 and 1030 to move relative to one another in a manner that inhibits the individual strut elements 1032 of the expandable member 1012 from buckling during compression of the expandable member 1012 as it is loaded or retrieved into a delivery catheter or sheath. In alternative embodiments, slit 1040 extends less than the entire length of expandable member 1012 and is arranged to inhibit buckling of strategically important strut elements that most affect the expandable member's ability to be effectively loaded or withdrawn into a delivery catheter or sheath. For example, in one embodiment, slit 1040 is provided only in the proximal end portion 1024 of the expandable member 1012 where the likelihood of buckling or bending of struts 1032 is most likely to occur. In another embodiment, slit 1040 is provided in both the proximal end portion 1024 and the cylindrical main body portion 1026 of expandable member 1012.

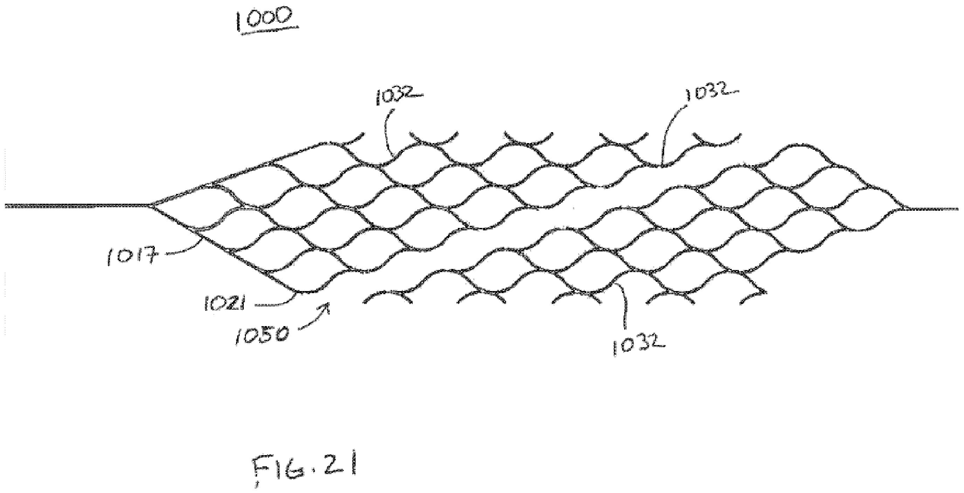

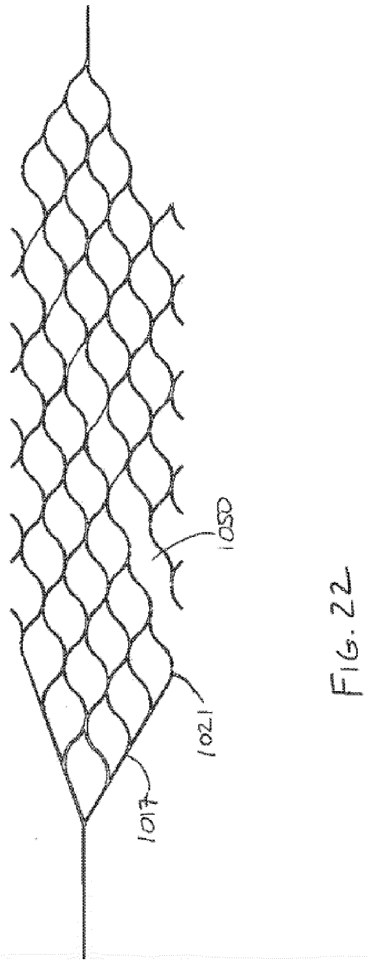

Figure 21 illustrates the treatment device 1000 of Figure 19 having a diagonally disposed/spiral slit 1050 that extends the entire circumference of the expandable member 1012. In one embodiment, as illustrated in Figure 21, the spiral slit 1050 originates at the distal position, or at a point adjacent to the distal position, of the proximal end portion 1024 of expandable member 1012. With respect to the embodiments having linear rail segments, such as the linear rail segments 1017 of Figure 19, the spiral slit 1050 originates at the distal position 1021 of one of the linear rail segments 1017, or at a point distally adjacent to the distal position 1021, as shown in Figure 21. Testing of the various vascular treatment devices described herein has shown that the occurrence of buckling tends to occur at the strut elements located adjacent to the distal positions of the proximal end portions of the expandable members. This phenomenon is exacerbated in the expandable members having proximal end portions with linear rail segments. For this reason, and with reference to Figure 21, the originating point of spiral slit 1050 is located at or adjacent to a distal position 1021 of one of the linear rail segments 1017. An advantage of the diagonally disposed and/or spiral slit, configuration of Figure 21 is that it, originates where the buckling tends to originate and further inhibits buckling of strut elements 1032 along the length of the expandable member 1012. As shown in Figure 22, in alternative embodiments slit 1050 extends diagonally along only a portion of the circumference of the cylindrical main body portion 1026 of the expandable member 1012. In the embodiment of Figure 22, slit 1050 originates at the distal position 1021 of linear rail segment 1017. In alternative embodiments, where buckling of individual strut elements 1032 originate at a point other than at the distal point of the proximal end portion 1024 of the expandable member 1012, the originating point of the slit 1050 is located at the origination point of the bucking (absent the slit 1050) and extends in a longitudinal direction distally therefrom.

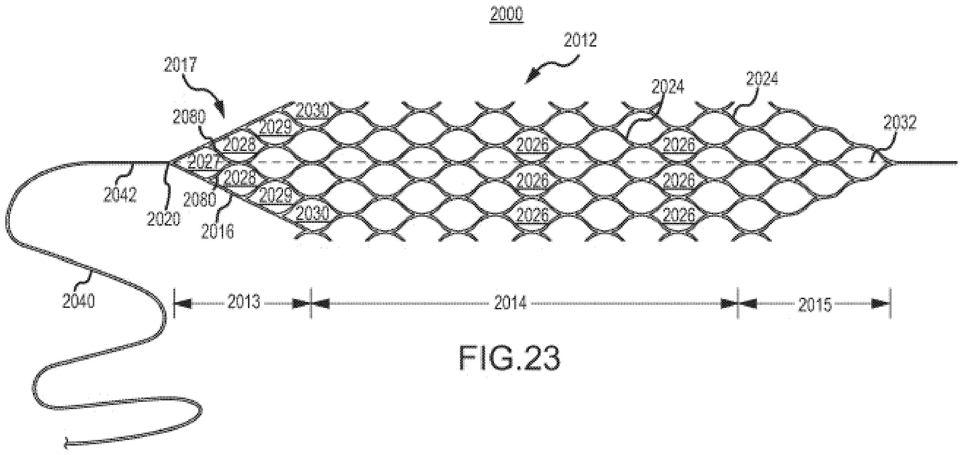

Figure 23 illustrates another example of a bodily duct or vascular treatment device 2000. Figure 23 depicts device 2000 in a two-dimensional plane view as if the device were cut and laid flat on a surface. Device 2000 includes a self-expandable member 2012 that is attached or otherwise coupled to an elongate flexible wire 2040 that extends proximally from the expandable member 2012. In one embodiment, the expandable member 2012 is made of shape memory material, such as Nitinol, and is preferably laser cut from a tube. In one embodiment, the expandable member 2012 has an integrally formed proximally extending wire segment 2042 that is used to join the elongate flexible wire 2040 to the expandable member 2012. In such an embodiment, flexible wire 2040 may be joined to wire segment 2042 by the use of solder, a weld, an adhesive, or other known attachment method. In an alternative embodiment, the distal end of flexible wire 2040 is attached directly to a proximal end 2020 of the expandable member 2012.

In the example of Figure 23, expandable member 2012 includes a plurality of generally longitudinal undulating elements 2024 with adjacent undulating elements being coupled to one another in a manner to form a plurality of circumferentially-aligned cell structures 2026. The expandable member 2012 includes a proximal end portion 2013, a cylindrical main body portion 2014 and a distal end portion 2015 with the cell structures 2026 in the main body portion 2014 extending continuously and circumferentially around a longitudinal axis 2032 of the expandable member 2012. The cell structures in the proximal end portion 2013 and distal end portion 2015 extend less than circumferentially around the longitudinal axis 2032 of the expandable member 2012. The proximal wall segments 2016 of cell structures 2027, 2028, 2029 and 2030 comprise linear or substantially linear strut elements as viewed in the two dimension plane view of Figure 23. In one embodiment, the linear strut elements 2016 are aligned to form continuous and substantially linear rail segments 2017 that extend from the proximal end 2020 of proximal end portion 2013 to a proximal-most end of main body portion 2014 (again, as viewed in the two dimension plane view of Figure 23) and preferably are of the same length. As described above in conjunction with Figures 6A and 6B, rail segments 2017 are not in fact linear but are of a curved and non-undulating shape. This configuration advantageously provides rail segments 2017 devoid of undulations thereby enhancing the rail segments' ability to distribute forces and resist buckling when a push force is applied to them. In alternative preferred embodiments, the angle Θ between the wire segment 2042 or 2040, which ever the case may be, and rail segments 2017 ranges between about 140 degrees to about 150 degrees. In one embodiment the linear rail segments 2017 have a width dimension which is greater than the width dimension of the adjacent strut segments of cell structures 2027 and/or 2028 and/or 2029 and/or 2030 and/or 2026. An enhanced width of the linear rail segments 2017 further enhances the rail segments' ability to distribute forces and resist buckling when a push force is applied to the expandable member. In another implementation the linear rail segments 2017 are provided with an enhanced thickness dimension, rather than an enhanced width dimension to achieve the same or similar result. In yet an alternative implementation, both the width and thickness dimensions of the linear rail segments 2017 are enhanced to achieve the same or similar results.

In one embodiment, the width and/or thickness of the internal strut elements 2080 of proximal-most cell structure 2027 is also enhanced so as to resist buckling of these elements while the expandable member is being pushed through a sheath or delivery catheter. In one exemplary embodiment, the "as-cut" nominal widths of the enhanced strut elements 2016 and 2080 are about 0.1143 mm (0.0045 inches), while the "as-cut" nominal width of the other strut elements are about 0.0762 mm (0.003 inches).

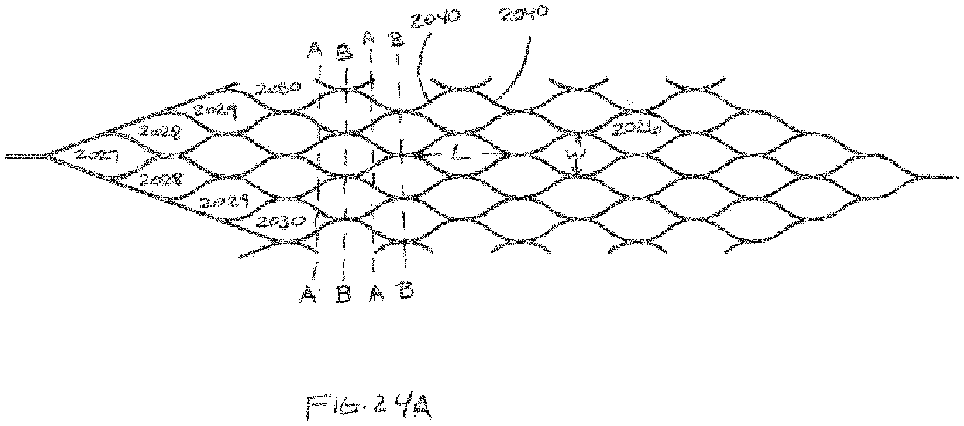

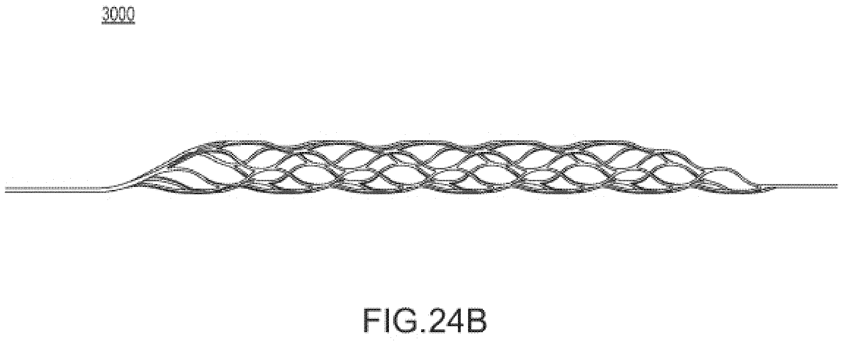

Figures 24A and 24B illustrate another example of a vascular treatment device 3000. Figure 24A depicts device 3000 in a two-dimensional plane view as if the device were cut and laid flat on a surface. Figure 24B depicts the device in its manufactured and/or expanded tubular configuration. The overall design of device 3000 is similar to the design of device 2000 depicted and described above in reference to Figure 23. The primary difference between the two designs lays in the length "L" to width "W" ratio of the cell structures 2026, 2027, 2028, 2029 and 2030. The length to width ratios of the cells structures of Figure 24A are generally greater than the length to width ratios of the respective cell structures of Figure 23. As illustrated, the lengths "L" of the cell structures of the device of Figure 24A, in the "as-cut" configuration are generally greater than the lengths of the respective cell structures of Figure 23, while the widths "W" of the cell structures of the device of Figure 24.A are generally smaller than the width of the respective cell structures of Figure 23. As a result, the slope of the individual strut elements 2040 in the cell structures of Figure 24A are generally smaller than the slopes of the respective strut elements in the cell structures of Figure 23. By reducing the slope of the strut elements 2040 and leaving the other dimensional and material characteristics constant, the effective radial force along the length of the struts 2040 is reduced. The effect of such a reduction is that the summation of axial force components along lines A-A of the device of Figure 24 more closely matches the summation of the radial force components along lines B-B as compared to the device of Figure 23. Through experimentation, the inventors have discovered that an "as-cut" cell structure length to width ratio of greater than about 2.0, and an "expanded" cell structure length to width ratio of a greater than about 1.25, advantageously resulted in a longitudinal radial force distribution along the length of the expandable member 2012 that enhanced the expandable member's ability to be pushed through and withdrawn into a lumen of a delivery catheter.

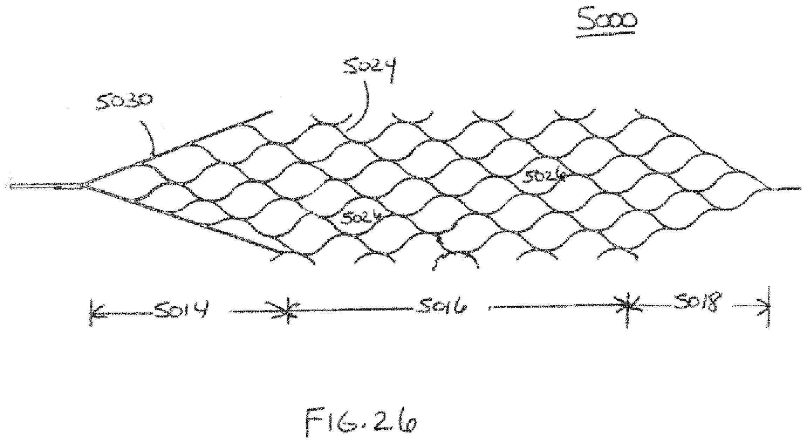

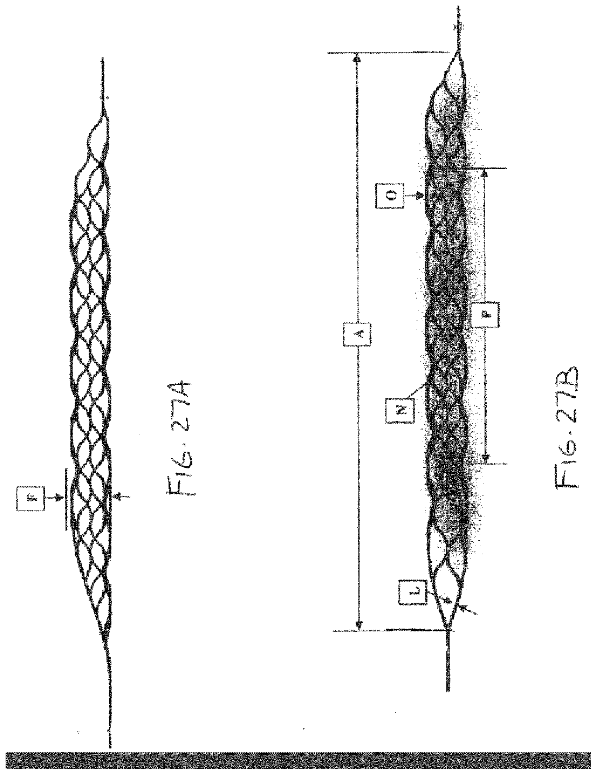

Figures 26, 27A and 27B illustrate another example of an expandable member 5000. Expandable member 5000 includes a plurality of generally longitudinal undulating elements 5024 with adjacent undulating elements being out-of-phase with one another and connected in a manner to form a plurality of diagonally disposed cell structures 5026 angularly disposed between about 40.0 to about 50.0 degrees with respect to one another. In one implementation, the cell structures are diagonally displaced along about a 45.0 degree line. The expandable member 5000 includes a proximal end portion 5014, a cylindrical main body portion 5016 and a distal end portion 5018 with the cell structures 5026 in the main body portion 5016 extending continuously and circumferentially around a longitudinal axis of the expandable member 5000. The cell structures 5026 in the proximal end portion 5014 and distal end portion 5018 extend less than circumferentially around the longitudinal axis of the expandable member 5000. In one implementation, the expandable member has an unexpanded or crimped nominal diameter of about 1.0 millimeters and a designed maximum implantable diameter of about 4.0 millimeters.

In one embodiment, expandable member 5000 has an overall length dimension A of about 36.0 ± 2.0 millimeters with the main body portion 5016 having a length P of about 19.0 ± 2.0 millimeters. In one implementation the strut width dimension N and thickness dimension O within the main body portion 5016 are about 0.0543 mm ± 0.01016 mm (0.0021 ± 0.0004 inches) and about 0.08128 mm ± 0.0127 mm (0.0032 ± 0.0005 inches), respectively, while the strut width dimension L of the proximal rails 5030 is about 0.09906 mm ± 0.01016 mm (0.0039 ± 0.004 inches).

In use, expandable member 5000 is advanced through the tortuous vascular anatomy or bodily duct of a patient to a treatment site in an unexpanded or compressed state (not shown) of a first nominal diameter and is movable from the unexpanded state to a radially expanded state of a second nominal diameter greater than the first nominal diameter for deployment at the treatment site. In alternative exemplary embodiments the first nominal diameter (e.g., average diameter of main body portion 5016) ranges between about 0.4318 mm (0.017 inches) to about 0.762 mm (0.030 inches), whereas the second nominal diameter (e.g., average diameter of main body portion 5016) is between about 2.5 to about 5.0 millimeters, in one implementation, the dimensional and material characteristics of the cell structures 5026 residing in the main body portion 5016 of the expandable material 5000 are selected to produce sufficient radial force and contact interaction to cause the cell structures 5026 to engage with an embolic obstruction residing in the vascular in a manner that permits partial or full removal of the embolic obstruction from the patient. In other embodiments the dimensional and material characteristics of the cell structures 5026 in the main body portion 5016 are selected to produce a radial force per unit length of between about 0.005 N/mm to about 0.050 N/mm, preferable between about 0.010 N/mm to about 0.050 N/mm, and more preferably between about 0.030 N/mm and about 0.050 N/mm. In one embodiment, the diameter of the main body portion 5016 in a designed fully expanded implanted state is about 4.0 millimeters with the cell pattern, strut dimensions and material being selected to produce a radial force of between about 0.030 N/mm to about 0.050 N/mm when the diameter of the main body portion is reduced to 1.5 millimeters. In the same or alternative embodiment, the cell pattern, strut dimensions and material(s) are selected to produce a radial force of between about 0.010 N/mm to about 0.020 N/mm when the diameter of the main body portion is reduced to 3.0 millimeters.

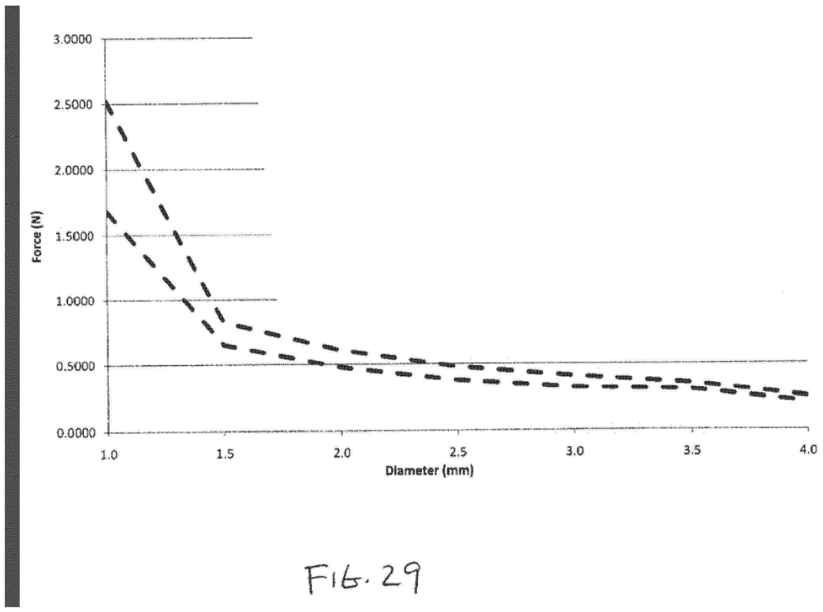

In one implementation, as shown in the graph of Figure 29, the cell structures are constructed to have dimensional and material characteristics to create an overall radial force exerted along the length of the expandable member 5000 of between about 1.50N and about 2.50N when the expandable member 5000 is in the compressed or crimped state. About a -1.5N to a about a -3.5N overall reduction in radial force along the length of the expandable member per millimeter of expansion occurs during about an initial. 0.50mm diametric range of expansion from the compressed or crimped state. Subsequent to the about 0.5mm diametric range of expansion, about a -0.10N to about a -0.50N overall reduction in radial force along the length of the expandable member per millimeter of expansion occurs until a non-zero radial force value is achieved if and when the designed maximum implanted diameter is achieved. Advantageously, the expandable member 5000 exerts a relatively high radial force during its initial expansion to enhance the likelihood that the struts of expandable member engage an obstruction within the duct of a patient upon initial deployment of the device, in addition, the rate at which the radial force diminishes is initially much greater during the initial expansion of the device than during subsequent expansion. In the example depicted by Figure 29, the initial rate of reduction in the radial force during about the first 0.5mm of expansion is about 20.0 to 30.0 times greater than the rate of reduction during subsequent expansions. An advantage of this radial force characteristic is that high radial force values can be achieved during initial deployment of the expandable member to enhance integration of the struts of the expandable member into the duct obstruction with a subsequent large reduction in radial force after the initial expansion, the large reduction facilitating or enhancing the ability of the obstruction to be removed from the duct of the patient without complications and with limited adverse interactions with the duct (e.g., less damage to the duct wall, etc.). Another advantage of the radial force characteristics depicted, in Figure 29 is that during subsequent expansions, the rate of decrease in the over radial force along the length of the expandable member decreases in a linear-like fashion at a much reduced rate providing a level predictability of the radial force being exerted at the different expandable member diameters. Also, advantageously, the radial force exerted by the expandable member is designed to achieve a non-zero value when the expandable member is at a designed maximum implantable diameter.

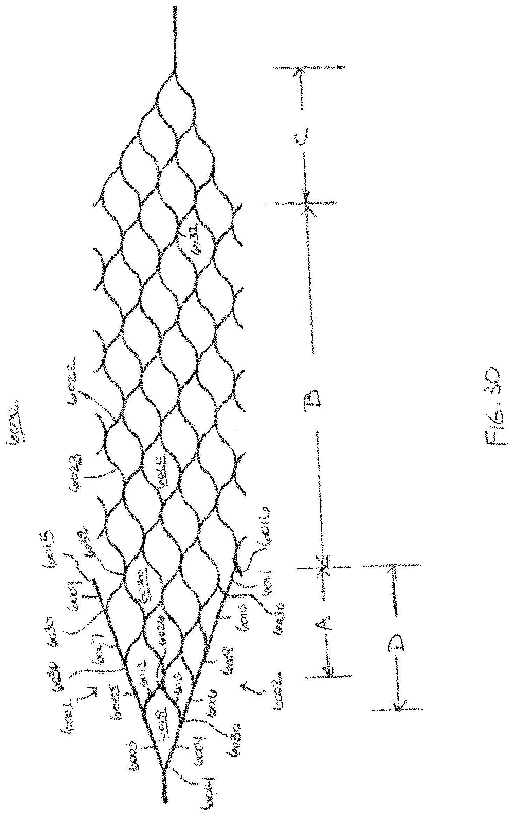

Figure 30 illustrates clot retrieval devices 6000 according to other examples where, among other features, the strut elements of rail segments 6001 and 6002 have varying width dimensions. Figure 30 depicts a clot retrieval device in a two-dimensional plane view as if the device were cut, and laid flat on a surface. Figure 30 depicts the device in its manufactured (as-cut) configuration. In one implementation, rail segment 6001 transitions from a maximum width dimension at or near its proximal end 6014 to a minimum width dimension at or near its distal end 6015. In a like manner, rail segment 6002 transitions from a maximum width dimension at or near its proximal end 6014 to a minimum width dimension at, or near its distal end 6016. As previously discussed, the width dimensions of the rail segments are selected to enhance their ability to distribute forces and to resist buckling when a push force is applied to the proximal end 6014 of the vascular treatment device. In some implementations the percentage change between the maximum rail width dimension and the minimum rail width dimension is between about 20.0% and about 50.0%. In other implementations the percentage change between the maximum rail width dimension and the minimum rail width dimension is between about 25.0% and about 45.0%. In other implementations the percentage change between the maximum rail width dimension and the minimum rail width dimension is between about 35.0% and about 45.0%. In an exemplary implementation the width dimension of the rail segments transitions from a maximum width dimension of about 0.011938 mm ± 0.01016 mm (0.0047 ± 0.0004 inches) to a minimum width dimension of about 0.06858 mm ± 0.01016 mm (0.0027 ± 0.0004 inches). In another exemplary implementation the width dimension of the rail segments transitions from a maximum width dimension of about 0.011938 mm ± 0.01016 mm (0.0047 ± 0.0004 inches) to a minimum width dimension of about 0.0889 mm ± 0.01016 mm 0.0035 ± 0.0004 inches. In another exemplary implementation the width dimension of the rail segments transitions from a maximum width dimension of about 0.011938 mm ± 0.01016 mm (0.0047 ± 0.0004 inches) to a minimum width dimension of about 0.09398 mm ± 0.01016 mm (0.0037 ± 0.0004 inches). As discussed above, post-polishing of the devices generally involve an etching process that typically results in a 40% to 50% reduction in the as-cut cross-sectional dimensions.

Although Figure 30 represents rail segments devoid of undulations, as previously described herein, it is appreciated that rail segments such as those shown in Figures 1A and 4A are also contemplated. Moreover, it is appreciated that other than the rail width characteristics disclosed above, any number of the features and/or characteristics of the vascular treatment devices previously disclosed herein with respect to Figures 1 through 29 (e.g., dimensional, spatial, relational, etc.) may be incorporated into a clot retrieval device 6000 according to Figure 30.

In some implementations the width of rails 6001 and 6002 taper along their length (or a portion thereof) in a substantial uniform and diminishing fashion. In some implementations discrete portions of the rails have a substantially uniform width dimension with transitional tapers being used to join rail portions of different widths. In some implementations discrete portions of the rails have a substantially uniform width dimension with stepped transitions between rail portions of different widths. In other implementations two or more of the preceding width transitional methods are utilized. Although not required, it is preferable that the width transitions occur at portions along the rail struts other than at a junction of the struts (e.g., junctions 6030).

In some implementations, as previously described, struts 6012 and 6013 of the most proximal cell structure 6018 also have an enhanced width dimension that may be equal to or less than the maximum rail width dimension for the purpose of enhancing the pushability of the clot retrieval device as it is advanced through the tortuous anatomy of a patient. In some implementations less than the entire length of struts 6012 and 6013 are provided with an enhanced width dimension. For example, in some implementations an enhanced width portion extends from a proximal most end of struts 6012 and 6013 and terminates a distance prior to juncture 6026. The configuration of struts 6012 and 6033 may also be altered in manners previously disclosed.

With continued reference to Figure 30, in exemplary implementations all or portions of struts 6003 and 6004 (and optionally all or the proximal portions of struts 6005 and 6006) have width dimensions between about 0.1143 mm (0.0045 inches) and about 0.127 mm (0.0050 inches), all or portions of struts 6007 and 6008 (and optionally all or the distal portions of struts 6005 and 6006) have width dimensions between about 0.0889 mm (0.0035 inches) and about 0.0914 mm (0.0036 inches), all or portions of struts 6009 and 6010 (and optionally all or the distal portions of struts 6007 and 6008) have width dimensions between about 0.06858 mm (0.0027 inches) and about 0.0889 mm (0.0035 inches), and with a substantial portion of the strut elements in the remaining portions of the device (portions A, B and C) having width dimensions between about 0.06858 mm (0.0027 inches) and about 0.08636 mm (0.0034 inches). In one or more of the immediately preceding implementations, the width dimension of struts 6012 and 6013 is between about 0.08382 mm (0.0033 inches) and about 0.12 mm (0.0047 inches), and preferably between about 0.08382 mm (0.0033 inches) and about 0.1016 mm (0.0040 inches). It is to be appreciated that the dimensions disclosed throughout this disclosure relate to exemplary implementations and are also subject to customary manufacturing tolerances. Variations in the dimensions are possible and contemplated.

Although not required, it is preferable that the width transitions occur at portions along the struts themselves other than at a junction of the struts (e.g., junctions 6030 and 6032).

In one exemplary implementation struts 6003-6006 have a width dimension of about 0.1194 mm (0.0047 inches), struts 6007, 6008 and a proximal portion of strut 6010 have a width dimension of about 0.0914 mm (0.0036 inches), struts 6009, 6011 and a distal portion of strut 6010 have a width dimension of about 0.0889 mm (0.0035 inches), struts 6012-6013 have a width dimension of about 0.0914 mm (0.0036 inches), with all or a substantial portion of the remaining strut elements of the treatment device having a width dimension of about 0.06858 mm (0.0027 inches).

Testing has shown the proximal taper region of the treatment devices of Figure 30 to possess good force transmission characteristics along with good radial force characteristics that provide good sheathing and re-sheathing of the proximal taper portion into an introducer sheath and/or delivery catheter.

In another exemplary implementation struts 6003-6006 have a width dimension of about 0.1194 mm (0.0047 inches), struts 6007, 6008 and a proximal portion of strut 6010 have a width dimension of about 0.0914 mm (0.0036 inches), struts 6009, 6011 and a distal portion of strut 6010 have a width dimension of about 0.0889 mm (0.0035 inches), struts 6012-6013 have a width dimension of about 0.0914 mm (0.0036 inches), the remaining strut elements in section A of the clot retrieval device having a width dimension of about 0.08382 mm (0.0033 inches) and the remaining strut elements generally located in sections B and C of the clot retrieval device having a width dimension of about 0.06858 mm (0.0027 inches). The increased width dimension of the struts in section A advantageously reduces the likelihood of struts buckling within the proximal taper region of the clot retrieval device and also increases the radial strength of the proximal taper region.

In another exemplary implementation struts 6003-6006 have a width dimension of about 0.1194 mm (0.0047 inches), struts 6007, 6008 and a proximal portion of strut 6010 have a width dimension of about 0.0914 mm (0.0036 inches), struts 6009, 6011 and a distal portion of strut 6010 have a width dimension of about 0.0889 mm (0.0035 inches), the remaining strut elements in section D of the treatment device having a width dimension of about 0.08382 mm (0.0033 inches) and the remaining strut elements of sections B and C of the treatment device having a width dimension of about 0.06858 mm (0.0027 inches). The increased width dimension of the struts in section A. advantageously reduces the likelihood of struts buckling within the proximal taper region of the clot retrieval device during its delivery to a treatment site of a patient and also increases the radial strength of the proximal taper region.

In another exemplary implementation struts 6003-6006 have a width dimension of about 0.1194 mm (0.0047 inches), struts 6007, 6008 and a proximal portion of strut 6010 have a width dimension of about 0.0914 mm (0.0036 inches), struts 6009, 6011 and a distal portion of strut 6010 have a width dimension of about, 0.0889 mm (0.0035 inches), struts 6012-6013 have a width dimension of about 0.0914 mm (0.0036 inches), the strut elements generally located in section C of the clot retrieval device having a width dimension of about, 0.08382 mm (0.0033 inches), and the remaining strut elements of sections A and B of the clot retrieval device having a width dimension of about 0.06858 mm (0.0027 inches). The increased width dimension of the struts in section C advantageously reduces the likelihood of struts buckling within the distal taper region of the clot retrieval device during its delivery to a treatment site of a patient. The increased width dimension also increases the radial strength of the proximal taper region that enhances the ability of the distal taper region to remain open while the clot retrieval device is withdrawn from a patient. This feature is particularly advantageous when the clot retrieval device is used for clot removal in that it enables the distal taper section to remain open and sweep away remaining portions of the clot when the clot retrieval device is being withdrawn from the patient.

According to some implementations the clot retrieval devices 6000 according to Figure 30 are laser cut from a tube having an inner diameter of about 2.667 millimeters and a wall thickness of between about 0.102 millimeters to about 0.126 millimeters. In use, a clot retrieval device 6000 according to an implementation of that shown in Figure 30 is advanced through the tortuous vascular anatomy or bodily duct of a patient to a treatment site in an unexpanded or compressed state of a first nominal diameter and is movable from the unexpanded state to a radially expanded state of a second nominal diameter greater than the first nominal diameter for deployment at the treatment site. In alternative exemplary embodiments the second nominal diameter (e.g., average diameter of main body portion) is about 4.0 ± 0.5 millimeters. In some implementation, the dimensional and material characteristics of the cell structures 6020 generally residing in the main body (section B) of the expandable material are selected to produce sufficient radial force and contact interaction to cause the cell structures 6020 to engage with an embolic obstruction/clot residing in the vascular in a manner that permits partial or full removal of the embolic obstruction from the patient.

In some implementations the dimensional and material characteristics of the elements along the expandable length of the retrieval device are selected to produce a radial force per unit length of between about 0.030 N/mm to about 0.055 N/mm when the outer diameter of the retrieval device is restrained to 1.5 millimeters. In some implementations the dimensional and material characteristics of the elements along the expandable length are selected to produce a radial force per unit length of between about 0.035 N/mm to about 0.050 N/mm when the outer diameter of the retrieval device is restrained to 1,5 millimeters. In some implementations the dimensional and material characteristics of the elements along the expandable length are selected to produce a radial force per unit length of between about 0.037 N/mm to about 0.049 N/mm when the outer diameter of the retrieval device is restrained to 1.5 millimeters. Among the same or alternative implementations, the dimensional and material characteristics of the elements along the expandable length of the retrieval device are selected to produce a radial force of between about 0.010 N/mm to about 0.020 N/mm when the nominal diameter of the main body portion is about 3.0 ± 0.5 millimeters.

In the implementations of Figure 30, many of the cell structures (excluding those that are formed at least in part by rail segments 6001 and 6002) are shown having similar shapes with most of the cell structure including a pair of short struts 6022 and a pair of long struts 6023. According to some implementations the area of the cells are about 4,00 ± 0,5 mm². In an exemplary implementation the cell areas are about 4.2 mm². In exemplary implementations, short struts 6022 have a length of between about 2.032 mm (0.080 inches) and about 2.54 mm (0.100 inches), long struts 6023 have a length of between about 3.3 mm (0.130 inches) and about 3.56 mm (0.140 inches) to produce a staggered cell arrangement about the circumference of the treatment device. In some implementations the overall length of the expandable portion of the clot retrieval device is between about 35.0 to about 45.0 millimeters with the main body portion (section B) having a length of about 20.0 to about 25.0 millimeters. In one exemplary embodiment the overall length of the expandable portion of the clot retrieval device is about 42.7 millimeters with the main body portion (section B) having a length of about 21.7 millimeters and the proximal and distal taper regions having a length of about 12.4 millimeters and about 8.6 millimeters, respectively.

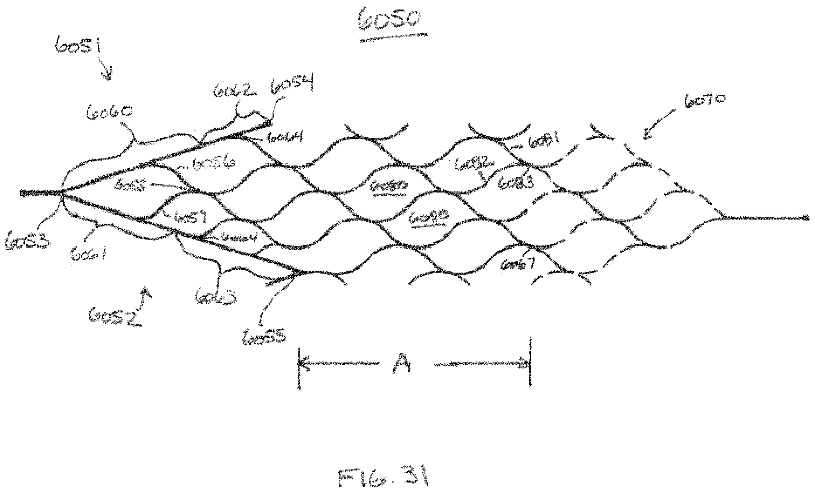

Figure 31 illustrates clot retrieval devices 6050 according to other implementations where, among other features, the strut elements of rail segments 6051 and 6052 have varying width dimensions. Clot retrieval device 6050 is particularly adapted for the treatment of small diameter vessels/duct. In one implementation, as shown in Figure 31, the circumference of the main body portion (section A) comprises three cell structures 6080, but is not limited to such a construction. Figure 31 depicts the clot retrieval treatment device 6050 in a two-dimensional plane view as if the device were cut and laid flat on a surface. Figure 31 depicts the device in its manufactured (as-cut) configuration. In one implementation, rail segment 6051 transitions from a maximum width dimension at or near its proximal end 6053 to a minimum width dimension at or near its distal end 6054. In a like manner, rail segment 6052 transitions from a maximum width dimension at or near its proximal end 6053 to a minimum width dimension at or near its distal end 6055. As previously discussed, the width dimensions of the rail segments are selected to enhance their ability to distribute forces and to resist buckling when a push force is applied to the proximal end 6053 of the vascular treatment, device. In some implementations the percentage change between the maximum rail width dimension and the minimum rail width dimension is between about 20.0% and about 30.0%, and preferably between about 20% and about 25%. In an exemplary implementation the width dimension of the rail segments transitions from a maximum width dimension of about 0.1194 mm ± 0.01016 mm (0.0047 ± 0.0004 inches) to a minimum width dimension of about 0.0914 mm ± 0.01016 mm (0.0036 ± 0.0004 inches).

Although Figure 31 represents rail segments 6053 and 6052 that are devoid of undulations,, as previously described herein, it is appreciated that rail segments such as those shown in Figures 1A and 4A are also contemplated. Moreover, it is appreciated that other than the rail width characteristics disclosed above, any number of the features and/or characteristics of the treatment devices previously described herein with respect to Figures 1 through 29 (e.g., dimensional, spatial, relational, etc.) may be incorporated into a clot retrieval device 6050 according to Figure 31.

In some implementations the width of rail 6051 and 6052 taper along their length (or a portion thereof) in a substantial uniform and diminishing fashion. In some implementations discrete portions of the rails have a substantially uniform width dimension with only transitional tapers being used to join rail portions of different widths. In some implementations discrete portions of the rails have a substantially uniform width dimension with stepped transitions between rail portions of different widths. In other implementations two or more of the preceding width transitional methods are utilized. Although not required, it is preferable that the width transitions occur at portions along the rail struts other than strut junctions (e.g., junctions 6064).

In some implementations struts 6056 and 6057 of the most proximal cell structure also have enhanced width dimensions that may be equal to or less than the maximum rail width dimension for the purpose of enhancing the pushability of the clot retrieval device as it is advanced through the tortuous anatomy of a patient. In some implementations less than the entire length of struts 6056 and 6057 are provided with an enhanced width dimension. For example, in some implementations an enhanced width portion extends from a proximal most end of struts 6056 and 6057 and terminates a distance prior to juncture 6058. Moreover, the configuration of struts 6056 and 6057 may also be altered in manners previously disclosed.

With continued reference to Figure 31, in exemplary implementations rail portions 6060 and 6061 have width dimensions of about 0.1194 mm (0.0047 inches) and rail portions 6062 and 6063 have width dimensions of about 0.0914 mm (0.0036 inches), with a substantial portion of the strut elements in the remaining portions of the device 6050 having a width dimension of about 0.0686 mm (0.0027 inches). In other exemplary implementations rail portions 6060 and 6061 have width dimensions of about 0.1194 mm (0.0047 inches) and rail portions 6062 and 6063 have width dimensions of about 0.0914 mm (0.0036 inches), with the struts in a distal portion 6070 of device (illustrated with dashed lines) having a width dimension of about 0.0584 mm (0.0023 inches) and a majority of the remaining struts having a width dimension of about 0.0686 mm (0.0027 inches). The reduced width dimension of distal portion 6070 produces a region of lower radial strength that in smaller vessels or ducts minimizes surface interactions between the distal portion 6070 and the vessel/duct to prevent or minimize the occurrence of damage to the vessel/duct wall while the clot retrieval device is proximally withdrawn from a patient.

Testing has shown the proximal taper region of the clot retrieval devices 6050 to possess good force transmission characteristics along with good radial force characteristics that provide good sheathing and re-sheathing of the proximal taper portion into an introducer sheath and/or delivery catheter.

According to some implementations the clot retrieval devices 6050 according to Figure 31 are laser cut from a tube having an inner diameter of about 2.130 millimeters and a wall thickness of between about 0.104 millimeters to about 0.128 millimeters. In use, a clot retrieval device 6050 according to an implementation of that shown in Figure 31 is advanced through the tortuous vascular anatomy or bodily duct of a patient to a treatment site in an unexpanded or compressed state of a first nominal diameter and is movable from the unexpanded state to a radially expanded state of a second nominal diameter greater than the first nominal diameter for deployment at the treatment site. In alternative exemplary embodiments the second nominal diameter (e.g., average diameter of main body portion) is about 3,0 ± 0.5 millimeters. In some implementation, the dimensional and material characteristics of the cell structures 6080 residing in the main body portion (section A) are selected to produce sufficient radial force and contact interaction to cause the cell structures 6080 to engage with an embolic obstruction residing in the vascular in a manner that, permits partial or full removal of the embolic obstruction from the patient,

In some implementations the dimensional and material characteristics of the elements along the expandable length of the retrieval device are selected to produce a radial force per unit length of between about 0.015 N/mm to about 0.035 N/mm when the outer diameter of the retrieval device is restrained to 1.5 millimeters. In some implementations the dimensional and material characteristics of the elements along the expandable length are selected to produce a radial force per unit length of between about 0.017 N/mm to about 0.033 N/mm when the outer diameter of the retrieval device is restrained to 1.5 millimeters. Among the same or alternative implementations, the dimensional and material characteristics of the elements along the expandable length of the retrieval device are selected to produce a radial force of between about 0.010 N/mm to about 0.020 N/mm when the nominal diameter of the main body portion is about 2.0 ± 0.5 millimeters.

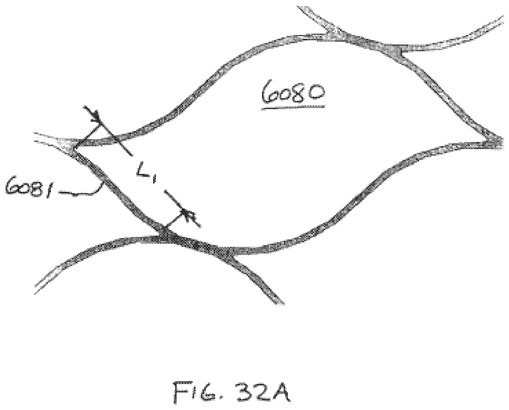

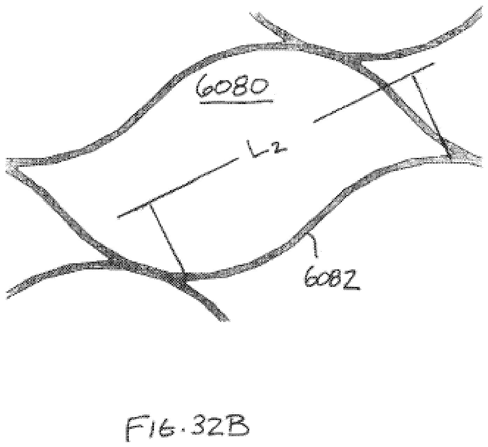

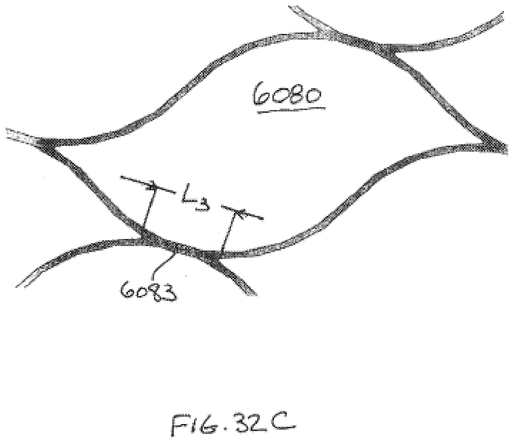

In the implementations of Figure 31, many of the cell structures (excluding those that are formed at least in part by rail segments 6051 and 6052) are shown having similar shapes with most of the cell structure including a pair of short struts 6081 and a pair of long struts 6082 that are joined by connector regions 6083. in exemplary implementations (as shown in Figures 32A-C), short struts 6081 have a linear length L₁ of about 1.397 mm ± 0.254 mm (0.055 ± 0.010 inches), long struts 6082 have a linear length, L3, of about 3.25 mm ± 0.254 mm (0.128 ± 0.010 inches) and connector regions 6083 have a linear length, L3, of about 0.94 mm ± 0.254 mm (0.0371 ± 0.010 inches). In one or more implementations the cell structures 6080 have an area of about 4.5 mm² to about 5.5 mm². In one exemplary implementation the cell structures 6080 have an area of about 5.0 mm². In exemplary implementations the overall length of the expandable portion of the clot retrieval device is between about 25.0 millimeters and about 35.0 millimeters with the main body portion (section A) having a length of between about 10.0 millimeters and about 15.0 millimeters. In one exemplary implementation the overall length of the expandable portion of the clot retrieval device is about 30.7 millimeters with the main body portion (section A) having a length of about 13.1 millimeters and the proximal and distal taper regions having a length of about 10.9 millimeters and about 6.7 millimeters, respectively.

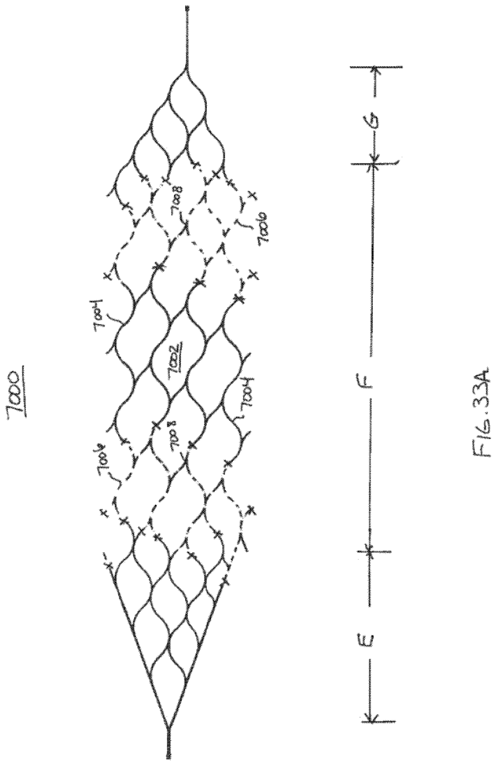

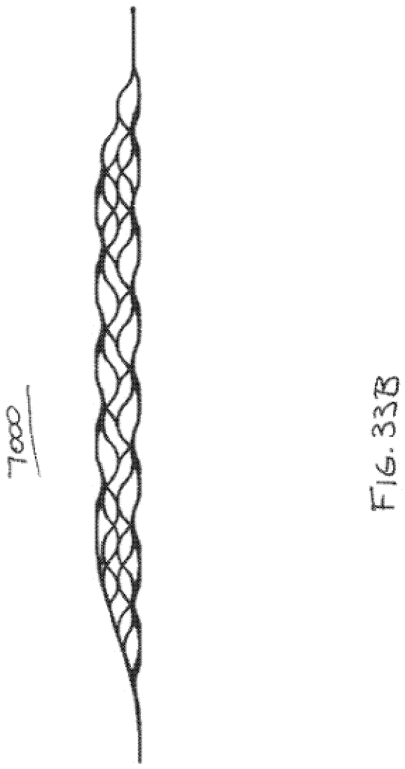

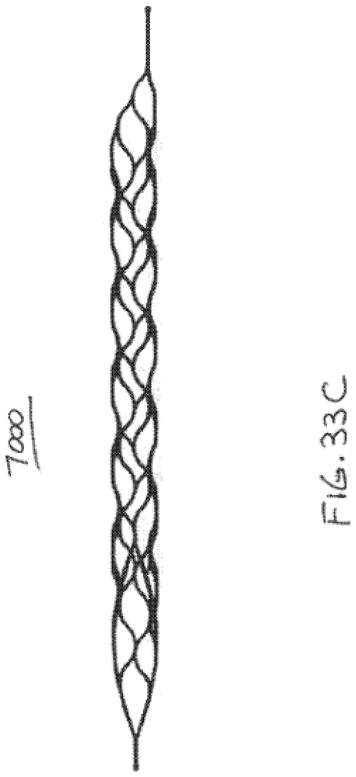

Turning now to Figure 33A, an alternative implementation to the clot retrieval devices described above in conjunction with Figure 30 is depicted. Figures 33B and 33C illustrate exemplary three-dimensional top and side views of the clot retrieval devices 7000 of Figure 33A. Sections of the treatment, device 7000 that are generally identified as regions F. and G are in many respects similar, and in some instances the same, to the same general regions of the clot retrieval devices 6000 described above. As an example, the width dimension of the struts generally located in region G may in different implementations take different values to establish any of a variety of desired distal taper characteristics as disclosed above. In addition, region E may assume any of a variety of implementations as previously disclosed above in conjunction with the retrieval devices of Figure 30. As show in Figure 33A, the sizes of the cell structures 7002 generally located in a central region F of the device 7000 are larger than those in the implementations of devices 6000 described above. An advantage of the decreased strut density in the central region F of device 7000 is that it enhances the integration of an embolic obstruction/clot within region F of the device. In the treatment devices 7000 of Figures 33, the larger cell structures are created by the omission of selected long struts 6022 in the device 6000 of Figure 30 to create cell structures 7002 having areas that are about double the size of cells 7024. In one implementation, cell structures 7020 have an area of between about 8.0 mm² and about 8.5 mm². In one exemplary implementation cell structures 7020 have an area of about 8.3 mm². It is important to note that any of a number of other methods may be used to create the larger cell structures. A particular advantage of the implementations of Figures 33 is that good strut nesting characteristics are preserved to facilitate a low profile delivery state of the device 7000.

A decrease in the strut density in a region generally results in a lower radial strength within the region. In a clot retrieval device this reduction can adversely affect the device's ability to integrate with an embolic obstruction/clot. To compensate for this reduction in radial strength, in some implementations selective strut portions 7006 (denoted by dashed lines) generally located within region F of the retrieval devices are provided with a width dimension greater than the width dimension of strut portions 7004 (denoted by solid lines). In accordance with some implementations the width dimensions of strut portions 7006 are selected so that the over-all radial strength per unit length of expandable portion of the retrieval device is similar to that absent the removal of struts to create the larger sized cell structures. As an example, in the implementations described above where decreased strut density is achieved by the omission of certain long struts 6022 in a device of Figure 30, the width of struts 7006 are selected so that the over-all radial strength per unit length of the expandable portion of the retrieval device is similar to that of devices 6000 described above. For example, in some implementations strut portions 7004 have a width dimension of about 0.06858 mm (0.0027 inches) with strut portions 7006 having a width dimension of about 0.0889 mm (0.0035 inches) so that the over-all radial strength per unit length of the expandable portion is similar to the same area of the retrieval devices 6000 having mostly unitary cell sizes and strut width dimensions of about 0.06858 mm (0.0027 inches).

Although not required, as illustrated in Figure 33A, the transition of strut widths preferably occur at locations (denoted by "x") other than junctions 7008. Although not required, the width transitions preferably comprise tapers that provide a relatively smooth transition between the different, width dimensions.

Strut portions of enhanced width 7006 are one method of creating a desired over-all radial strength per unit length. Other methods are also available. For example, strut portions 7006 may instead have an enhanced thickness dimension over strut portions 7004, or may have a combination of enhanced thickness and width dimensions. In other implementations the width dimension of a majority, substantially all or all of the struts generally located in section F are enhanced to compensate for the reduction in strut density.

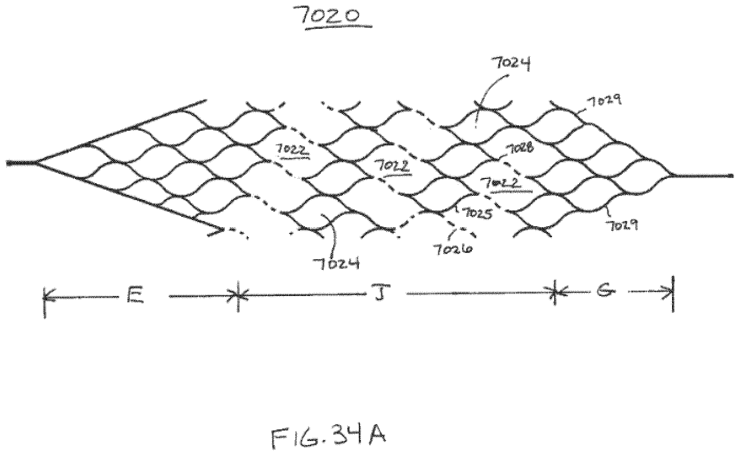

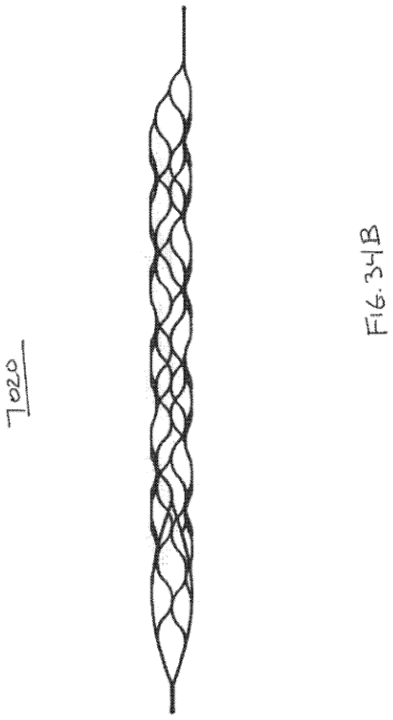

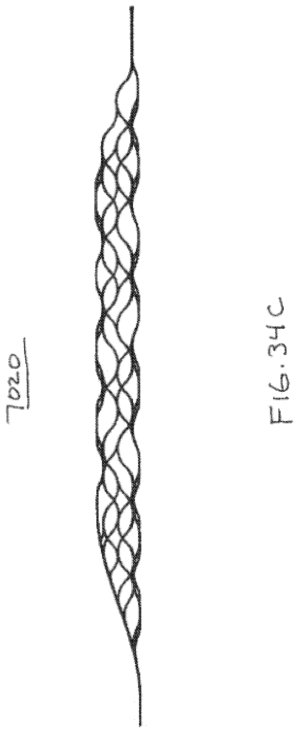

With reference to Figure 34A, alternative implementations to the clot retrieval devices described above in conjunction with Figure 30 are depicted. Figures 34B and 34C illustrate exemplary three-dimensional top and side views of the clot retrieval devices 7020 of Figure 34A. Sections of the treatment device 7020 that are generally identified as regions E and G are in many respects similar, and in some instances the same, to the same general regions of the clot retrieval devices 6000 described above. As an example, the width dimension of the struts in region G may, in different implementations, take different values to establish any of a variety of desired distal taper characteristics as disclosed above. In addition, region E may assume any of a variety of implementations as previously disclosed above in conjunction with the retrieval devices of Figure 30. As shown in Figure 34A, the sizes of some of the cell structures 7022 in a central region J of the device 7020 are larger than those in the implementations of devices 6000 described above to provide circumferentially extending zones of decreased strut density that are generally separated by circumferentially extending rows of non-enlarged cell structures 7024, In the treatment devices 7020 of Figures 34, the larger cell structures are created by the omission of selected long struts 6022 in the device 6000 of Figure 30 to create cell structures 7022 having areas of about, double in size. In one implementation cell structures 7022 have an area of about 8.3 mm². It is important to note that any of a number of other methods may be used to create the larger cell structures. A particular advantage of the implementations of Figures 34 is that good strut nesting characteristics are preserved to facilitate a low profile delivery state of the device 7020.

As discussed above, a decrease in the strut density in a region generally results in a lower radial strength within the region. In a clot retrieval device this reduction can adversely affect the device's ability to integrate with an embolic obstruction/clot. To compensate for this reduction in radial strength, selective strut portions 7026 (denoted by dashed lines) generally located within region J of the retrieval devices are provided with a width dimension greater than the width dimension of strut portions 7025 (denoted by solid lines). In accordance with some implementations the width dimensions of strut portions 7026 are selected so that the over-all radial strength per unit length of the expandable portion of the retrieval de vice is similar to that absent the removal of struts to create the larger sized cell structures. As an example, in the implementations described above where decreased strut density is achieved by the omission of certain long struts 6022 in a device of Figure 30, the width of struts 7026 are selected so that the over-all radial strength per unit length of the expandable portion of the retrieval device is similar to that of devices 6000 described above. For example, in some implementations strut portions 7025 have a width dimension of about 0.06858 mm (0.0027 inches) with strut portions 7026 having a width dimension of about 0.0889 mm (0.0035 inches) so that the over-all radial strength per unit length of the expandable portion of the retrieval device is similar to the same area of the retrieval devices 6000 having mostly unitary cell sizes and strut width dimensions of about 0.06858 mm (0.0027 inches). In some implementation the width of the struts 7029 have a width dimension of between 0.07874 mm (0.0031 inches) and about 0.08383 mm (0.0033 inches) similar to those previously discussed above with respect to some implementations of device 6000.

In some implementations, as illustrated in Figure 34A, the transition of some or all of the strut widths occur at locations other than junctions 7028, while in other implementations the transition of some or al of the strut widths occur at locations other than junctions 7028. Although not required, the width transitions preferably comprise tapers that provide a relatively smooth transition between the different width dimensions.

Strut portions of enhanced width 7026 are one method of creating in region J a desired overall radial strength. Other methods are also available. For example, strut portions 7026 may instead have an enhanced thickness dimension over strut portions 7025, or may have a combination of enhanced thickness and width dimensions.

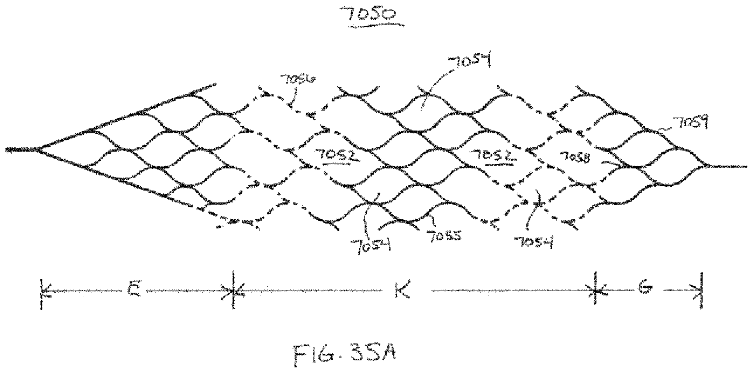

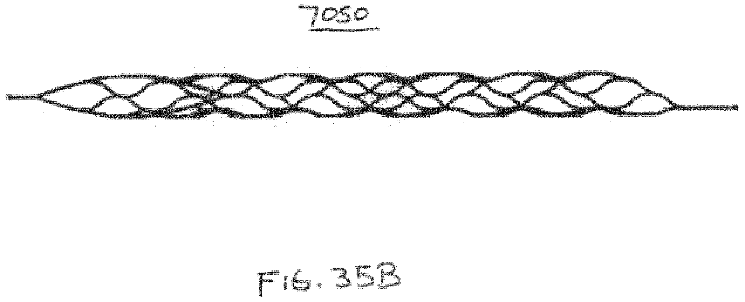

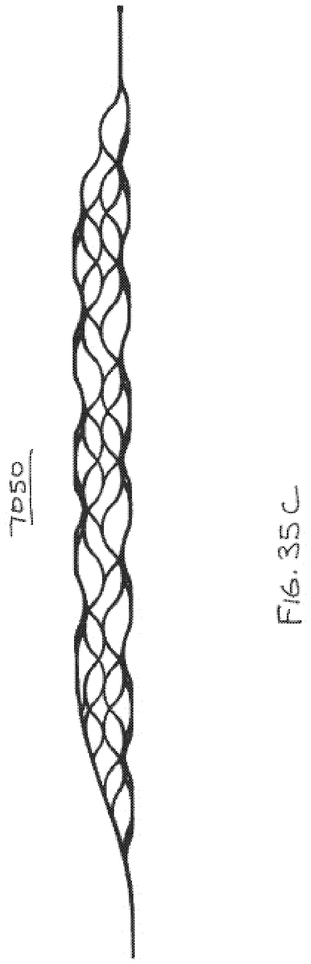

With reference to Figure 35A, an alternative implementation to the clot retrieval devices described above in conjunction with Figure 30 is depicted. Figures 35B and 35C illustrate exemplary three-dimensional top and side views of the clot retrieval devices 7050 of Figure 35A. Sections of the treatment device 7050 that are generally identified as regions E and G are in many respects similar, and in some instances the same, to the same general regions of the clot retrieval devices 6000 described above. As an example, the width dimension of the struts generally located in region G may in different implementations take different values to establish any of a variety of desired distal taper characteristics as disclosed above. In addition, region E may assume any of a variety of implementations as previously disclosed above in conjunction with the retrieval devices of Figure 30. As shown in Figure 35A, the sizes of some of the cell structures 7052 in a central region K of the device 7050 are larger than those in the implementations of devices 6000 described above to provide zones of decreased strut density that are dispersed among non-enlarged cell structures 7054. In the treatment devices 7050 of Figures 35, the larger cell structures are created by the omission of selected long struts 6022 in the device 6000 of Figure 30 to create cell structures 7052 having areas of about double the size of cells 7054. In one implementation the area of cell structures 7052 is about 8.3 mm². It is important to note that any of a number of other methods may be used to create the larger cell structures. A particular advantage of the implementations of Figures 35 is that good strut nesting characteristics are preserved to facilitate a low profile delivery state of the device 7050.

As discussed above, a decrease in the strut density in a region generally results in a lower radial strength within the region. In a clot retrieval device this reduction can adversely affect the device's ability to integrate with an embolic obstruction/clot. To compensate for this reduction in radial strength, selective strut portions 7056 (denoted by dashed lines) generally located within region K of the retrieval devices are provided with a width dimension greater than the width dimension of strut portions 7055 (denoted by solid lines). In accordance with some implementations the width dimensions of strut, portions 7056 are selected so that the over-all radial strength per unit length of the expandable portion of the retrieval device is similar to that absent the removal of struts to create the larger sized cell structures. As an example, in the implementations described above where decreased strut density is achieved by the omission of certain long struts 6022 in a device of Figure 30, the width of struts 7056 are selected so that the over-all radial strength per unit length of the expandable portion of the retrieval device is similar to that of devices 6000 described above. For example, in some implementations strut portions 7055 have a width dimension of about 0.06858 mm (0.0027 inches) with strut portions 7056 having a width dimension of about 0.0889 mm (0.0035 inches) so that the over-all radial strength per unit length of the expandable portion of the retrieval device is similar to the same area of the retrieval devices 6000 having mostly unitary cell sizes and strut width dimensions of about, 0.06858 mm (0.0027 inches). In some implementations the width of the struts 7059 have a width dimension of between 0.07874 mm (0.0031 inches) and about 0.08383 mm (0.0033 inches) similar to those previously discussed above with respect to some implementations of device 6000.

Although not required, as illustrated in Figure 35A, the transition of strut widths preferably occur at locations other than junctions 7058. Although not required, the width transitions preferably comprise tapers that provide a relatively smooth transition between the different width dimensions.

Strut portions of enhanced width 7056 are one method of creating a desired over-all radial strength per unit length. Other methods are also available. For example, strut portions 7056 may instead have an enhanced thickness dimension over strut portions 7055, or may have a combination of enhanced thickness and width dimensions.

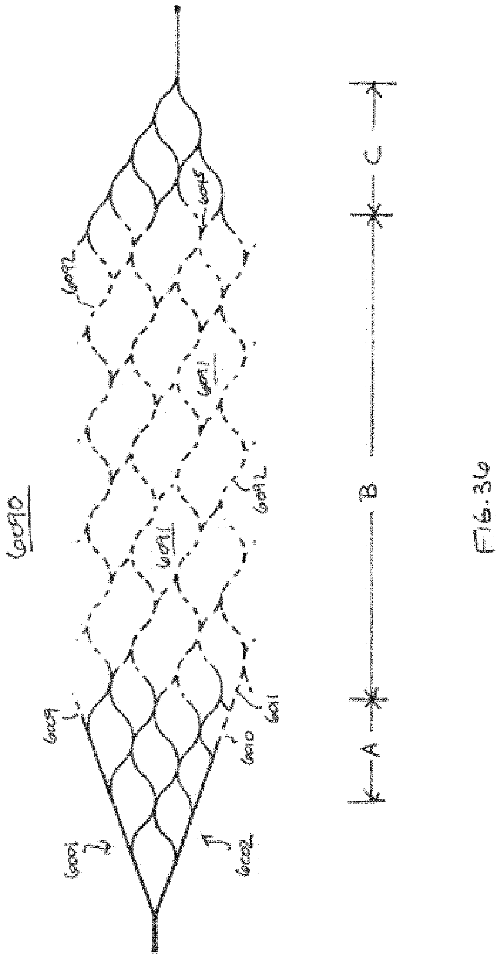

Figure 36 illustrates clot retrieval devices 6090 similar to those of Figure 30, with a difference in the size of the cell structures 6091 generally located in region B of the device. As illustrated in Figure 36, cell structures 6091 are of a greater size than the cell structures 6020 of the device shown in Figure 30. As previously discussed, an advantage of larger sized cell structures within the main body portion of the retrieval device is that it enhances clot integration into the main body portion when a radial strength of the main body portion is property provided. For the purpose of providing sufficient radial strength in region B of the device 6090, the struts 6092 (denoted by dashed lines) generally located within region B have an enhanced width dimension, which in one implementation is about 0.0889 mm (0.0035 inches). In one implementation the width dimension of the struts 6092 generally located in region B are similar to or the same as the width dimension of the distal sections of rail segments 6001 and/or 6002 (e.g., having the same or similar width dimension of one or more of struts 6009, 6010 and 6011). Although not required, the transition in width dimensions preferably occur at locations other than at junctions 6045, as illustrated in Figure 36.

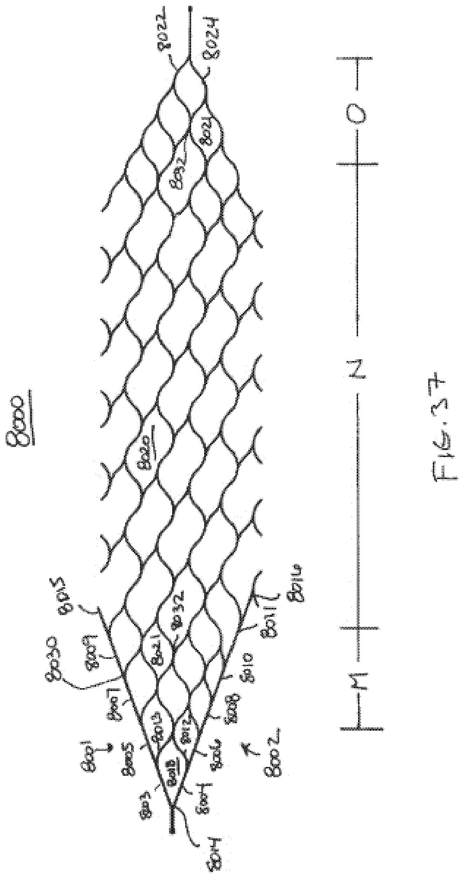

Figure 37 illustrates clot retrieval devices 8000 according to other implementations where, among other features, the strut elements of rail segments 8001 and 8002 have varying width dimensions. Figure 37 depicts a clot retrieval device in a two-dimensional plane view as if the device were cut and laid flat, on a surface. Figure 37 depicts the device in its manufactured (as-cut) configuration. In one implementation, rail segment 8001 transitions from a maximum width dimension at or near its proximal end 8014 to a minimum width dimension at or near its distal end 8015. In a like manner, rail segment 8002 transitions from a maximum width dimension at or near its proximal end 8014 to a minimum, width dimension at, or near its distal end 8016. As previously discussed, the width dimensions of the rail segments are selected to enhance their ability to distribute forces and to resist buckling when a push force is applied to the proximal end 8014 of the clot retrieval device. In some implementations the percentage change between the maximum rail width dimension and the minimum rail width dimension is between about 20.0% and about 35.0%. In other implementations the percentage change between the maximum rail width dimension and the minimum rail width dimension is between about 25.0% and about 30.0%. In an exemplary implementation the width dimension of the rail segments transitions from a maximum width dimension of about 0.1194 mm ± 0.01016 mm (0.0047 ± 0.0004 inches) to a minimum width dimension of about 0.06858 mm ± 0.01016 mm (0.0027 ± 0.0004 inches). In another exemplary implementation the width dimension of the rail segments transitions from a maximum width dimension of about 0.1194 mm ± 0.01016 mm (0.0047 ± 0.0004 inches) to a minimum width dimension of about 0.08636 mm ± 0.01016 mm (0.0034 ± 0.0004 inches).

Although Figure 37 represents rail segments that are devoid of undulations, as previously described herein, it is appreciated that rail segments such as those shown in Figures 1A and 4A are also contemplated. Like the devices of Figure 30 disclosed above, it is appreciated that other than the rail width characteristics disclosed in the preceding paragraph, any of a number of the features and/or characteristics of the vascular treatment devices described in conjunction with the devices of Figures 1 -29 (e.g., dimensional, spatial, relational, etc.) may be incorporated into a clot retrieval device 8000 according to Figure 37.

In some implementations the width of rails 8001 and 8002 taper along their length (or a portion thereof) in a substantial uniform and diminishing fashion. In some implementations discrete portions of the rails have a substantially uniform width dimension with only transitional tapers being used to join rail portions of different widths. In some implementations discrete portions of the rails have a substantially uniform width dimension with stepped transitions between rail portions of different widths. In other implementations two or more of the preceding width transitional methods are utilized. Although not required, it is preferable that the width transitions occur at portions along the rail struts other than at a junction of the struts (e.g., junctions 8030).

In some implementations, as previously described, struts 8012 and 8013 of the most proximal cell structure 8018 also have an enhanced width dimension that may be equal to or less than the maximum, rail width dimension for the purpose of enhancing the pushability of the clot retrieval device as it is advanced through the tortuous anatomy of a patient. In some implementations less than the entire length of struts 8012 and 8013 are provided with an enhanced width dimension. For example, in some implementations an enhanced width portion extends from a proximal most end of struts 8012 and 8013 and terminates a distance prior to their juncture. The configuration of struts 8012 and 8013 may also be altered in manners previously disclosed.

With continued reference to Figure 37, in exemplary implementations all or portions of struts 8003 and 8004 (and optionally all or portions of struts 8005 and 8006) have width dimensions of about 0.1143 mm (0.0045 inches) to about 0.127 mm (0.0050 inches), all or portions of struts 8007 and 8008 (and optionally all or portions of struts 8005 and 8006) have width dimensions of about 0.0914 mm (0.0036 inches) to about 0.1194 mm (0.0040 inches), all or portions of struts 8009 and 8010 (and optionally all or portions of struts 8007 and 8008) have width dimensions of about 0.08636 mm (0.0034 inches) to about 0.0914 mm (0.0036 inches). In some implementations the remainder of the struts generally located in region M of the device have width dimensions of about 0.06858 mm (0.0027 inches), the struts in region N have width dimensions of about 0.08636 mm (0.0034 inches) to about 0.0914 mm (0.0036 inches), and the struts generally located in region O have a width dimension of about 0.07874 mm (0.0031 inches) to about 0.8382 mm (0.033 inches). In one or more of the immediately preceding implementations, the width dimension of struts 8012 and 8013 is between about 0.0914 mm (0.0036 inches) and about 0.1194 mm (0.0047 inches). It is to be appreciated that the dimensions disclosed relate to exemplary implementations and are also subject to customary manufacturing tolerances. Variations in the dimensions are also possible and contemplated.

Although not required, it is preferable that the width transitions occur at portions along the struts themselves other than at, a junction of the struts (e.g., junctions 8030 and 8032).

As illustrated in Figure 37, the strut density in the region generally identified by "N" is notably less than the strut densities in the regions generally identified by "M" and "O". As a consequence, the cell structures 8020 generally located in region N are of a larger size than the cell structures 8021 generally located in regions N and O. As previously discussed, an advantage of larger sized cell structures within the main bod}' portion of the retrieval device is that it enhances clot integration into the main body portion (region N) when a radial strength of the main body portion is properly provided. For the purpose of providing sufficient, radial strength in region N of the device, the struts within region have an enhanced width dimension as compared to the cell struts generally residing in region M (other than the struts 8003-8013) and the cell struts generally residing in region O. In one implementation the width dimension of the struts in region N are similar to or the same as the width dimension of the distal struts 8009, 8010 and/or 8011 of rail segments 8001 and/or 8002.

In an exemplary implementation struts 8003-8006 have a width dimension of about 0.1194 mm (0.0047 inches), struts 8007, 8008, and a proximal portion of strut 8010 have a width dimension of about 0.1194 mm (0.0040 inches), struts 8009, 803 3 and a distal portion of strut 8010 have a width dimension of about 0.08636 mm (0.0034 inches), struts 8032-8013 have a width dimension of about 0.1194 mm (0.0040 inches). In some implementations the remainder of the struts in region M of the device have width dimensions of about 0.06858 mm (0.0027 inches), the struts in region N have width dimensions of about 0.08636 mm (0.0034 inches), and the struts in region O have a width dimension of about 0.07874 mm (0.0031 inches). The increased width dimension of the struts in section O advantageously reduces the likelihood of struts buckling within the distal taper region of the clot retrieval device during its delivery to a treatment site of a patient. The increased width dimension also increases the radial strength of the distal taper region that enhances the ability of the distal taper region to remain open while the clot retrieval device is withdrawn from a patient so that it may sweep away remaining portions of the clot when the clot retrieval device is being withdrawn from the patient.

According to some implementations the clot retrieval devices 8000 according to Figure 37 are laser cut from a tube having an inner diameter of about 3.77 millimeters and a wail thickness of between about 0.097 millimeters to about 0.131 millimeters. In use, a clot retrieval device 8000 according to an implementation of that shown in Figure 37 is advanced through the tortuous vascular anatomy or bodily duct of a patient to a treatment site in an unexpanded or compressed state of a first nominal diameter and is movable from the unexpanded state to a radially expanded state of a second nominal diameter greater than the first nominal diameter for deployment at the treatment site. In alternative exemplary embodiments the second nominal diameter (e.g., average diameter of main body portion) is about 5.5 ± 0.5 millimeters. In some implementation, the dimensional and material characteristics of the cell structures 8020 residing in the main bod}' (section N) are selected to produce sufficient radial force and contact interaction to cause the cell structures 8020 to engage with an embolic obstruction/clot residing in the vascular in a manner that permits partial or full removal of the embolic obstruction from the patient.

In some implementations the dimensional and material characteristics of the elements along the expandable length of the retrieval device are selected to produce a radial force per unit length of between about 0.040 N/mm to about 0.065 N/mm when the outer diameter of the retrieval device is restrained to 1.5 millimeters. In some implementations the dimensional and material characteristics of the elements along the expandable length are selected to produce a radial force per unit length of between about 0.045 N/mm to about 0.060 N/mm when the outer diameter of the retrieval device is restrained to 1.5 millimeters. In some implementations the dimensional and material characteristics of the elements along the expandable length are selected to produce a radial force per unit length of between about 0.050 N/mm to about 0.060 N/mm when the outer diameter of the retrieval device is restrained to 1.5 millimeters. In some implementations the dimensional and material characteristics of the elements along the expandable length are selected to produce a radial force per unit length of between about 0.049 N/mm to about 0.057 N mm when the outer diameter of the retrieval device is restrained to 1.5 millimeters. Among the same or alternative implementations, the dimensional and material characteristics of the elements along the expandable length of the retrieval device are selected to produce a radial force of between about 0.010 N/mm to about 0.020 N/mm when the nominal diameter of the main body portion is about 4.5 ± 0.5 millimeters.

In the implementations of Figure 37, the cell structures in regions M and O (excluding those that are formed at least in part by rail segments 8001 and 8002) are shown having similar shapes with the cell structures 8021 including a pair of short struts 8022 and a pair of long struts 8024. In exemplary implementations the area of cell structures 8021 is between about 4.5 mm² and about 5.5 mm². In one exemplary implementation the area of cell structures 8021 is about 5.0 mm² to about 5.2 mm². The cell structures 8020 generally located in region N, in one implementation, comprise a shape consisting of two adjoining cell structures 8021 with a long strut 8024 being omitted between them. Although other types of large sized cell structures are contemplated, an advantage of the cell construction illustrated in Figure 37 is that it, possesses good nesting capability to permit the retrieval device to achieve a small delivery profile.

In some implementations the overall length of the expandable portion of the clot retrieval device is between about 55.0 millimeters and about 65.0 millimeters with the main body portion (section N) having a length of between about 25 millimeters and about 35.0 millimeters and the proximal and distal taper regions having a length of between about 10.0 to about 20.0 millimeters. In one exemplary embodiment the overall length of the expandable portion of the clot retrieval device is about 58.4 millimeters with the main body portion (section N) having a length of about 29.3 millimeters and the proximal and distal taper regions having a length of about 16.6 millimeters and 12.5 millimeters, respectively.

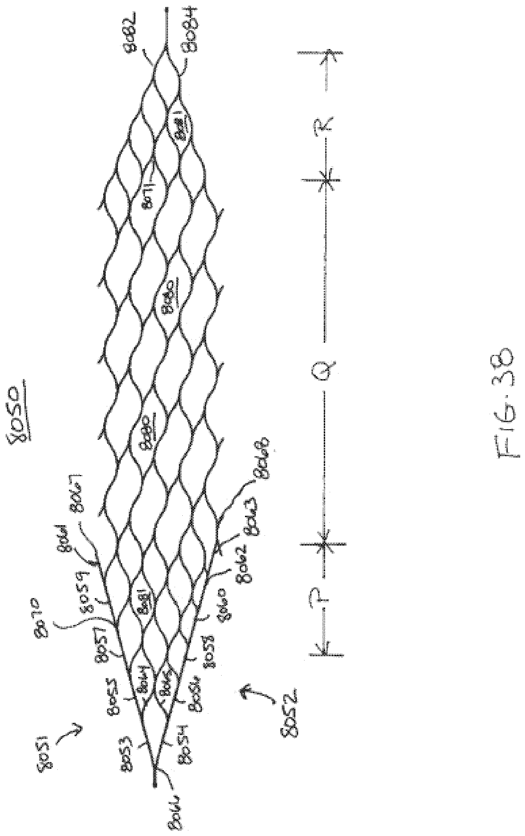

Figure 38 illustrates clot retrieval devices 8500 according to other implementations where, among other features, the strut elements of rail segments 8051 and 8052 have varying width dimensions. Figure 38 depicts a clot retrieval device in a two-dimensional plane view as if the device were cut and laid flat on a surface. Figure 38 depicts the device in its manufactured (as-cut) configuration. In one implementation, rail segment 8051 transitions from a maximum width dimension at or near its proximal end 8066 to a minimum width dimension at or near its distal end 8067. In a like manner, rail segment 8052 transitions from a maximum width dimension at or near its proximal end 8066 to a minimum width dimension at or near its distal end 8068. As previously discussed, the width dimensions of the rail segments are selected to enhance their ability to distribute forces and to resist buckling when a push force is applied to the proximal end 8064 of the clot retrieval device. In some implementations the percentage change between the maximum rail width dimension and the minimum rail width dimension is between about 20.0% and about 35.0%. In other implementations the percentage change between the maximum rail width dimension and the minimum rail width dimension is between about 22.0% and about 27.0%. In an exemplary' implementation the width dimension of the rail segments transitions from a maximum width dimension of about 0.1194 mm ± 0.01016 mm (0.0047 ± 0.0004 inches) to a minimum width dimension of about 0.0035 ± 0.01016 mm (± 0.0004 inches).

Although Figure 38 represents rail segments that are devoid of undulations, as previously described herein, it is appreciated that rail segments such as those shown in Figures 1A and 4A are also contemplated. Like the devices of Figure 30 disclosed above, it is appreciated that other than the rail width characteristics disclosed in the preceding paragraph, any of a number of the features and/or characteristics of the vascular treatment devices described in conjunction with the devices of Figures 1-29 (e.g., dimensional, spatial, relational, etc.) may be incorporated into a clot retrieval device 8050 according to Figure 38.

In some implementations the width of rails 8051 and/or 8052 taper along their length (or a portion thereof) in a substantial uniform and diminishing fashion. In some implementations discrete portions of the rails have a substantially uniform width dimension with only transitional tapers being used to join rail portions of different widths. In some implementations discrete portions of the rails have a substantially uniform width dimension with stepped transitions between rail portions of different widths. In other implementations two or more of the preceding width transitional methods are utilized. Although not required, it is preferable that the width transitions occur at portions along the rail struts other than at a junction of the struts (e.g., junctions 8070).

In some implementations, in a manner previously described, struts 8064 and 8065 of the most proximal cell structure also have an enhanced width dimension that may be equal to or less than the maximum rail width dimension for the purpose of enhancing the pushability of the clot retrieval device as it is advanced through the tortuous anatomy of a patient. In some implementations less than the entire length of struts 8064 and 8065 are provided with an enhanced width dimension. For example, in some implementations an enhanced width portion extends from a proximal most end of struts 8064 and 8065 and terminates a distance prior to their juncture. The configuration of struts 8064 and 8065 may also be altered in manners previously disclosed.

With continued reference to Figure 38, in exemplary implementations all or portions of struts 8053 and 8054 (and optionally all or portions of struts 8055 and 8056) have width dimensions of about 0.1143 mm (0.0045 inches) to about 0.127 mm (0.0050 inches), all or portions of struts 8057 and 8058 (and optionally all or portions of struts 8055, 8056, 8059 and 8060) have width dimensions of about 0.0914 mm (0.0036 inches) to about 0.1194 mm (0.0040 inches), all or portions of struts 8059 and 8060 (and optionally all or portions of struts 8061, 8062 and 8063) have width dimensions of about 0.08636 mm (0.0034 inches) to about 0.0914 mm (0.0036 inches). In some implementations the remainder of the struts generally located in region P of the device have width dimensions of about 0.06858 mm (0.0027 inches), the struts generally located in region Q have width dimensions of about 0.08636 mm (0.0034 inches) to about 0.0914 mm (0.0036 inches), and the struts generally located in region R have a width dimension of about 0.07874 mm (0.0031 inches) to about 0.8382 mm (0.033 inches). In one or more of the immediately preceding implementations, the width dimension of struts 8064 and 8065 is between about 0.0914 mm (0.0036 inches) and about 0.1194 mm (0.0047 inches). It is to be appreciated that the dimensions disclosed relate to exemplary implementations and are also subject to customary manufacturing tolerances. Variations in the dimensions are possible and contemplated.

Although not required, it is preferable that the width transitions occur at portions along the struts themselves other than at, a junction of the struts (e.g., junctions 8070 and 8071).

As illustrated in Figure 38, the strut density in the region generally identified by "Q" is notably less than the strut densities in the regions generally identified by "P" and "R". As a consequence, the cell structures 8080 generally located in region Q are of a larger size than the cell structures 8081 generally located in regions P and R. As previously discussed, an advantage of larger sized cell structures within the main body portion of the retrieval device is that it enhances clot integration into the main body portion (region Q) when a radial strength of the main body portion is properly provided. For the purpose of providing sufficient radial strength in region Q of the device, the struts generally located within region Q have an enhanced width dimension as compared to the cell struts generally located in region P (other than the struts 8053-8065) and the cell struts generally located in region R. In one implementation the width dimension of the struts in region Q are similar to or the same as the width dimension of the distal sections of rails 8051 and 8052 (e.g., struts 8061, 8062 and/or 8063).

In an exemplary implementation struts 8003-8006 and a proximal portion of struts 8055 and 8056 have a width dimension of about 0.1194 mm (0.0047 inches), struts 8057, 8058, and a distal and proximal portions of struts 8055,8056 and 8059,8060, respectively, have a width dimension of about 0.1016 mm (0.0040 inches), struts 8009, 8011 and a distal portion of strut 8010 have a width dimension of about 0.08636 mm (0.0034 inches), struts 8012-8013 have a width dimension of about 0.1194 mm (0.0040 inches), struts 8061, 8062, 8063 and the distal portions of struts 8059 and 8060 have a width dimension of about 0.0889 mm (0.0035 inches). In some implementations the remainder of the struts generally located in region P of the device have width dimensions of about 0.06858 mm (0.0027 inches), the struts generally located in region Q have width dimensions of about 0.0889 mm (0.0035 inches), and the struts generally located in region R have a width dimension of about 0.07874 mm (0.0031 inches). The increased width dimension of the struts in section R advantageously reduces the likelihood of struts buckling within the distal taper region of the clot retrieval device during its delivery to a treatment site of a patient. The increased width dimension al so increases the radial strength of the distal taper region that enhances the ability of the distal taper region to remain open while the clot retrieval device is withdrawn from a patient, so that it may sweep away remaining portions of the clot when the clot retrieval device is being withdrawn from the patient.

According to some implementations the clot retrieval devices 8050 according to Figure 38 are laser cut from a tube having an inner diameter of about 3.77 millimeters and a wall thickness of between about 0.097 millimeters to about 0.131 millimeters. In use, a clot retrieval device 8050 according to an implementation of that shown in Figure 38 is advanced through the tortuous vascular anatomy or bodily duct of a patient to a treatment, site in an unexpanded or compressed state of a first nominal diameter and is movable from the unexpanded state to a radially expanded state of a second nominal diameter greater than the first nominal diameter for deployment at the treatment site. In alternative exemplary embodiments the second nominal diameter (e.g., average diameter of main body portion) is about 6.0 ± 0.5 millimeters. In some implementation, the dimensional and material characteristics of the cell structures 8080 residing in the main body (section Q) are selected to produce sufficient radial force and contact interaction to cause the cell structures 8080 to engage with an embolic obstruction/clot residing in the vascular in a manner that permits partial or full removal of the embolic obstruction from the patient. In some implementation, the dimensional and material characteristics are selected to produce a radial force per unit length in the expandable portion of the retrieval device of between about 0.010 N/mm to about 0.020 N/mm when the diameter of the main body portion is reduced to about 5.0 ± 0.5 millimeters.

In the examples of Figure 38, the cell structures generally located in regions P and R (excluding those that are formed at least in part by rail segments 8051 and 8052) are shown having similar shapes with the cell structures 8081 including a pair of short struts 8082 and a pair of long struts 8084. In an exemplary implementation the area of cell structures 8081 is about 9.2 mm². The cell structures 8080 generally located in region Q, in one implementation, comprise a shape consisting of two adjoining cell structures 8081 with a long strut 8084 being omitted between them. Although other types of large sized cell structures are contemplated, an advantage of the cell construction illustrated in Figure 38 is that it possesses good nesting capability to permit the retrieval device to achieve a small delivery profile.

In some implementations the overall length of the expandable portion of the clot retrieval device is between about 65,0 millimeters and about 75.0 millimeters with the main body portion (section Q) having a length of between about 25.0 millimeters and about 35.0 millimeters. In one exemplary implementation the overall length of the expandable portion of the clot retrieval device is about 71.9 millimeters with the main body portion (section Q) having a length of about 32.3 millimeters and the proximal and distal taper regions having a length of about 22.5 millimeters and 17.1 millimeters, respectively.

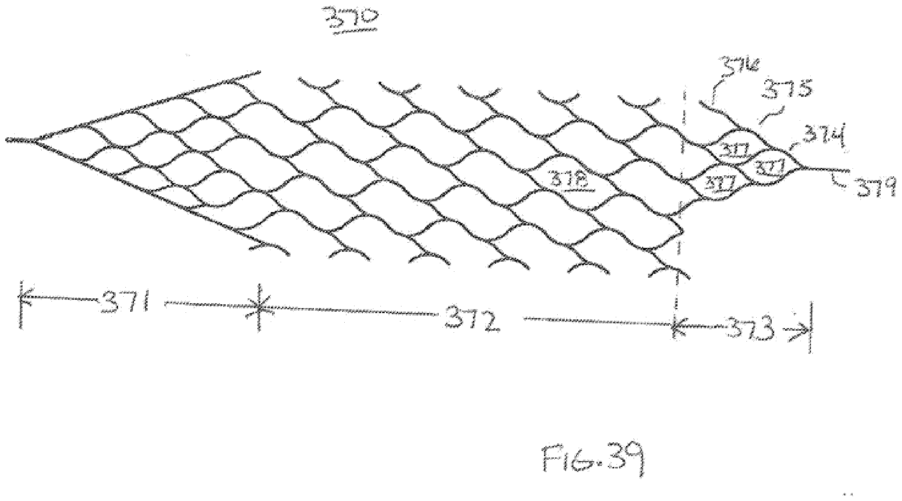

Figure 39 depicts a two dimensional view of another example of a duct obstruction retrieval device 370. As with some of the other implementations previously described, the retrieval device 370 comprises a proximal tapered end portion 371, a cylindrical main body portion 372 and a distal tapered end portion 373. A difference in the distal tapered end portion 373 as compared to the distal tapered end portions previously described is that the distal tapered end portion 373 has less than three full rows of cell structures so as to reduce the distal taper length. In the example of Figure 39 the distal tapered end portion comprises two full rows of cell structures 374 and 375 and a partial row of cell structures 376. (For the sake of clarity, although row 375 in the implementation of Figure 39 includes a single cell structure, it is in any case considered a row of cell structures.) The inclusion of a distal tapered end portion in the retrieval device that culminates into a distal antenna provides a number of advantages over retrieval devices that would otherwise terminate in a blunt end. One advantage is that once the retrieval device has been positioned and expanded in a vessel of a patient the tapered end provides a greater degree of placement adjustment over a retrieval device having a blunt end. Another advantage is that the distal tapered end portion is more atraumatic than a blunt end. The reduced taper length achieved by limiting the construction of the distal tapered end portion 373 to less than three full rows of cell structures has been found to advantageously result in a distal taper that is both more stable and more atraumatic than those having a greater number of full rows of cell structures. In retrievers having cell structures of different sizes, like those of cell structures 376 and 377, it is preferable that the full rows of cells in the distal tapered end portion 373 be comprised of substantially all small-sized cell structures 377 like that shown in Figure 39.

According to some implementations the length of the distal tapered end portion 373 in the as-cut manufactured state is less than about 30% of the length of the main body portion 372, and preferably less than about 25% of the length of the main body portion 372. In one implementation the lengths of the main body portion 372 and the distal tapered end portion 373 are about 26 mm and 6 mm, respectively. In another implementation the distal tapered end portion 373 has a length of between about 4.5 mm to about 5.0 mm. According to some implementations the combined length of the distal tapered end portion 373 and the distal antenna 379 is less than about 10 mm.

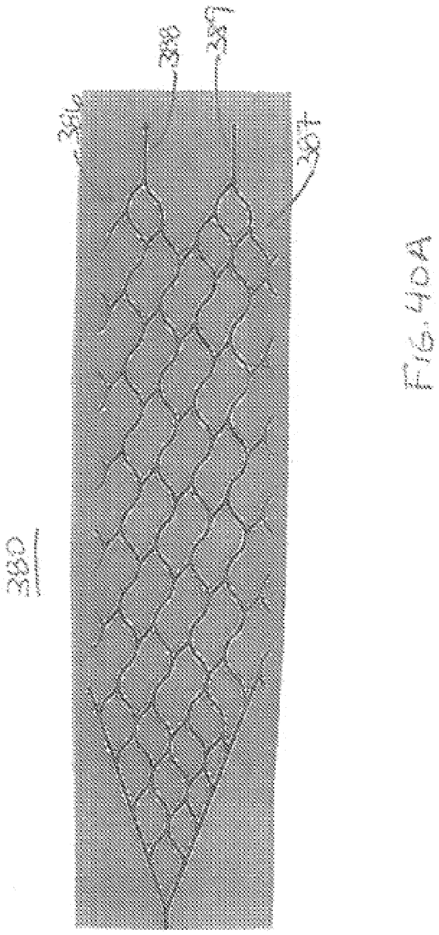

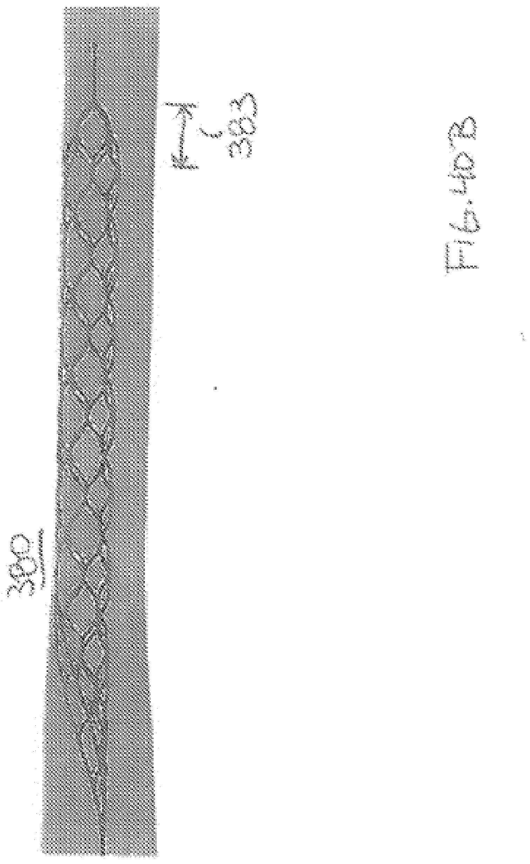

Figure 40A shows a two dimensional view of another example of a duct obstruction retrieval device 380. Like the retrieval device 370 shown in Figure 39, retriever 380 comprises a distal tapered end portion comprising less than three full rows of cell structures. Retriever device 380 differs from retriever 370 in that the distal tapered end portion comprises cell structures that are bifurcated into a first set of cell structures 386 and a second set of cell structures 387 with the first cell of cell structures 386 terminating at a first distal antenna 388 and the second set of cell structures 387 terminating at a second distal antenna 389. Figure 40B depicts a three dimensional view of retrieval device 380 with the reference number 383 denoting the distal tapered end portion of the device. As shown in Figure 40B, distal antenna 388 and distal antenna 389 are joined to form a retrieval device having a distal tapered end portion with a closed end.

It is important to note that although the retrieval devices 370 and 380 have been described as comprising distal antennas, in other implementations like retrieval devices are provided without distal antennas. The same applies to each of the implementations disclosed and contemplated herein, in addition, with reference to the retrieval device 380 of Figure 40, in another implementation only a single distal antenna is provided that is chosen between distal antenna 388 and distal antenna 389. In such an implementation the retrieval device would possess an open distal end with the second set of cell structures 387 being available to sweep along the treatment vessel to capture dislodged material.

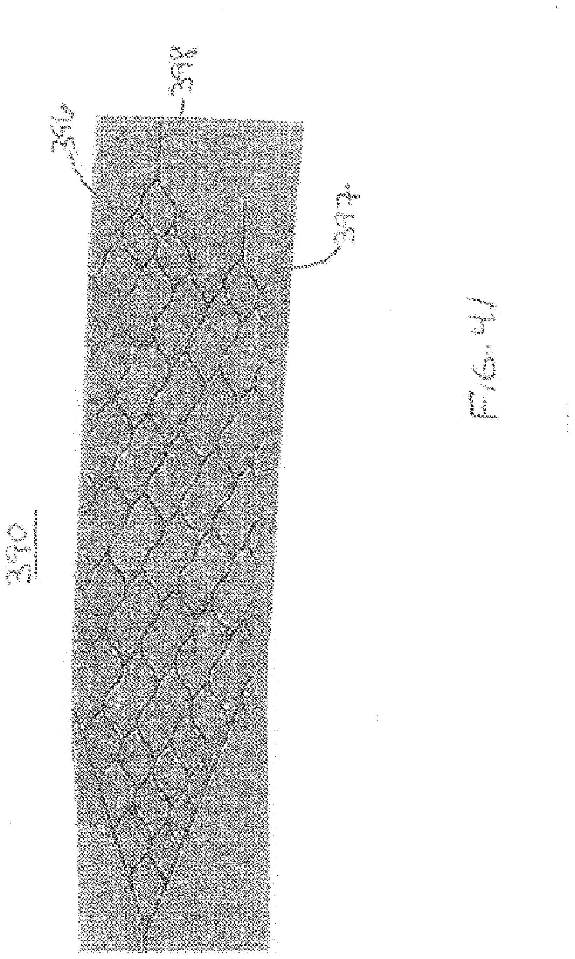

Figure 41 is a two dimensional view of another example of a duct obstruction retrieval device 390 that comprises a distal tapered end portion comprising less than three full rows of cell structures. Like retrieval device 380, the distal tapered end portion of retrieval device 390 has cell structures that are bifurcated into a first set of cell structures 396 and a second set of cell structures 397 with the first cell of cell structures 396 terminating at a first distal antenna 398 and the second set of cell structures 397 terminating at a second distal antenna 399. As shown in Figure 41, the first and second distal antennas 398 and 399 are longitudinally off-set from one another. In one implementation a radiopaque material, feature (e.g., flared strut) or component (e.g., a coil) is positioned on each of the first and second antennas. By virtue of their off-set construction, a radiopaque component, for example, on each of the antennas enables the distal end of the retrieval device and the distal tapered end portion of the retrieval device to be visually delineated during the treatment procedure.

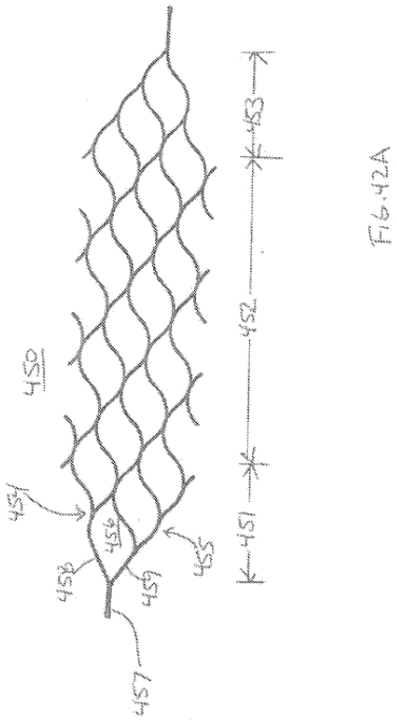

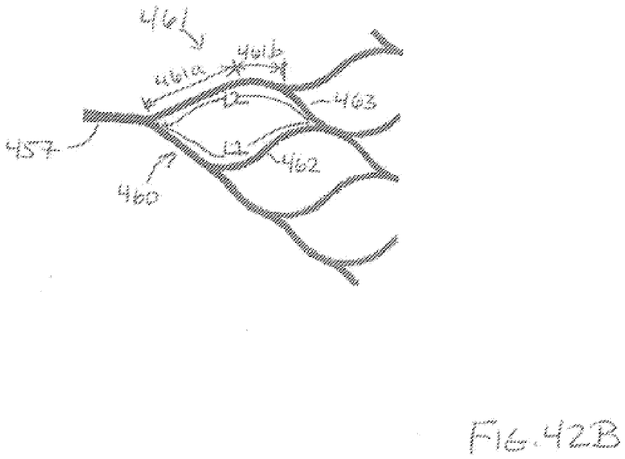

Figure 42A is a two dimensional view of an example of a duct obstruction retrieval device 450. The retrieval device 450 comprises an expandable member that has a proximal tapered end portion 451, a cylindrical, main body portion 452 and a distal tapered end portion 453. The outer-most cell structures in the proximal tapered end portion have outer wall segments that form first and second rail segments 454 and 455, respectively. Each of the rail segments 454 and 455 extend from a proximal -most end of the expandable member to a position at or near the proximal end of the cylindrical main body portion 452. In the example of Figure 42, each of the rail segments 454 and 455 are undulating. A proximal antenna 457 extends proximally from a proximal-most cell structure 456.

The proximal-most cell structure 456, as shown in greater detail in Figure 42B, comprises first and second outer struts 460 and 461, respectively, and first and second inner struts 462 and 463, respectively. As shown in the layout of Figure 42B, the first outer strut 460 and a first portion 461 a of the second outer strut 46.1 are straight in the two dimensional layout while the first inner strut 462, second inner strut 463 and the second portion 461b of strut 461 are curvilinear in the two dimensional layout. In the manufactured, three dimensional configuration the first outer strut 460 and the first portion 461a of the second outer strut 461 are curved and devoid of undulations. As a result of being oriented at the proximal end of the expandable member and being co-extensive to the proximal antenna, the straight strut segments of the proximal-most cell structure 456 enhance the pushability of the retrieval device 450 as it is delivered, through the anatomy of a patient as compared to retrieval devices having proximal-most cell structure with only curved struts in the two dimensional layout.

In some implementations, the total length of struts 460 and 462 (L1) and the total length of struts 461 and 463 (L2) are substantially the same in order to promote a nesting of the struts when the expandable member transitions from the expanded state to the unexpanded state. According to some implementations the difference in length between L1 and L2 is less than 5.0%, while in other implementations the difference in length between LI and L2 is less than 1.0%.

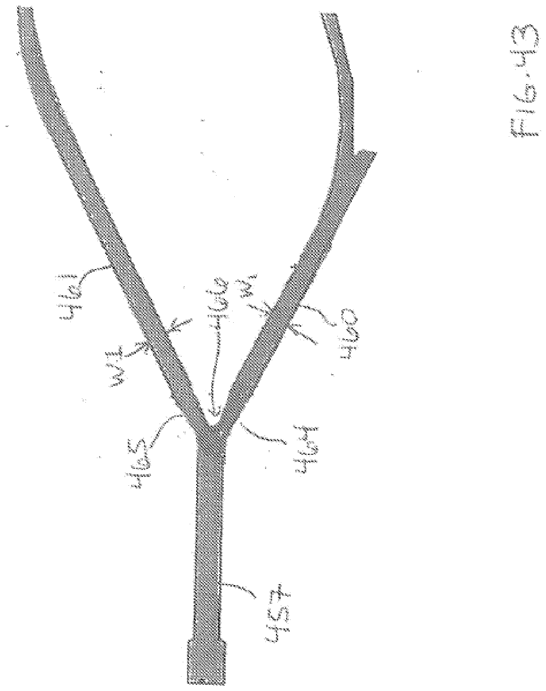

Figure 43 illustrates a variation of the proximal-most cell structure 456. As depicted, each of struts 460 and 461 have an area of reduced width 464 and 465, respectively, that are located adjacent their junction 466 with the proximal antenna 457. The inclusion of the reduced width areas 464 and 465 locally enhances the proximal-most cell structure's ability to collapse by reducing the amount of force needed to initiate and effectuate the collapse. Thus, for example, when the retrieval device 450 is first introduced into an introducer sheath for placement within a delivery catheter or is withdrawn into a delivery catheter after the expandable member has deployed inside a patient, the areas of reduced width 464 and 465 cause the shuts 460 and 461 to be more easily folded in the area of the junction 466 with less force than would otherwise be required absent the areas of reduced width. This makes the retrieval device 450 more manageable when being handled by healthcare professionals when the retrieval device 450 is being introduced into the delivery catheter for the first time, thus reducing the likelihood of the retrieval device being damaged during the introduction process. As previously discussed, after the retrieval device 450 has been introduced and expanded inside the duct of a patient there may be occasions when the retrieval device is proximally withdrawn back into the delivery catheter. This may occur, for example, upon the retrieval device being improperly placed in the duct or upon the completion of a retrieval procedure. In each of these instances because less force is required to collapse the expandable member of the retrieval device several advantages are realized. One advantage is that it reduces the likelihood of the retrieval device 450 acting upon the delivery catheter in a manner that would cause an inadvertent displacement of the delivery catheter within the duct of the patient. Another advantage is that it reduces the likelihood of excessive force being applied at the attachment between the proximal antenna 457 and the elongate wire (e.g. elongate wire 40 shown in Figure 1A) that would result in a failure at the junction.

In the implementation of Figure 43 the areas of reduced width 464 and 465 comprise tapers. In other implementations the areas of reduced width are denoted by a stepped reduction in strut width. The amount by which the width is reduced in areas 464 and 465 will vary according to the nominal widths of struts 460 and 461. In any event, it is important that the amount of width reduction is consistent with the radial force and structural integrity requirements of the expandable member. It has been discovered that a reduction of width in the as-cut manufactured state of between about 5.0% and about 20.0% is suitable for struts having a nominal width of between about 0.145 mm (0.0057 inches) and about 0.06858 mm (0.0027 inches), with a preferable range being between about 10.0% and about 20.0% in width reduction. In one implementation the width dimension W1 of struts 460 and 461 is about 0.135 mm (0.0053 inches) with the minimum width dimension of the areas of reduced width being 0.1194 mm (0.0047 inches). In another implementation the width dimension W1 of struts 460 and 461 is about 0.145 mm (0.0057 inches) with the minimum width dimension of the areas of reduced width being 0.112 mm (0.0046 inches).

In some implementations the as-cut width dimensions of struts 460 and 461 are different, with the width dimension of their respective areas of reduced width 464 and 465 also being different. For example, in one implementation strut 460 has a width dimension of about 0.127 mm (0.0050 inches), strut 461 has a width dimension of about 0.145 mm (0.0057 inches), and areas of reduce width 464 and 465 have width dimensions of about 0.10668 mm (0.0042 inches) and about 0.112 mm (0.0046 inches), respectively.

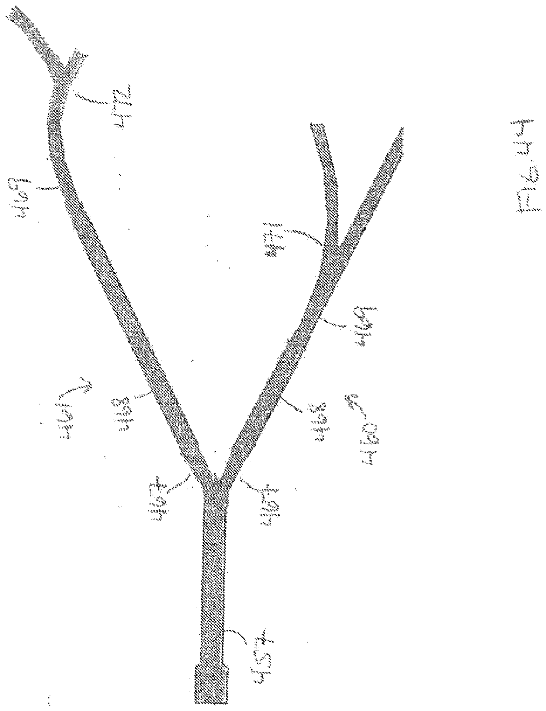

Figure 44 shows another variation of the proximal-most cell structure 457 wherein outer struts 460 and 461 comprise a proximal section 467, a midsection 468 and a distal section 469. Because the width dimensions of the outer struts 460 and 461 of the proximal-most cell structure 456 are generally made greater than most of struts in the remaining portion of the retrieval device 450 for the purpose of enhancing the pushability of the expandable member, the bulk of material at the junctures 471 and 472 located at the distal end of the struts may impede the expandable member's ability to collapse. For this reason, in the implementation of Figure 44 the distal sections 469 have a reduced width dimension in order to reduce the amount of material occupying the juncture regions 471 and 472. Although Figure 44 also shows the proximal sections 467 having a reduced width dimension (similar to that described above), in some implementations this is not the case. In a manner described above, the sections of reduced width may comprise tapers and/or steps.

Another advantage of the example depicted in Figure 44 is that the midsection 468 of struts 460 and 461 may be provided with a sufficient width to enhance the visibility of the device under fluoroscopy without materially impacting the ability of the proximal end of the proximal tapered region 451 to collapse or to otherwise assume its unexpanded state. According to one implementation the width dimension of the strut midsections 468 is about 0,1346 mm (0,0053 inches) and the minimum width dimension of the proximal and distal sections 467 and 469 being 0.1194 mm (0.0047 inches) and 0.1041 mm (0.0041 inches), respectively. As with some of the Figure 43 implementations, in some Figure 44 implementations the width dimensions of struts 460 and 461 are different, with the width dimension of one or more of their respective proximal sections, midsections and distal sections being different.

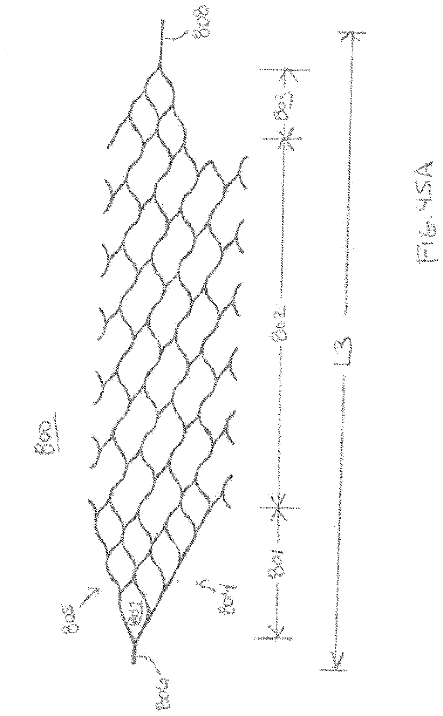

Figure 45A is a two dimensional view of a duet obstruction retrieval device 800 according to another implementation. The retrieval device 800 comprises an expandable member that has a proximal tapered end portion 801, a cylindrical main body portion 802 and a distal tapered end portion 803. The outer-most cell structures in the proximal tapered end portion have outer wall segments that form on one side a non-undulating rail segment 804 and on the other side an undulating rail segment 805. Each of the rail segments 804 and 805 extend from a proximal-most end of the expandable member to a position at or near the proximal end of the cylindrical main body portion 802, A proximal antenna 806 extends proximally from a proximal-most cell structure 807 while a distal antenna 808 extends distally from the distal end of distal tapered section 803. The distal tapered section 803 is similar to that described above in conjunction with Figure 39. In some implementations the proximal -most cell structure 807 has the same features and characteristics as proximal-most cell structure 456 in the implementations of Figures 42-44 above.

As a result of the diagonal disposition of the cell structures in the retrieval device, the straight line length along which rails 804 and 805 pass are different in the as-cut manufactured state with the straight line length that passes along rail 804 being longer than the straight line length that passes along rail 805. The linear configuration of rail 804 in combination with the undulating configuration of rail 805 advantageously results in the rails 804 and 805 having lengths that more closely approach one another when the retrieval device assumes it's unexpanded/de livery state. According to one implementation, rails 804 and 805 are configured to achieve substantially the same length when the retrieval device 800 is in the unexpanded/delivery state. In some implementations, the difference in length between rails 804 and 805 is between about 0% to about 5% when the retrieval device 800 is in the unexpanded/delivery state.

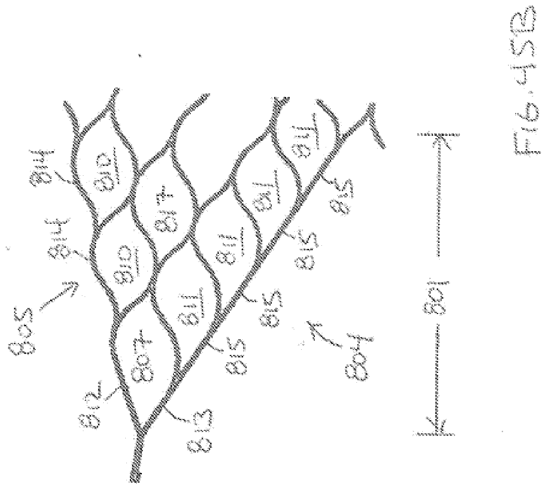

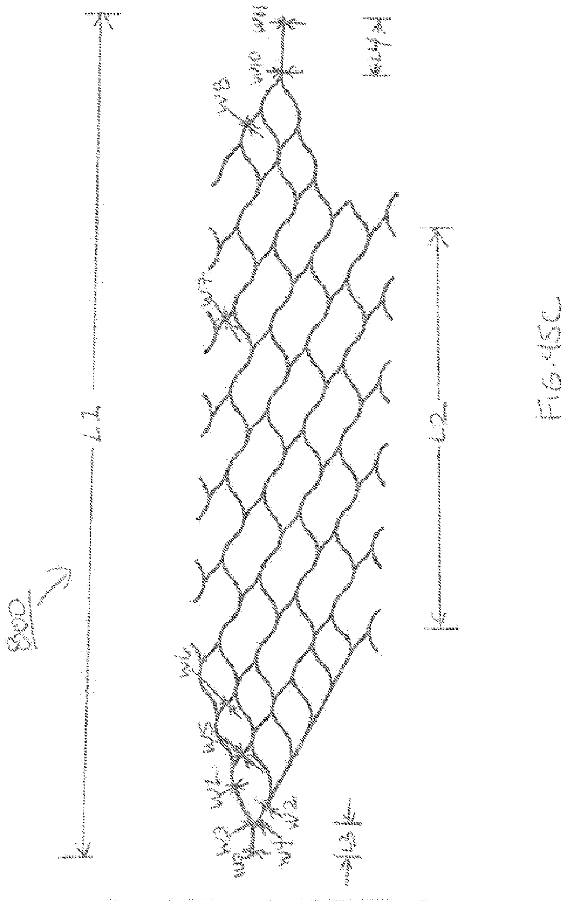

As discussed earlier, the retrieval devices disclosed and contemplated herein are generally laser cut from a tube and in their actual three dimension configuration generally comprise tube like structures. Figure 45, like many of the other figures, represents a retrieval device as it would appear in a two dimension layout, that is, as if it were cut along its length and laid out on a flat surface. With this in mind, and with reference to Figure 45B, in the two dimension layout the cell structures are polygons comprising a plurality of struts. As shown, rail segments 804 and 805 are constructed by the outer walls of the outer most cell structures 807, 810 and 811 in the proximal tapered section 810. Undulating rail segment 805 is formed by a first outer wall 812 of the proximal-most cell structure and the outer walls 814 of outer cell structures 810, whereas the non-undulating rail segment 804 is formed by a second outer wall 813 of the proximal- most cell structure and the outer walls 815 of outer cell structures 811. As represented in Figure 45B, in the two dimension layout the outer walls 814 are curvilinear and the outer walls 815 are straight. As will be appreciated, when in the tubular form, the rail 804 will be curved when the expandable member assumes an expanded state, but will nonetheless be devoid of undulations. Rail 805 will also assume an additional degree of curvature in its three dimensional state, but unlike rail 804 will comprise undulations.

An advantage of the proximal tapered section 801 design is that the non-undulating rail segment 804 provides the aforementioned benefits related to pushability and kink resistance, while the undulating rail segment 805 accommodates the inclusion of a larger number of symmetric-shaped polygons and/or nearly symmetric-shaped polygons within the section 801. The inclusion of an increased number of symmetric-shaped and/or nearly symmetric-shaped polygons in the distal tapered end portion 801 improves its ability to assume it's unexpanded or compressed state and also provides for a more uniform and compact configuration. Because symmetrically shaped cell structures have better nesting tendencies than their non-symmetric counter-parts, the aforementioned advantages are achieved, at least in part, by the increased number of symmetrically shaped cell structures disposed within the proximal tapered end portion 801.

Another advantage of a proximal tapered end portion having one non-undulating rail segment 804 and one undulating rail segment 805 is that the inclusion of the undulating rail segment provides more freedom in the design of the cell structures within the proximal tapered end portion as opposed to a design having two non-undulating rails. As shown in Figure 45B, the outer cell structures 811, along which the non-undulating rail segment 804 is formed, comprise structures that are considerably more symmetric than those, like for example, shown in Figure 30.

With reference to Figure 45C, according to one implementation the retriever device 800 has the following as-cut dimensional characteristics; L1= 56.44 mm ± 0.50 mm; L2= 26.85 mm ± 0.50 mm; L3= 2.0 mm ± 0.1 mm; L4= 4.0 mm ± 0.3 mm; W1= 0.137 mm (0.0054 inches) ± 0.01016 mm (± 0.0004 inches); W2= 0.142 mm (0.0056 inches) ± 0.01016 mm (± 0.0004 inches); W3= 0.1194 mm (0.0047 inches) ± 0.01016 mm (± 0.0004 inches); W4= 0.1194 mm (0.0047 inches) ± 0.01016 mm (± 0.0004 inches); W5= 0.1194 mm (0.0040 inches) ± 0.01016 mm (± 0.0004 inches); W6= 0.06858 mm (0.0027 inches) ± 0.01016 mm (± 0.0004 inches); W7= 0.08636 mm (0.0034 inches) ± 0.01016 mm (± 0.0004 inches); W8= 0.0787 mm (0.0031 inches) ± 0.01016 mm (± 0.0004 inches); W9= 0.254 mm (0.010 inches) ± 0.178 mm (± 0.007 inches); W10= 0.0889 mm (0.0035 inches) ± 0.01016 mm (± 0.0004 inches); W11= 0.0635 mm (0.0025 inches) ± 0.01016 mm (± 0.0004 inches). In one implementation the length of the proximal tapered end portion and the distal tapered end portion is about 13 mm and 7mm, respectively.

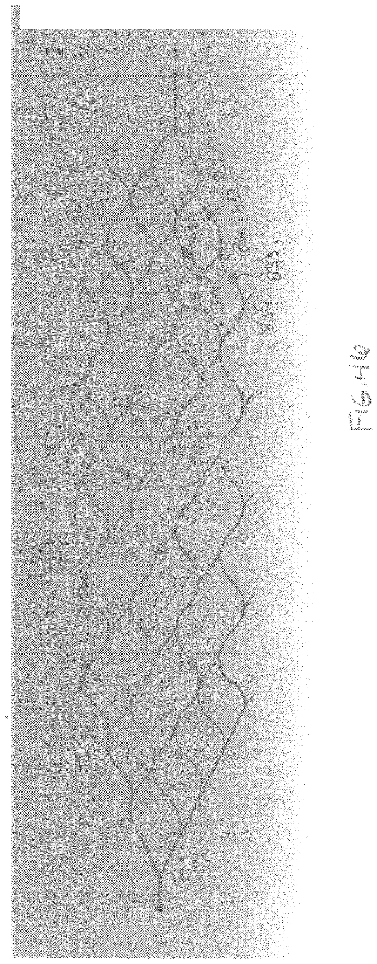

Figure 46 illustrates another example of an obstruction retrieval device 830 wherein portions 833 of some struts 832 in the distal tapered end portion 831 of the retriever device are flared to enhance the radiopacity of the distal region of the device. In some implementations during manufacture each of portions 833 are laser cut so as to possess an enhanced width dimension with respect, to the remainder of strut 832. Besides in themselves enhancing radiopacity, the flared portions (or portions of enhanced width) provide a good platform for receiving a radiopaque coating such as, for example, a gold coating. In the implementation of Figure 46 the flared portions 88 are positioned a sufficient distance from the strut junctions 834 so as to not interfere with the retriever's ability to compress. In the example of Figure 46 the flared portions 833 are also longitudinally staggered so that when the retriever 830 is in the compressed state no more than a single flared portion 833 will occupy a longitudinal position. Such a configuration lessons the impact the flared portions 833 may have on the retriever's lowest achievable diameter dimension along the distal tapered end portion 833. In the example of Figure 46, the flared portions comprise nodes which in one implementation have a diameter of about 0.015 inches. In other embodiments the flared portions 833 are longitudinal in nature and occupy a substantial length of the struts 832. In such implementations the flared portions 833 may have a width of between about 0.0889 mm (0.0035 inches) to about 0.1143 mm (0.0045 inches).

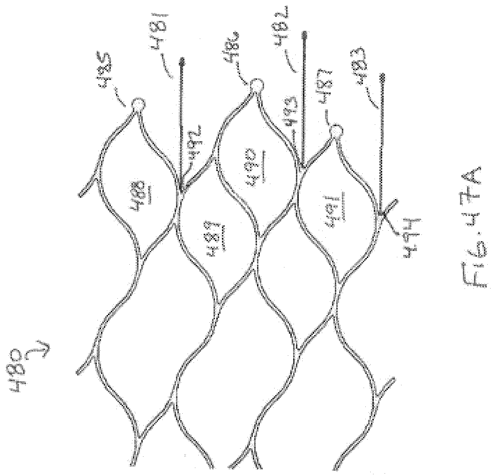

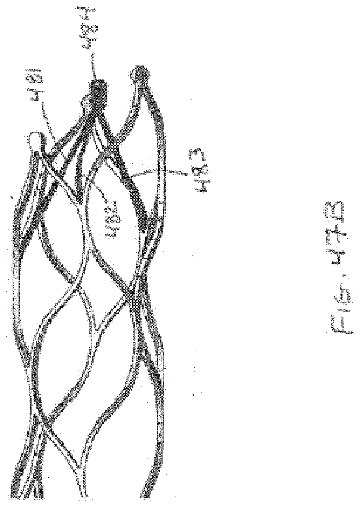

Figures 47A and 47B illustrate an example of a distal segment of an obstruction retrieval device 480. Figure 47A depicts the device 480 in a two-dimensional layout as if it were cut along its length and laid out on a flat surface. While Figure 47A depicts the device 480 in its as-cut configuration, the three-dimensional representation of Figure 47B shows the device 480 in a post-cut manufactured state.

With reference to Figure 47A, the distal segment of device 480 comprises a plurality of distal cell structures 488-491 with a set of antennas 481, 482 and 483 extending distally from the junction regions 492-494 of cell structures 488-491. In some implementations tabs 485-487, or other enhanced dimension features, are provided at one or more ends of the distal most cell structures for the purpose of identifying the distal end of the device under fluoroscopy by virtue of their enhanced dimensional characteristics and/or as a result of being endowed with a radiopaque material. As shown in Figure 47A, in some implementations the distal-most cell structures are smaller than the adjacent cell structures in the main body portion of the device 480.

As shown in Figure 47B, at, a point in time after the device 480 has been formed, such as being cut by a laser, the distal ends of antennas 481, 482 and 483 are joined together at the juncture 484 so as to provide the internal cavity of device 480 with a closed-end. In effect, the closed-end forms a basket that facilitates the collection of particulates, such as embolic material, that may become dislodged during a retrieval procedure. In some implementations the juncture 484 is formed by soldering together the distal ends of the antennas 481-483. In some implementations the distal ends of the antennas 481-483 are positioned within an encasement, such as a coil spring or other perforated structure, with a solder or other bonding agent being applied within and/or about the encasement to effectuate a bonding together of the distal ends of the antennas 481-483. In some implementations the encasement comprises a rounded atraumatic distal tip. In some implementations the encasement comprises a radiopaque material.

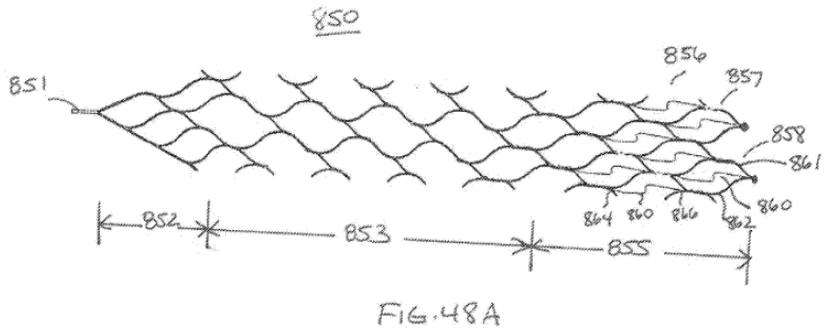

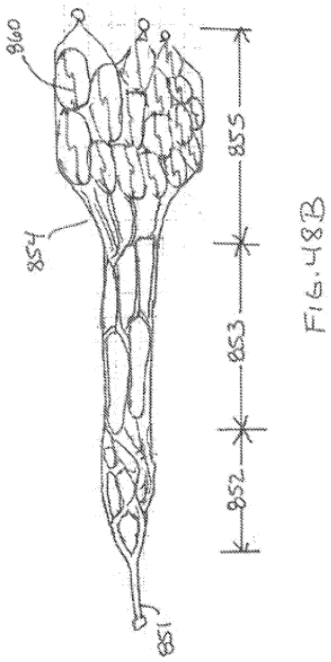

Figures 48A and 48B illustrate an obstruction retrieval device 850 according to one implementation. Figure 48A depicts the device 850 in a two-dimensional layout as if it were cut along its length and laid out on a flat surface. While Figure 48A depicts the device 850 in its as-cut configuration, the three-dimensional representation of Figure 48B shows the device 480 in a post-cut manufactured state. The device includes a proximal antenna 851, a proximal taper portion 852, a main body portion 853 and a distal portion 855, In the as-cut manufactured state the main body portion 853 and the distal portion 855 have the same, or substantially same, diameter. At a point in time after the device 850 has been cut, such as by laser cutting, the device 850 is formed so that the unconstrained configuration of the distal portion 855 has a diameter that is greater than that of the unconstrained main body portion 853. The post as-cut form of the device 850 may be achieved with the use of mandrels or other tools and methods known in the art. In some implementation the ratio of the unconstrained diameter of the distal portion 855 (absent the transition portion 854) and the main body portion 853 is between about 1.2/1.0 and about 2.0/1.0. For example, according to one implementation the average unconstrained diameter of the main body portion 853 is about 2.0 millimeters and the average unconstrained diameter of the distal portion 855, absent the transition portion 864, is about 4.0 millimeters. According to some implementations the ratio of the unconstrained length of the distal portion 855 (absent the transition portion 854) and the unconstrained length of the main body portion is between about 0.2 to about 0.7. For example, according to one implementation the unconstrained length of the main body portion 855 is between about 15 to 25 millimeters and the unconstrained length of the distal portion (absent the transition portion 854) is between about 5 to 10 millimeters.

According to some implementations, as depicted in Figure 48.A, the cell structures in the main body portion 853 are larger in size than those in the distal portion 855. The lower strut density in the main body portion 853 facilitates an integration of the retrieval device 850 within an obstruction. The higher strut density in the distal portion 855 facilitates the entrapment of dislodge particles as discussed in more detail below. Additionally, in some implementations the retrieval device is constructed in a manner that results in a radial force being exerted by the main body portion 853 that is greater than the radial force exerted by the distal portion 855 when the retrieval device 850 is deployed within a duct of a patient. In such an implementation, the main body portion 853 is situated to capture an obstruction while the distal portion 855 more gently acts against a wall of the duct distal to the obstruction to entrap portions of the obstruction that become dislodged during and after its capture. As such, according to one method the retrieval device 850 is placed at the treatment site of a patient by use of a delivery catheter, as previously disclosed herein. The retrieval device 850 is positioned at a distal end of the delivery catheter so that the main body portion 853 is positioned at the site of the obstruction to be retrieved. When sheathed within the delivery catheter the main body portion 853 and the distal portion 855 have the same, or substantially the same, diameter. Thereafter, the delivery catheter is withdrawn proximally to cause the constrained retrieval device to expand at the treatment site so that the main body portion 853 is at least partially forced into the obstruction and so that at least a portion of the distal portion 855 more gently rests against the duct wall distal to the obstruction. Upon the obstruction being captured within the main body portion 853 of device 850, the device may be removed from the patient in a manner consistent with one or more of the methods previously disclosed herein. During such removal, as the retrieval device is pulled proximally the distal portion 855 sweeps along the duct wall to entrap portions of the obstruction that may have become dislodged. By virtue of its enhanced diametric dimension, the distal portion 855 maintains contact with the duct wall during all or a portion of the removal procedure.

As discussed above, a lower strut density in the main body portion 853 facilitates an integration of the retrieval device 850 within an obstruction. However, in some implementations the retrieval device is constructed in a manner that, results in a radial force being exerted by the main body portion 853 that is greater than the radial force exerted by the distal portion 855 when the retrieval device 850 is deployed within a duct of a patient. To achieve this variation in a radial force, in some implementations the width dimension of the struts in the main body portion 853 of the retrieval device are cut to have a larger width dimension of at, least some or all of the struts in the distal portion 855.

As shown in Figures 48A and 48B, in some implementations the strut density in the distal segment 855 is further enhanced by the inclusion of non-linear struts 860 in at least some of the cell structures. In some implementations the non-linear struts extend between the proximal end 864 and distal end 866 of cell structures. In some implementations the non-linear struts 860 extend between the proximal end 864 and distal end 866 of cell structures with the non-linear strut 861 having substantially the same length as the upper strut 861 and/or lower strut 862 in the as-cut configuration. Such a construction enhances the ability of the cell structure struts to nest resulting in a lower achievable constrained diameter of the retrieval device. In some implementations the nonlinear struts 861 extend between the proximal end 864 and distal end 866 of cell structures with the upper, lower and non-linear struts 860, 862 and 861, respectively, having substantially the same length in the as-cut configuration. So as not to greatly impact the radial force produced in the distal segment 855, in some implementations the non-linear struts 860 have a width dimension less than the width dimension of the upper and lower struts 861 and 862. In some implementations the ratio of the width dimension of struts 860 and the width dimension of each of the upper and lower struts 861 and 862, respectively, is between about 0.70 and 0.80. For example, according to one implementation each of the upper and lower struts, 361 and 362, have a width dimension of about 0.0889 mm (0.0035 inches) while strut 360 has a width dimension of about 0.0635 mm (0.0025 inches).

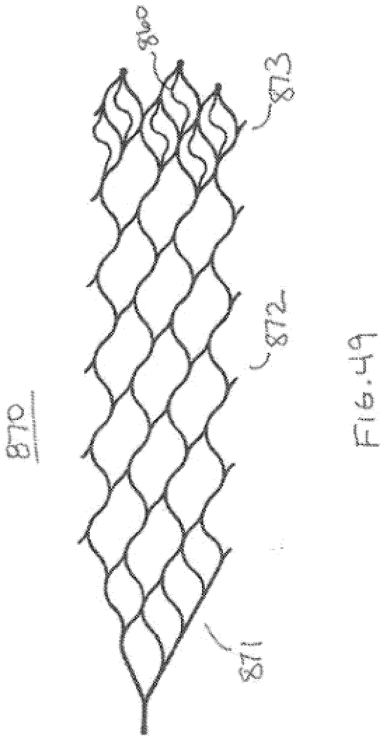

Figure 49 illustrates a variation to the as-cut configuration shown in Figure 48A. As shown in Figure 49, the retrieval device 870 comprises a proximal distal portion 871, a main body portion 872 and a distal portion 873, the distal portion being shorter in length than that depicted in Figure 48A.

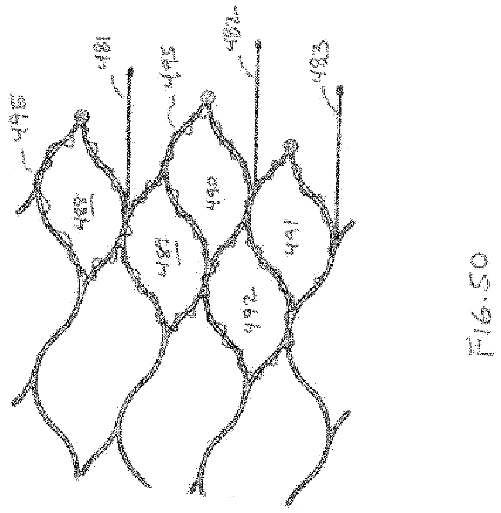

Figure 50 illustrates a distal segment of a retrieval device similar to that shown in FIGS. 47A and 47B having one or more radiopaque wires or ribbons 495 wound about the struts that form cell structures 488-492. Throughout the remainder of the disclosure the term "wire" is used broadly to include wires, ribbons, or like structures. Although the entirety of the cell struts that form cell structures 488-492 may be wound with one or more radiopaque wires 495 as shown in FIG. 50, in accordance with the invention, only a selected number of struts may possess radiopaque wire windings. An advantage of incorporating the radiopaque wire windings into the distal segment of the retrieval device is that it enhances the visibility of the distal end of the device under fluoroscopy. In addition, when a sufficient number of distal member struts are endowed with wire windings, such as shown in FIG, 50, the wire windings enhance the stiffness of the distal segment. An advantage of increasing the stiffness of the distal segment is that it inhibits prolapse of the distal segment as the retrieval device is advanced through a delivery catheter or treatment duct of a patient. In one implementation the one or more wires comprise platinum. However, it is to be appreciated that any of a number of other radiopaque materials may be used. The one or more wires may comprise a core structure, such as stainless steel, that is clad or otherwise coated with a radiopaque material. The one or more wires may also comprise a polymeric structure impregnated, doped or otherwise coated with a radiopaque material. In some implementations the cross-sectional area of the one or more wires varies to provide a variation in radiopacity and/or stiffness within the distal segment. In some implementations the diameter or width dimension of the one or more wires 495 is in the range of between about 20% to about 50% less than the width dimension of the struts which form the distal segment.

Although not shown in FIG. 50, in some implementations small recesses are provided in at least some of the struts of cell structures 488-492 for the purpose of guiding the placement of the wire windings to designated locations. Preferably, the recesses are sized to receive only a portion of the wire so that only a portion of the wire resides within the recess and a portion of the wire resides outside the recess.

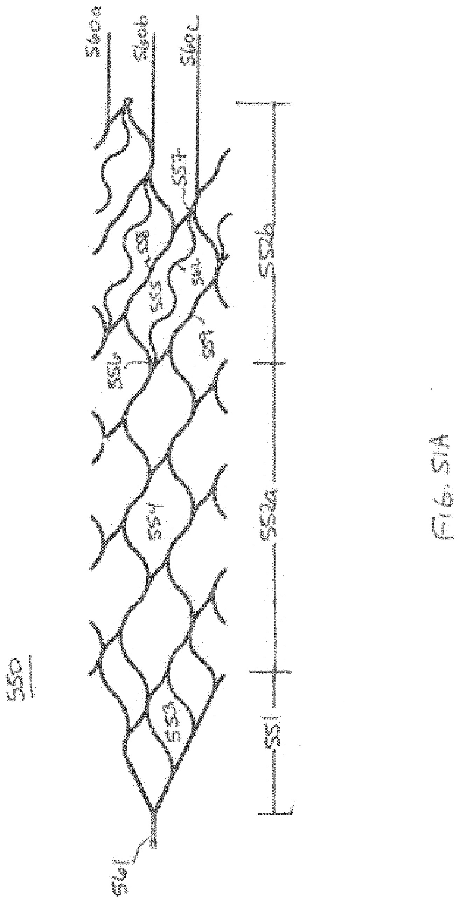

FIGS. 51A through 51D illustrate other aspects of a clot retrieval device 550 which are in some ways similar to the retrieval device 850 depicted in FIGS. 48A and 48B. FIGS. 51A-51D depict the device 550 in a two-dimensional layout as if it were cut along its length and laid out on a flat surface. The device includes a proximal antenna 561, a proximal taper portion 551, a main body portion 552a and a distal portion 552b. In the as-cut manufactured state the main body portion 552a and the distal portion 552b have the same, or substantially same, diameter. At a point in time after the device 550 has been cut, such as by laser cutting, the device 550 is formed so that the unconstrained configuration of the distal portion 552b has a diameter that is greater than that of the unconstrained main body portion 552a. The post as-cut form of the device 550 may be achieved with the use of mandrels or other tools and methods known in the art. In some implementations, the ratio of the unconstrained diameter of the distal portion 552b and the main body portion 552a is between about 1.2/1.0 and about 2.0/1.0. For example, according to one implementation the average unconstrained diameter of the main body portion 552a is about 2.0 millimeters and the average unconstrained diameter of the distal portion 552b is about, 4.0 millimeters. According to some implementations the ratio of the unconstrained length of the distal portion 552b and the unconstrained length of the main body portion 552a is between about 0.2 to about 0.7. For example, according to one implementation the unconstrained length of the main body portion 552a is between about, 15 to 25 millimeters and the unconstrained length of the distal portion is between about 5 to 10 millimeters.

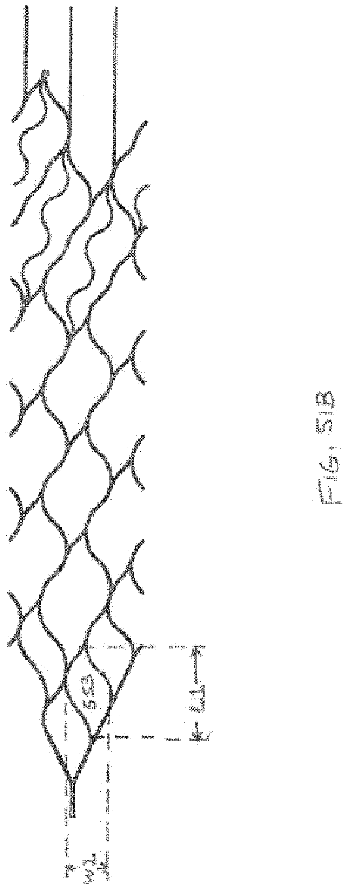

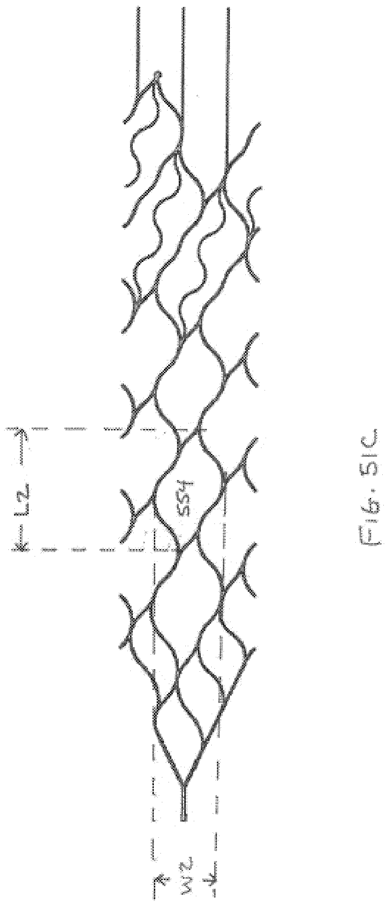

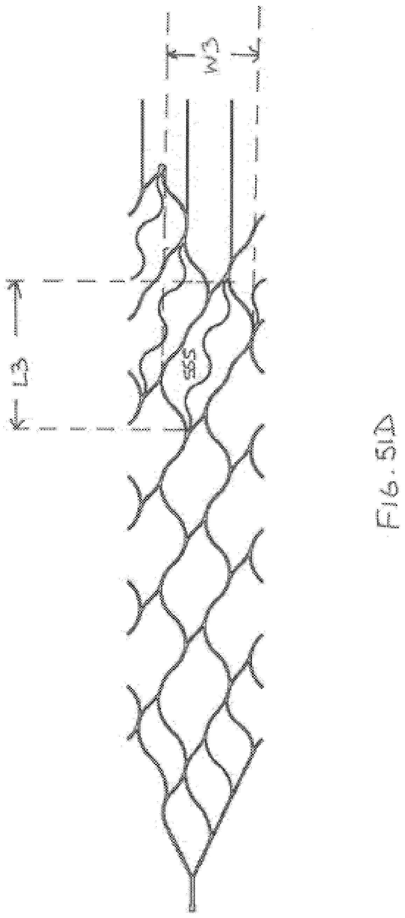

According to some implementations, cell structures in the proximal taper portion 551, main body portion 552 and distal portion 552b are of different sizes. In the example of FIG. 51A, the cell sizes are multiples of one another with cell structure 554 comprising an area approximately equal to two of cell structure 553 and cell structure 555 comprising an area approximately equal to three of cell structures 553. It is important to note that cell sizes that are multiples of one another are not required. FIG. 51B illustrates the length dimension LI and width dimension W1 of cell structure 553. FIG. 51C illustrates the length dimension L2 and width dimension W2 of cell structure 554. FIG. 51D illustrates the length dimension L3 and width dimension W3 of cell structure 555.

According to some implementations cell structures 553, 554 and 555 each have an average length to width ratio greater than one when the retrieval device is in an unexpanded state and when the retrieval device is in an expanded state. The ability of the cell structures 553, 554, and 555 to maintain an average length to width ratio of greater than one inhibits the cells from collapsing lengthwise as the device 550 travels through a delivery catheter or duct of a patient. In other words, the cell structures of device 550 are inhibited from collapsing lengthwise on themselves in an accordion like fashion due to their length to width ratios being greater than one.

Another aspect is reflected in the length L3 of the cell structures 555 in the distal portion 552b of the device. Because the distal portion 552b assumes an expanded diameter that is greater than the expanded diameter of the remaining portions of the device, the length dimension 1.3 is selected to be sufficiently long in comparison to its width dimension W3 so as to ensure that the cell structures 555 maintain their length dimension to be greater than their width dimensions when the retrieval device 550 transitions from an unexpanded to an expanded state.

In some implementations the average length to width ratio of the cell structures in the distal portion 552b of the cylindrical main body portion are greater than the average length to width ratio of the cell structures in the proximal portion 552a of the cylindrical main body portion, the average length to width ratio of the cell structures in the proximal portion 552a of the cylindrical main body portion being greater than the average length to width ratio of the cell structures in the proximal end portion 551, and with the average length to width ratio of the cell structures in the proximal end portion being greater than one when the self-expandable member is in the unexpanded and expanded configuration.

In some implementations the retrieval device 550 is constructed in a manner that results in a radial force being exerted by the main body portion 552a that is greater than the radial force exerted by the distal portion 552b when the retrieval device 550 is deployed within a duct of a patient. In such an implementation, the main body portion 552a is situated to capture an obstruction while the distal portion 552b more gently acts against a wall of the duct distal to the obstruction to entrap portions of the obstruction that become dislodged during and after its capture. As such, according to one method the retrieval device 550 is placed at the treatment site of a patient by use of a delivery catheter, as previously disclosed herein. The retrieval device 550 is positioned at a distal end of the delivery catheter so that the main body portion 552a is positioned at the site of the obstruction to be retrieved. When sheathed within the delivery catheter the main body portion 552a and the distal portion 552b have the same, or substantially the same, diameter. Thereafter, the delivery catheter is withdrawn proximally to cause the constrained retrieval device to expand at the treatment site so that the main body portion 552a is at least partially forced into the obstruction and so that at least a portion of the distal portion 552b more gently rests against the duct wall distal to the obstruction. Upon the obstruction being captured within the main body portion 552a of device 550, the device may be removed from the patient in a manner consistent with one or more of the methods previously disclosed herein. During such removal, as the retrieval device is pulled proximally the distal portion 552b may sweep along the duet wall to entrap portions of the obstruction that may have become dislodged. By virtue of its enhanced diametric dimension, the distal portion 552b maintains contact with the duet wall during all or a portion of the removal procedure.

As discussed above, a lower strut density in the main body portion 552a (as compared to the strut density in the proximal taper portion 551) facilitates an integration of the retrieval device 550 within an obstruction. However, in some implementations the retrieval device is constructed in a manner that, results in a radial force being exerted by the main body portion 552a that is greater than the radial force exerted by the distal portion 552b when the retrieval device 550 is deployed within a duct of a patient. To achieve this variation in a radial force, in some implementations the width dimension of the struts in the main body portion 552a of the retrieval device are cut to have a larger width dimension of at least some or all of the struts in the distal portion 552b.

As shown in FIG. 51A, in some implementations the strut density in the distal segment 552b is further enhanced by the inclusion of non-linear struts 562 in at least some of the cell structures. In some implementations the non-linear struts 562 extend between the proximal end 556 and distal end 557 of cell structures. In some implementations the non-linear struts 562 (or intermediate struts) extend between the proximal end 556 and distal end 557 of cell structures with the non-linear strut 562 having substantially the same length as the upper strut 558 and/or lower strut 559 in the as-cut configuration. Such a construction enhances the ability of the cell structure struts to nest resulting in a lower achievable constrained diameter of the retrieval device. So as not to greatly impact the radial force produced in the distal segment 552b, in some implementations the non-linear struts 562 have a width dimension less than the width dimension of the upper and lower struts 558 and 559. In some implementations the ratio of the width dimension of struts 562 and the width dimension of each of the upper and lower struts 558 and 559, respectively, is between about 0.70 and 0.90. For example, according to one implementation each of the upper and lower struts, 558 and 559, have an as-cut width dimension of about 0.0889 mm (0.0035 inches) while strut 562 has an as-cut width dimension of about 0.0635 mm (0.0025 inches).

Although not shown in FIGS. 51A-51D, in some implementations the cell structures in the distal segment 552b have wire windings selectively woven through their struts in order to endow the distal segment with a desired radiopacity and/or stiffness as discussed above in relation to FIG. 50.

In some implementation, a plurality of antennas 560a, 560b and 560c extend distally to the distal-most, circumferential row of cell structures in the distal segment 552b. In a manner like that disclosed above in conjunction with the device of FIGS, 47A and 47B, the distal ends of antennas 560a, 560b and 560c are joined together so as to provide the internal cavity of device 550 with a partially closed-end. In effect, the closed-end forms a basket that facilitates the collection of particulates, such as embolic material, that ma}' become dislodged during a retrieval procedure. In some implementations the juncture of antennas 560a, 560b and 560c is formed by soldering together the distal ends of the antennas. In some implementations the distal ends of the antennas are positioned within an encasement, such as a coil spring or other perforated structure, with a solder or other bonding agent being applied within and/or about the encasement to effectuate a bonding together of the distal ends of the antennas. In some implementations the encasement comprises a rounded atraumatic distal tip. In some implementations the encasement, comprises a radiopaque material.

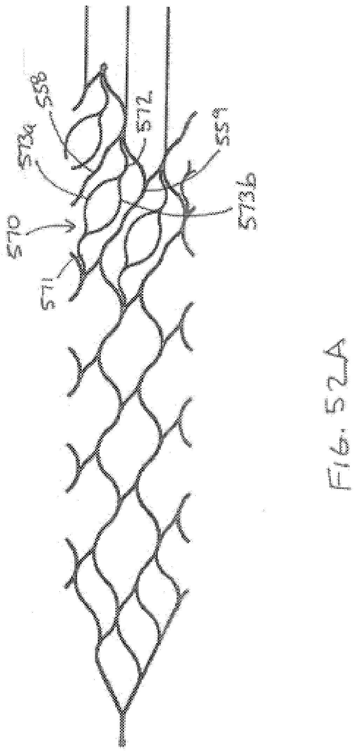

FIG. 52A illustrates a retrieval device similar to that disclosed in FIGS. 51A-51D. A difference lies in the construction of the non-linear/intermediate struts 570 that are disposed in the distal segment cell structures 555. As shown in FIG. 52A, the intermediate strut 570 comprises first and second curvilinear elements 571 and 572, respectively, between which are bifurcation struts 573a and 573b. An advantage of the configuration of strut 570 is that it provides additional coverage, as compared to strut 562, to assist in entrapping embolic debris. In some implementations the non-linear struts extend between the proximal end 556 and distal end 557 of cell structures 555. In some implementations the non-linear strut 570 extends between the proximal end 556 and distal end 557 of cell structures with the combined length of elements 571, 572 and 573a being approximately the same length as the upper strut 558 and/or the combined length of elements 571, 572 and 573b being substantially the same length as the lower strut 559 in the as-cut configuration. So as not to greatly impact the radial force produced in the distal segment 552b, in some implementations the non-linear struts 570 have a width dimension less than the width dimension of the upper and lower struts 558 and 559. In some implementations the ratio of the width dimension of struts 570 and the width dimension of each of the upper and lower struts 558 and 559, respectively, is between about 0.70 and 0.90. For example, according to one implementation each of the upper and lower struts, 558 and 559, have an as-cut width dimension of about 0.0889 mm (0.0035 inches) while strut 360 has an as-cut width dimension of about 0.0635 mm (0.0025 inches).

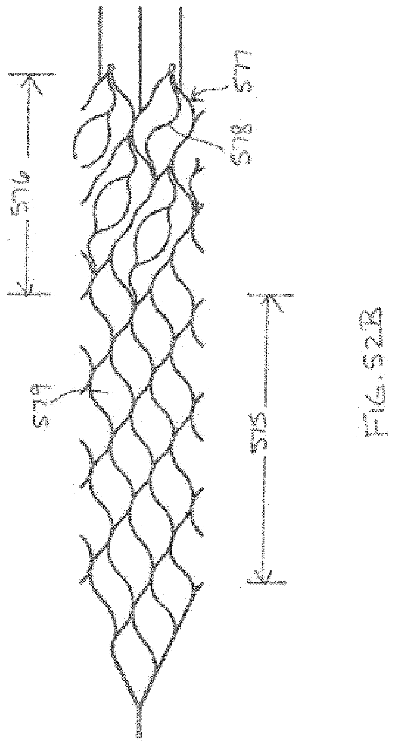

FIG. 52B illustrates a variation to the retrieval device shown in FIG, 52A with differences existing in the size of the cell structures 579 in the proximal section 575 of the cylindrical body portion and the inclusion of cell structure 577 in the distal section 576 of the cylindrical body portion. As shown in FIG. 52B, cell structures 579 are smaller in size to the similarly situated cell structures in the retrieval device of FIG. 52A as a result of additional struts being added (those depicted by dashed lines) to essentially reduce the size of the cell structures by half. As noted above, another difference lies in the inclusion of cell structure 577 at the distal end of the retrieval device to provide a device with a substantially uniform circumferential end. In one implementation an intermediate strut 578 extends between the opposite ends of cell structure 577 in a manner similar to that described above with respect to intermediate struts 562.

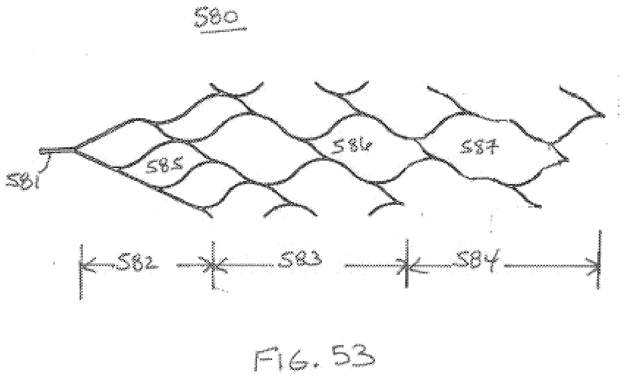

FIG. 53 illustrates a retrieval device 580 that comprises different size cell structures along its length, similar to that disclosed above in conjunction with the device 550 illustrated in FIGS. 51A- 51D. FIG, 53 depicts the device 580 in a two-dimensional layout as if it were cut along its length and laid out on a flat surface. The device includes a proximal antenna 581, a proximal taper portion 582, a main body portion 583 and a distal portion 584. In the as-cut manufactured state the main body portion 583 and the distal portion 584 have the same, or substantially same, diameter. At a point in time after the device 580 has been cut, such as by laser cutting, the device 580 is formed so that the unconstrained configuration of the distal portion 584 has a diameter that is greater than that of the unconstrained main body portion 583. The post as-cut form of the device 580 may be achieved with the use of mandrels or other tools and methods known in the art. In some implementation the ratio of the unconstrained diameter of the distal portion 584 and the main body portion 583 is between about 1.2/1.0 and about 2.0/1.0. For example, according to one implementation the average unconstrained diameter of the main body portion 583 is about 2.0 millimeters and the average unconstrained diameter of the distal portion 584 is about 4.0 millimeters.

As illustrated in FIG. 53, the cell structures in the proximal taper portion 582, main body portion 583 and distal portion 584 are of different sizes. In the example of FIG. 53, the cell sizes are multiples of one another with cell structures 586 comprising an area approximately equal to two of cell structure 585 and cell structure 587 comprising an area approximately equal to three of cell structures 585. It is important to note that cell sizes that are multiples of one another are not required. As with cell structures 553, 554 and 555 in device 550 described above, cell structures 585, 586 and 587 each have an average length to width ratio greater than one when the retrieval device 580 is in an unexpanded state and when the retrieval device 580 is in an expanded state. As discussed above, the ability of the cell structures to maintain an average length to width ratio of greater than one inhibits the cells from collapsing lengthwise as the retrieval device travels through a delivery catheter or duet of a patient. In other words, the cell structures of device 580 are inhibited from collapsing lengthwise on themselves in an accordion like fashion due to their length to width ratios being greater than one.

Another aspect is reflected in the length of the cell structures 587 in the distal portion 584 of the device. Because the distal portion 584 assumes an expanded diameter that is greater than the expanded diameter of the remaining portions of the device, the length dimension of cell structure 587 is selected to be sufficiently long in comparison to its width dimension so as to ensure that the cell structure maintains its length dimension to be greater than its width dimensions when the retrieval device 580 transitions from an unexpanded to an expanded state.

In some implementations the retrieval device 580 is constructed in a manner that results in a radial force being exerted by the main body portion 583 that is greater than the radial force exerted by the distal portion 584 when the retrieval device 580 is deployed within a duct of a patient. In such an implementation, the main body portion 583 is situated to capture an obstruction while the distal portion 584 more gently acts against a wall of the duct distal to the obstruction to entrap portions of the obstruction that become dislodged during and after its capture. As such, according to one method the retrieval device 580 is placed at the treatment site of a patient by use of a delivery catheter, as previously disclosed herein. The retrieval device 580 is positioned at a distal end of the delivery catheter so that the main body portion 583 is positioned at the site of the obstruction to be retrieved. When sheathed within the delivery catheter the main body portion 583 and the distal portion 584 have the same, or substantially the same, diameter. Thereafter, the delivery catheter is withdrawn proximally to cause the constrained retrieval device to expand at the treatment site so that the main body portion 583 is at least partially forced into the obstruction and so that at least a portion of the distal portion 584 more gently rests against the duct wall distal to the obstruction. Upon the obstruction being captured within the main body portion 583 of device 580, the device may be removed from the patient in a manner consistent with one or more of the methods previously disclosed herein. During such removal, as the retrieval device is pulled proximally the distal portion 584 may sweep along the duct wall to entrap portions of the obstruction that may have become dislodged. By virtue of its enhanced diametric dimension, the distal portion 584 maintains contact with the duet wall during all or a portion of the removal procedure.

Although not shown in FIG. 53, in some implementations the cell structures in the distal segment 584 have wire windings selectively woven through their struts in order to endow the distal segment with a desired radiopacity and/or stiffness as discussed above in relation to FIG. 50.

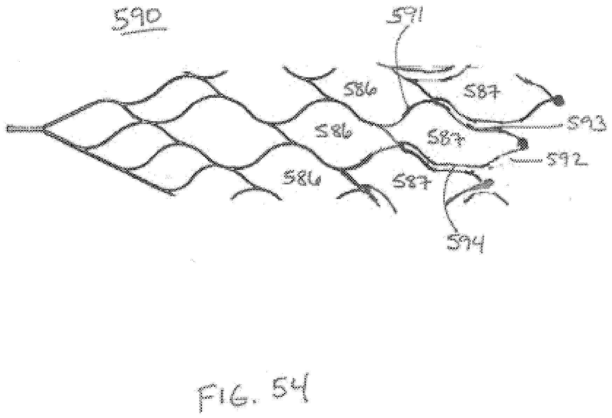

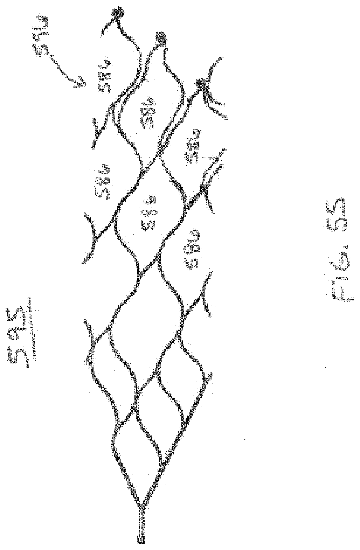

The retrieval device 590 illustrated in FIG. 54 is similar to device 580 with a difference being the manner in which the cell structures 587 are interconnected. Cell structures 587 have a proximal side 591, a distal side 592, a top side 593 and a bottom side 594. As shown in FIG. 54, cell structures 587 are coupled with the adjoining cell structures 586 along at least a portion of the proximal side 591. However, the top and bottom sides 593 and 594 of cell structures 587 are unattached. As mentioned above, it is desirable that the length to width ratio of cell structures 587 remain greater than one when the retrieval device 590 is moved through a delivery catheter or duct of a patient. By de-coupling the top and bottom sides of the cell structures, forces that would normally be applied to the cell structures during expansion to cause them to appreciably expand in width are largely removed. This assists in ensuring that the length to width ratio of cell structures 587 remains greater than one when the device 590 assumes an expanded state. As shown in FIG. 55, as a result of the de-coupling of the distal-most circumferential row of cell structures, the selection of smaller sized cell structures is accommodated. For example, as shown in FIG. 55 the distal-most circumferential row of cell structures 596 may comprise cell structures that are the same or similar to proximally situated cell structures 586. It is appreciated, however, that the size and shape of the distal-most circumferential row of cell structures need not mimic those of the proximally situated cell structures.

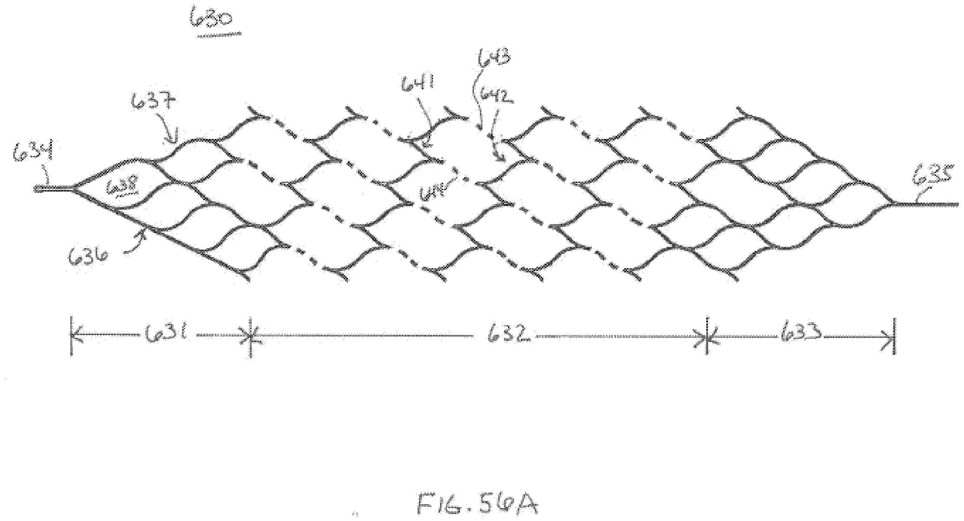

Turning now to FIG. 56A, a retrieval device 630 is shown having a similar construction as the device 800 depicted in FIG. 45A, albeit with fewer cell structures and for use in smaller diameter vessels/ducts. FIG. 56A is a two dimensional view of another example of a retrieval device 630. The retrieval device 630 comprises an expandable member that has a proximal tapered end portion 631, a cylindrical main body portion 632 and a distal tapered end portion 633. The outer-most cell structures in the proximal tapered end portion have outer wall segments that form on one side a non-undulating rail segment 636 and on the other side an undulating rail segment 637. Each of the rail segments 636 and 637 extend from a proximal-most end of the expandable member to a position at or near the proximal end of the cylindrical main body portion 632. A proximal antenna 634 extends proximally from a proximal-most cell structure 638 while a distal antenna 635 extends distally from the distal end of distal tapered section 633. The cell structures 640 in the cylindrical main body portion 632 comprise facing proximal and distal flexure elements 641 and 642, respectively, which generally comprise convex and/or concave like structures, such as for example V-like and U-like structures. The proximal and distal flexure elements 641 and 642 are interconnected by a pair of diagonally extending and circumferentially spaced-apart struts 643 and 644.

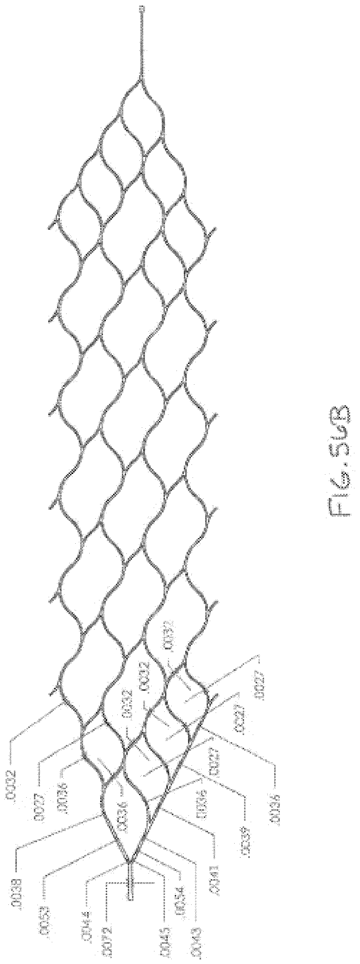

FIG. 56B shows the as-cut width dimensions in inches of the various struts in the proximal tapered end portion 631 according to one implementation, each of the dimensions having a tolerance of ± 0.01016 mm (±0.0004 inches). According to one implementation the struts in the proximal tapered end portion 631 have an as-cut thickness dimension of about 0.1143 mm ± 0.01016 mm (0.0045 ± 0.0004 inches).

As previously discussed, it is desirable that the cylindrical main body portion 632 possess sufficient radial strength to cause at least a partial integration of its struts into an obstruction targeted for full or partial removal. However, the radial strength of the cylindrical main body portion 632 must be sufficiently low to avoid undue damage to the vessel or duct under treatment, in order to achieve a desired radial strength the cross-sectional area and/or width and/or thickness of the struts that form the cylindrical main body portion 632 must be properly dimensioned.

Another feature of consideration is that of flexibility. The cylindrical main body portion 632 should possess sufficient flexibility to permit the retrieval device 630 to be advanced and retracted though the tortuous anatomy of a patient. However, the cylindrical main body portion 632 should also possess sufficient stiffness to permit it to be pushed through a delivery catheter and a duct of a patient without it collapsing on itself. It has been discovered that stiffness also plays a factor in the ability of the retrieval device to be withdrawn into a delivery catheter at a point in time after it has been deployed. As discussed above, upon a misplacement of the retrieval device within a duct of a patient, and sometimes upon removal of the retrieval device from the patient, the retrieval device is fully or partially withdrawn back into the delivery catheter. It has been discovered that in the absence of a requisite amount of stiffness within the cylindrical main body portion it is difficult to withdraw the retrieval device back into the delivery catheter after it has been deployed. Tests have shown that in some situations when all the struts within the cylindrical main body portion have a uniform cross-section, insufficient stiffness results when the struts are sized to achieve a proper amount of radial force.

It has been discovered that the cross-section of flexure elements 641 and 642 most significantly impact radial force in the cylindrical main body portion 632 while the cross-section of diagonally disposed struts 643 and 644 contributing little if any to the radial force produced within the cylindrical main body portion 632. According to some implementations, in order to achieve the right, combination of radial strength and stiffness within the cylindrical main body portion 632, the cross-section of flexure elements 641 and 642 is different, than the cross-section of struts 643 and 644. Because in many instances the retrieval device is cut from a tube of uniform thickness, the width dimensions of the flexure elements 641, 642 and diagonally disposed struts 643, 644 are varied to achieve the desired radial force and stiffness characteristics. However, it is to be appreciated that dimensions other than width may be varied to achieve the same or similar results.

According to some implementations all or substantially all of the struts in the cylindrical main body portion 632 are of the same thickness with the flexure elements 641 and 642 having a first average width dimension and the diagonally disposed struts 643 and 644 having a second average width dimension that is greater than the first average width dimension. The second average width dimension is sufficiently large to compensate for the lack of stiffness that would otherwise exists if the second average width dimension was the same as the first average width dimension. In some implementations the as-cut second average width dimension is in the range of about 1.1 to about 2.0 times greater than the as-cut first average width dimension. In other implementations the as-cut second average width dimension is in the range of about 1.2 to about 1.5 times greater than the as-cut first average width dimension. According to one experiment the as-cut first average width dimension was about 0.08128 mm (0.0032 inches) and the as-cut second average width dimension was about 0.1016 mm (0.0040 inches). The results showed that the average deflection stiffness of the cylindrical main body portion 632 increased by about a 40% to 50% as a result of increasing the width dimension of the diagonally disposed struts 643 and 644 from 0.08128 mm (0.0032 inches) to 0.1016 mm (0.0040 inches). This occurs without an appreciable increase in radial force.

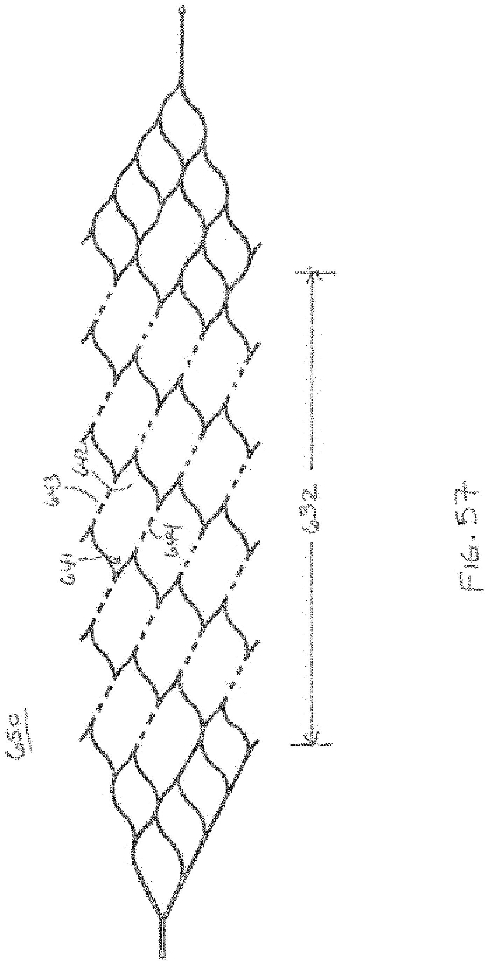

FIG. 57 is illustrates a retrieval device 650 similar to retrieval device 630 with a difference being in the construction of the diagonally disposed struts 643 and 644. As shown in the figures, struts 643 and 644 in retrieval device 630 are curvilinear while struts 643 and 644 in retrieval device 650 are straight.

According to some implementations the width dimensions of the diagonally disposed struts 643 and 644 are uniform along their length. With respect to the example dimensions above, it would mean that the entire length of struts 643 and 644 would have an average width dimension of 0.1016 mm (0.0040 inches). In other implementations struts 643 and 644 comprise middle segments that are disposed between opposite proximal and distal end segments, with the proximal end segments being coupled to the proximal flexure element and the distal end segments being coupled to the distal flexure element. Using the same example dimensions above, the flexure elements 641 and 642 along with the proximal and distal segments of struts 643 and 644 may have an average width dimension of 0.08128 mm (0.0032 inches) while the middle segment of struts 643 and 644 may have an average width dimension of 0.1016 mm (0.0040 inches). In other implementations struts 643 and 644 comprise middle segments that are disposed between opposite proximal and distal tapered end segments, with the proximal tapered end segments being coupled to the proximal flexure element 641 and the distal tapered end segments being coupled to the distal flexure element 642. Using the same example dimensions above, the flexure elements 643 and 642 may have an average width dimension of 0.08128 mm (0.0032 inches) with the middle segment of struts 643 and 644 having an average width dimension of 0.1016 mm (0.0040 inches), the average width dimension of the proximal and distal tapered end segments transitioning from 0.08128 mm (0.0032 inches) to 0.1016 mm (0.0040 inches).

As disclosed above in the description of the device depicted in FIG. 50, a process of weaving/winding a wire or ribbon about the struts of the retrieval device may be used for the purpose of enhancing the radiopacity of the device and also for affecting the stiffness of the device, in the implementations disclosed above, the discussion was limited to incorporating such a feature into the distal segment of retrieval devices. What follows is a description that involves the use of such wire windings in other portions of the device.

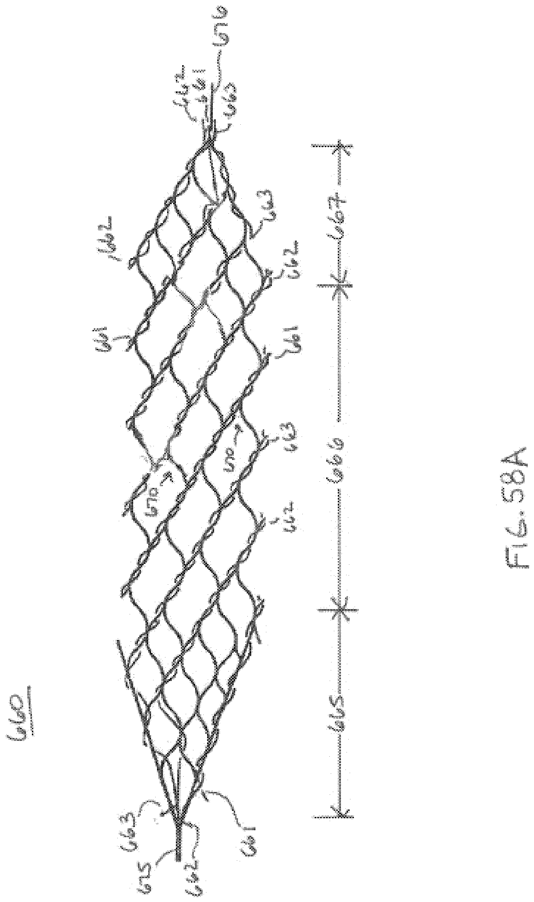

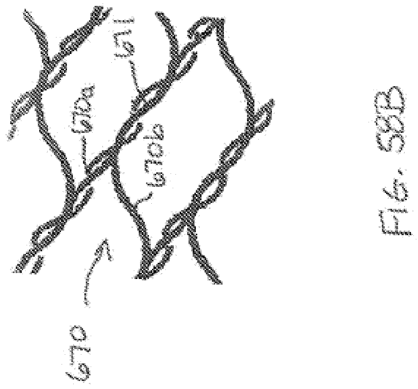

FIG. 58A illustrates a retrieval device 630 that has a similar construction to the retrieval device 650 shown in FIG. 56. The retrieval device 660 comprises wires that are wound about selective struts for the purpose of enhancing the radiopacity of the device 660 and/or to affect the stiffness of one or more portions of the device. In the exemplary implementation of FIG. 58A three radiopaque wires (or ribbons) 661, 662 and 663 are woven along the length of the retrieval device to enhance the radiopacity of the device along essentially its entire length and to enhance the stiffness of at least the cylindrical main body portion 666. In the implementation of FIG. 58A. the wires 661-663 are woven about the diagonally downward oriented struts (as viewed from left to right) in the cylindrical main body portion 666 so that only the short legs 670a of the flexure elements 670 possess wire windings. As shown in detail in FIG. 58B, the long legs 670b of the flexure elements 670 are free or substantially free of wire windings. A virtue of this winding configuration is that it enables the wires to be applied to the retrieval device, and particularly the cylindrical main body portion 666, in a manner that disproportionally affects stiffness and radial force. For example, the average deflection stiffness of the cylindrical main body portion 666 may be moderately to significantly increased without there being a corresponding increase in the radial force exerted by the cylindrical main body portion when it is in an unexpanded state. Prototypes have shown that the average deflection stiffness of the cylindrical main body portion 666 may be increased by up to 50% with there being relatively little to no increase in the radial force.

According to one exemplary implementation, the struts in the cylindrical main body portion 666 have an as-cut thickness dimension of about 0.1143 mm (0.0045 inches) with each of struts 670a, 670b and 671 having width dimensions of about 0.08128 mm (0.0032 inches), 0.08128 mm (0.0032 inches) and 0.1016 mm (0.0040 inches), respectively. In one implementation the wires comprise platinum with a width and/ or diameter of between about 0,0508 mm (0.0020 inches) and 0,0635 mm (0.0025 inches) with there being an average of about one to ten windings per strut, and most generally one to five windings per strut. It is to be appreciated that a single wire or any multiple thereof may be used in lieu of the three wire configurations depicted in FIG. 58A. Moreover, it is important to note that, in the case of enhancing radiopacity that the wire or wires may comprise any radiopaque material or combination of materials as discussed above in conjunction with FIG. 50. In the event that wire windings are applied only for the purpose of affecting stiffness, the wire or wires may comprise any material suitable for such purpose, such as for example metallic, polymeric and composite materials. In some implementations the cross-sectional area of the one or more wires varies to provide a variation in radiopacity and/or stiffness along the length of the device.

According to some implementations, the cylindrical main body portion 666 has a first average deflection stiffness in the absence of the one or more wires or ribbons 661-663 and a second average deflection stiffness with presence of the one or more wires or ribbons 661-663, the dimensional and material characteristics of the one or more wires or ribbons 661-663 and the number of windings per unit length of the diagonally extending and circumferentially spaced-apart struts in the cylindrical main body portion selected to cause the second average deflection stiffness to be greater than the first average deflection stiffness by a factor of between about 1.2 to about 1.8 with there being a disproportionally lower increase in the radial force exerted by the cylindrical main body portion when it is in an unexpanded state.

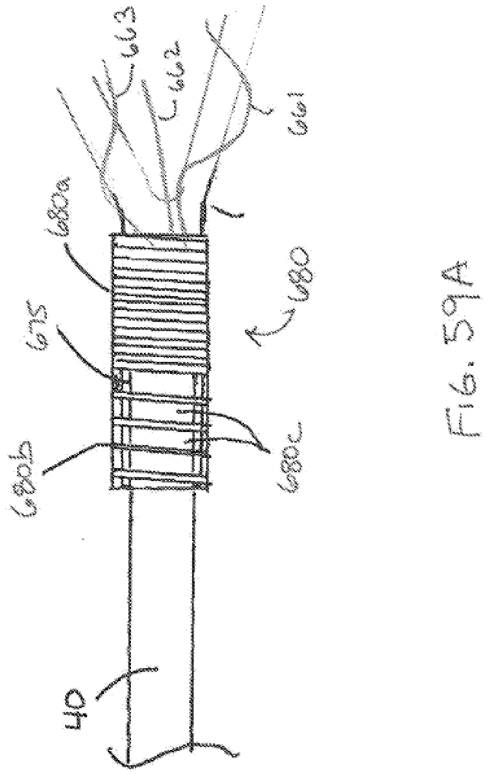

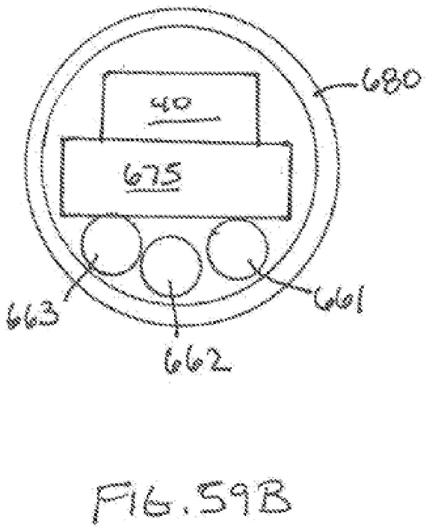

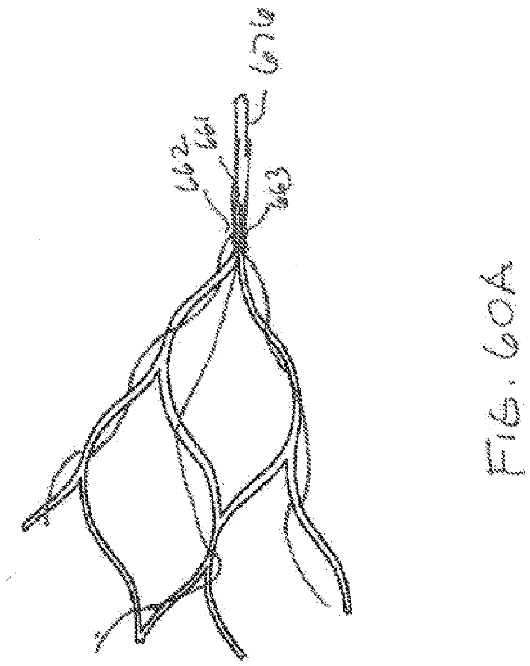

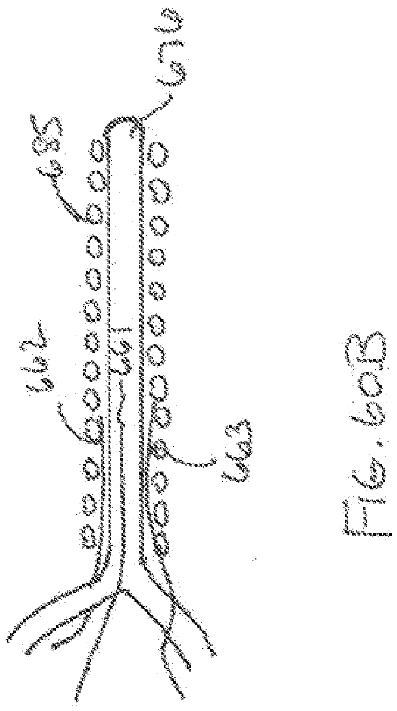

According to some implementations the proximal and distal end segments of wires 661-663 are coupled to the retriever device 660 as depicted in FIG. 59 and FIG. 60, respectively. It is important to note that other attachment/coupling configurations are also possible. FIG. 59A illustrate the proximal attachment of wires 661-663 at the location where the distal end of elongate wire 40 (see for example FIG. 1 A) is attached to the proximal antenna 675. In one embodiment, the distal end of wire 40 has a flat profile with a width of about 0.127 mm (0.005 inches) with the width and thickness of the proximal antenna 675 being about 0.16 mm (0,0063 inches) and about 0,0889 mm (0,0035 inches), respectively. FIG. 59B illustrates a cross-sectional view of the resulting joint where in one implementation the proximal ends of wires 661-663 reside on a bottom side of proximal antenna 675 and the distal end of elongate wire 40 resides on a top side of proximal antenna 675.

In one implementation, the distal end of elongate wire 40, the proximal ends of wires 661- 663 and the proximal antenna 675 are coupled together within a coil structure 680. In one implementation the coil structure 680 has a closely wrapped distal segment 680a, and a loosely wrapped proximal segment 680b that includes one or more gaps 680c. In one implementation, the length of proximal antenna 675 and the coil 680 are substantially equal. Upon the coil 680 being placed over the overlapping components a bonding agent is introduced into the internal cavity of the coil 680 to bond the elongate wire 40, proximal antenna 675 and wires 661-663 together with at least a portion of the coil. In one implementation, the end segments of wires 661-663 reside entirely within distal coil segment 680a. The bonding agent may be an adhesive, solder, or any other suitable bonding agent. When the bonding agent is a solder, a preceding step in the process may involve coating the various components with tin or another suitable wetting agent. In one implementation the solder is gold and is used to enhance the radiopacity of the joint so that the joint may serve as a proximal radiopaque marker. This implementation is particularly applicable in situations where wires 661-663 are non-radiopaque. In addition to the use of gold, all or portions of the coil may be made of a radiopaque material to further enhance the radiopacity of the joint. In other implementations, in lieu of the use of a single coil 680, two or more coils in abutting relationship are used with, for example, a distal closely wound coil and a proximal loosely wound coil with gaps situated proximal to the closely wound coil.

FIGS. 60A and 60B illustrate several methods by which the distal end segments of wires 661-663 may be attached to the distal antenna 676. In the implementation of FIG. 60A the distal ends of wires 661-663 are bonded directly to the distal antenna by use of a bonding agent such as solder or glue. FIG. 60B shows another implementation where the distal ends of wires 661-663 are interposed between the distal antenna 676 and a coil 685 that surrounds it. In such an implementation a bonding agent may be introduced into the interior of the coil 685 to effectuate a bonding together the coil 685, distal antenna 676 and wires 661-663.

While the above description contains many specifics, those specifics should not be construed as limitations on the scope of the disclosure, but merely as exemplifications of preferred embodiments thereof. For example, dimensions other than those listed above are contemplated. For example, retrieval devices having expanded diameters of anywhere between 1.0 and 100.0 millimeters and lengths of up to 5.0 to 10.0 centimeters are contemplated. Moreover, it is appreciated that many of the features disclosed herein are interchangeable among the various implementations. Those skilled in the art will envision many other possible variations that are within the scope of the disclosure. Further, it is to be appreciated that the delivery of a vascular treatment device of the implementations disclosed herein is achievable with the use of a catheter, a sheath or any other device that is capable of carrying the device with the expandable member in a compressed state to the treatment site and which permits the subsequent deployment of the expandable member at a vascular treatment site. The vascular treatment site may be (1) at the neck of an aneurysm for diverting flow and/or facilitating the placement of coils or other like structures within the sack of an aneurysm, (2) at the site of an embolic obstruction with a purpose of removing the embolic obstruction, (3) at the site of a stenosis with a purpose of dilating the stenosis to increase blood flow through the vascular, etc.

## Claims

1. An embolic obstruction retrieval device (660) comprising; an elongate self-expandable member (12, 112) having a radially expanded configuration and a radially unexpanded configuration,
the elongate self-expandable member (12, 112) comprising a plurality of generally longitudinal undulating elements (24) with adjacent undulating elements being interconnected in a manner to form a plurality of diagonally disposed cell structures (26),
the elongate self-expandable member (12, 112) having a proximal antenna (675), a proximal end portion (665) and a cylindrical main body portion (666) comprising a proximal section and a distal section,
the cell structures (26) in the cylindrical main body portion (666) extending circumferentially around a longitudinal axis of the elongate self-expandable member (12, 112),
the cell structures (26) in the proximal end portion (665) extending less than circumferentially around the longitudinal axis of the elongate self-expandable member (12, 112),
the cell structures (26) in the cylindrical main body portion (666) comprising proximal and distal flexure elements (670) that are interconnected by a pair of diagonally extending and circumferentially spaced-apart struts (670a, 670b, 671)
the proximal and distal flexure elements (670) each having a short leg (670a) and a long leg (670b), **characterized in that**
at least some of the diagonally extending and circumferentially spaced-apart struts (670a, 670b, 671) having one or more wires or ribbons (661, 662, 663) wound thereabout so as to enhance the average deflection stiffness of all or a portion of the cylindrical main body portion (666) when the elongate self-expandable member is in the radially expanded configuration,
the long legs (670b) of the proximal and distal flexure elements (670) being free or substantially free of the wires or ribbons (661, 662, 663) wound thereabout.

2. The embolic obstruction retrieval device (660) of claim 1, wherein the one or more wires or ribbons (661, 662, 663) comprise a radiopaque material.

3. The embolic obstruction retrieval device (660) of claim 1, wherein the one or more wires or ribbons (661, 662, 663) comprise a metallic material.

4. The embolic obstruction retrieval device (660) of claim 1, wherein the one or more wires or ribbons (661, 662, 663) comprise a polymeric material.

5. The embolic obstruction retrieval device (660) of claim 1, wherein in the radially expanded configuration the cylindrical main body portion (666) has a first average deflection stiffness in the absence of the one or more wires or ribbons (661, 662, 663) and a second average deflection stiffness with presence of the one or more wires or ribbons (661, 662, 663), the dimensional and material characteristics of the one or more wires or ribbons (661, 662, 663) and the number of windings per unit length of the diagonally extending and circumferentially spaced-apart struts (670a, 670b, 671) selected to cause the second average deflection stiffness to be greater than the first average deflection stiffness by a factor of between 1.2 to 1.8.

6. The embolic obstruction retrieval device (660) of claim 1, wherein at least some of the diagonally extending and circumferentially spaced-apart struts (670a, 670b, 671) comprise one or more winding guide features.

7. The embolic obstruction retrieval device (660) of claim 6, wherein the one or more winding guide features comprise recesses capable of receiving only a portion of the one or more wires or ribbons (661, 662, 663).

8. The embolic obstruction retrieval device (660) of claim 1, further comprising a first proximally extending elongate flexible wire connected to the proximal antenna (675).

9. The embolic obstruction retrieval device (660) of claim 8, wherein an end of the one or more wires or ribbons (661, 662, 663) is connected to the proximal antenna (675) at the connection location of the first proximally extending elongate flexible wire with the proximal antenna (675).

10. The embolic obstruction retrieval device (660) of claim 1, wherein in the radially expanded configuration the cylindrical main body portion (666) has a first average deflection stiffness in the absence of the one or more wires or ribbons (661, 662, 663) and a second average deflection stiffness with the presence of the one or more wires or ribbons (661, 662, 663),
the dimensional and material characteristics of the one or more wires or ribbons (661, 662, 663) and the number of windings per unit length of the diagonally extending and circumferentially spaced-apart struts (670a, 670b, 671) selected to cause the second average deflection stiffness to be greater than the first average deflection stiffness by a first multiplication factor,
and wherein in the radially unexpanded configuration the cylindrical main body portion (666) exerts a first radial force in the absence of the one or more wires or ribbons (661, 662, 663) and a second radial force with presence of the one or more wires or ribbons (661, 662, 663),
the dimensional and material characteristics of the one or more wires or ribbons (661, 662, 663) and the number of windings per unit length of the diagonally extending and circumferentially spaced-apart struts (670a, 670b, 671) selected to cause the second radial force to be greater than the first radial force by a second multiplication factor, the second multiplication factor being less than the first multiplication factor.

## Patentansprüche

1. Eine Vorrichtung zur Beseitigung von einem embolischen Verschluss (660) umfassend; ein langgestrecktes selbstexpandierbares Element (12, 112), das eine radial ausgedehnte Konfiguration und eine radial nicht ausgedehnte Konfiguration aufweist,
wobei das langgestreckte selbstexpandierbare Element (12, 112) eine Vielzahl von im Allgemeinen längsgerichteten welligen Elementen (24) mit angrenzenden welligen Elementen umfasst, die so miteinander verbunden sind, dass sie eine Vielzahl von schräg angeordneten Zellstrukturen (26) bilden,
wobei das langgestreckte selbstexpandierbare Element (12, 112) eine proximale Antenne (675), einen proximalen Endteil (665) und einen zylindrischen Hauptkörperteil (666) umfassend einen proximalen Abschnitt und einen distalen Abschnitt hat,
wobei sich die Zellstrukturen (26) im zylindrischen Hauptkörperteil (666) umlaufend um eine Längsachse des langgestreckten selbstexpandierbaren Elements (12, 112) herum erstrecken,
wobei sich die Zellstrukturen (26) im proximalen Endteil (665) weniger als umlaufend um die Längsachse des langgestreckten selbstexpandierbaren Elements (12, 112) herum erstrecken,
wobei die Zellstrukturen (26) im zylindrischen Hauptkörperteil (666) proximale und distale Biegeelemente (670) umfassen, die durch ein Paar von sich schräg erstreckenden und umlaufend räumlich getrennten Streben (670a, 670b, 671) miteinander verbunden sind
wobei die proximalen und distalen Biegeelemente (670) jeweils einen kurzen Zweig (670a) und einen langen Zweig (670b) haben, **dadurch gekennzeichnet, dass**
mindestens manche der sich schräg erstreckenden und umlaufend räumlich getrennten Streben (670a, 670b, 671) einen oder mehrere daherum gewickelte Fäden oder Bändeln (661, 662, 663) haben, um die durchschnittliche Biegesteifheit von dem gesamten oder von einem Teil des zylindrischen Hauptkörperteils (666) zu erhöhen, wenn das langgestreckte selbstexpandierbare Element in der radial ausgedehnten Konfiguration ist,
wobei die langen Zweige (670b) der proximalen und der distalen Biegeelemente (670) frei oder im Wesentlichen frei von daherum gewickelten Fäden oder Bändeln (661, 662, 663) sind.

2. Die Vorrichtung zur Beseitigung von einem embolischen Verschluss (660) des Anspruchs 1, wobei der eine oder die mehreren Fäden oder Bändel (661, 662, 663) ein röntgendichtes Material umfassen.

3. Die Vorrichtung zur Beseitigung von einem embolischen Verschluss (660) des Anspruchs 1, wobei der eine oder die mehreren Fäden oder Bändel (661, 662, 663) ein metallisches Material umfassen.

4. Die Vorrichtung zur Beseitigung von einem embolischen Verschluss (660) des Anspruchs 1, wobei der eine oder die mehreren Fäden oder Bändel (661, 662, 663) ein polymeres Material umfassen.

5. Die Vorrichtung zur Beseitigung von einem embolischen Verschluss (660) des Anspruchs 1, wobei der zylindrische Hauptkörperteil (666) in der radial ausgedehnten Konfiguration eine erste durchschnittliche Biegesteifheit in Abwesenheit von dem einen oder den mehreren Fäden oder Bändeln (661, 662, 663) und eine zweite durchschnittliche Biegesteifheit bei Anwesenheit von dem einen oder den mehreren Fäden oder Bändeln (661, 662, 663) aufweist, wobei die Größen- und Stoffeigenschaften des einen oder der mehreren Fäden oder Bändel (661, 662, 663) und die Anzahl von Wicklungen pro Längeneinheit der sich schräg erstreckenden und umlaufend räumlich getrennten Streben (670a, 670b, 671) so ausgewählt sind, dass die zweite durchschnittliche Biegesteifheit daher größer als die erste durchschnittliche Biegesteifheit um einen Faktor von zwischen 1,2 bis 1,8 ist.

6. Die Vorrichtung zur Beseitigung von einem embolischen Verschluss (660) des Anspruchs 1, wobei mindestens manche der sich schräg erstreckenden und umlaufend räumlich getrennten Streben (670a, 670b, 671) eine oder mehrere Wicklungsführungseigenschaften umfassen.

7. Die Vorrichtung zur Beseitigung von einem embolischen Verschluss (660) des Anspruchs 6, wobei die eine oder die mehreren Wicklungsführungseigenschaften Vertiefungen umfassen, die nur einen Teil der einen oder der mehreren Fäden oder Bändel (661, 662, 663) empfangen können.

8. Die Vorrichtung zur Beseitigung von einem embolischen Verschluss (660) des Anspruchs 1, weiterhin umfassend einen mit der proximalen Antenne (675) verbundenen ersten sich proximal erstreckenden langgestreckten flexiblen Faden.

9. Die Vorrichtung zur Beseitigung von einem embolischen Verschluss (660) des Anspruchs 8, wobei ein Ende des einen oder der mehreren Fäden oder Bändel (661, 662, 663) mit der proximalen Antenne (675) an der Verbindungsstelle des ersten sich proximal erstreckenden langgestreckten flexiblen Fadens mit der proximalen Antenne (675) verbunden ist.

10. Die Vorrichtung zur Beseitigung von einem embolischen Verschluss (660) des Anspruchs 1, wobei der zylindrische Hauptkörperteil (666) in der radial ausgedehnten Konfiguration eine erste durchschnittliche Biegesteifheit in Abwesenheit von dem einen oder den mehreren Fäden oder Bändeln (661, 662, 663) und eine zweite durchschnittliche Biegesteifheit bei Anwesenheit von dem einen oder den mehreren Fäden oder Bändeln (661, 662, 663) aufweist,
wobei die Größen- und Stoffeigenschaften des einen oder der mehreren Fäden oder Bändel (661, 662, 663) und die Anzahl von Wicklungen pro Längeneinheit der sich schräg erstreckenden und umlaufen räumlich getrennten Streben (670a, 670b, 671) so ausgewählt sind, dass die zweite durchschnittliche Biegesteifheit daher größer als die erste durchschnittliche Biegesteifheit um einen ersten Multiplikationsfaktor ist,
und wobei der zylindrische Hauptkörperteil (666) in der radial ausgedehnten Konfiguration eine erste radiale Kraft in Abwesenheit von dem einen oder von den mehreren Fäden oder Bändeln (661, 662, 663) und eine zweite radiale Kraft bei Anwesenheit von dem einen oder den mehreren Fäden oder Bändeln (661, 662, 663) ausübt,
wobei die Größen- und Stoffeigenschaften des einen oder der mehreren Fäden oder Bändel (661, 662, 663) und die Anzahl von Wicklungen pro Längeneinheit der sich schräg erstreckenden und umlaufen räumlich getrennten Streben (670a, 670b, 671) so ausgewählt sind, dass die zweite radiale Kraft daher größer als die erste radiale Kraft um einen zweiten Multiplikationsfaktor ist, wobei der zweite Multiplikationsfaktor geringer als der erste Multiplikationsfaktor ist.

## Revendications

1. Un dispositif d'élimination d'obstructions emboliques (660) comprenant ; un élément auto-expansible allongé (12, 112) ayant une configuration radialement étendue et une configuration radialement non étendue,
l'élément auto-expansible allongé (12, 112) comprenant une pluralité d'éléments ondulants généralement longitudinaux (24) avec des éléments ondulants adjacents étant interconnectés de manière à former une pluralité de structures cellulaires disposées de façon diagonale (26),
l'élément auto-expansible allongé (12, 112) ayant une antenne proximale (675), une partie d'extrémité proximale (665) et une partie de corps principal cylindrique (666) comprenant une section proximale et une section distale,
les structures cellulaires (26) dans la partie de corps principale cylindrique (666) s'étendant circonférentiellement autour d'un axe longitudinal de l'élément auto-expansible allongé (12, 112),
les structures cellulaires (26) dans la partie d'extrémité proximale (665) s'étendant moins que circonférentiellement autour de l'axe longitudinal de l'élément auto-expansible allongé (12, 112),
les structures cellulaires (26) dans la partie de corps principal cylindrique (666) comprenant des éléments de flexion proximaux et distaux (670) qui sont interconnectés par une paire d'entretoises s'étendant diagonalement et espacées circonférentiellement (670a, 670b, 671)
les éléments de flexion proximaux et distaux (670) ayant chacun une branche courte (670a) et une branche longue (670b), **caractérise en ce que**
au moins quelques-unes des entretoises s'étendant diagonalement et espacées circonférentiellement (670a, 670b, 671) ont un ou plusieurs fils ou rubans (661, 662, 663) enroulés autour de celles-ci afin d'augmenter la rigidité de flexion moyenne de toute ou d'une partie de la partie de corps principal cylindrique (666) lorsque l'élément auto-expansible allongé est dans la configuration radialement étendue,
les branches longues (670b) des éléments de flexion proximaux et distaux (670) étant livres ou essentiellement livres des fils ou rubans (661, 662, 663) enroulés autour de ceux-ci.

2. Le dispositif d'élimination d'obstructions emboliques (660) de la revendication 1, dans lequel l'un ou les plusieurs fils ou rubans (661, 662, 663) comprennent un matériau radio-opaque.

3. Le dispositif d'élimination d'obstructions emboliques (660) de la revendication 1, dans lequel l'un ou les plusieurs fils ou rubans (661, 662, 663) comprennent un matériau métallique.

4. Le dispositif d'élimination d'obstructions emboliques (660) de la revendication 1, dans lequel l'un ou les plusieurs fils ou rubans (661, 662, 663) comprennent un matériau polymère.

5. Le dispositif d'élimination d'obstructions emboliques (660) de la revendication 1, dans lequel dans la configuration radialement étendue la partie de corps principal cylindrique (666) a une première rigidité de flexion moyenne en l'absence de l'un ou des plusieurs fils ou rubans (661, 662, 663) et une deuxième rigidité de flexion moyenne en présence de l'un ou des plusieurs fils ou rubans (661, 662, 663), les caractéristiques dimensionnelles et de matériau de l'un ou des plusieurs fils ou rubans (661, 662, 663) et le nombre d'enroulements par unité de longueur des entretoises s'étendant diagonalement et espacées circonférentiellement (670a, 670b, 671) étant choisies de façon à faire que la deuxième rigidité de flexion moyenne soit supérieure à la première rigidité de flexion moyenne d'un facteur d'entre 1,2 à 1,8.

6. Le dispositif d'élimination d'obstructions emboliques (660) de la revendication 1, dans lequel au moins quelques-unes des entretoises s'étendant diagonalement et espacées circonférentiellement (670a, 670b, 671) comprennent une ou plusieurs caractéristiques de guidage d'enroulement.

7. Le dispositif d'élimination d'obstructions emboliques (660) de la revendication 6, dans lequel l'une ou les plusieurs caractéristiques de guidage d'enroulement comprennent des renfoncements capables de recevoir seulement une partie de l'un ou des plusieurs fils ou rubans (661, 662, 663).

8. Le dispositif d'élimination d'obstructions emboliques (660) de la revendication 1, comprenant en outre un premier fil flexible allongé s'étendant de façon proximale connecté à l'antenne proximale (675).

9. Le dispositif d'élimination d'obstructions emboliques (660) de la revendication 8, dans lequel une extrémité de l'un ou des plusieurs fils ou rubans (661, 662, 663) est connectée à l'antenne proximale (675) à l'endroit de la connexion du premier fil flexible allongé s'étendant de façon proximale avec l'antenne proximale (675).

10. Le dispositif d'élimination d'obstructions emboliques (660) de la revendication 1, dans lequel dans la configuration radialement étendue la partie de corps principal cylindrique (666) a une première rigidité de flexion moyenne en l'absence de l'un ou des plusieurs fils ou rubans (661, 662, 663) et une deuxième rigidité de flexion moyenne en présence de l'un ou des plusieurs fils ou rubans (661, 662, 663),
les caractéristiques dimensionnelles et de matériau de l'un ou des plusieurs fils ou rubans (661, 662, 663) et le nombre d'enroulements par unité de longueur des entretoises s'étendant diagonalement et espacées circonférentiellement (670a, 670b, 671) étant choisies pour faire que la deuxième rigidité de flexion moyenne soit supérieure à la première rigidité de flexion moyenne d'un premier facteur de multiplication,
et dans lequel dans la configuration radialement non étendue la partie de corps principal cylindrique (666) exerce une première force radiale en l'absence de l'un ou des plusieurs fils ou rubans (661, 662, 663) et une deuxième force radiale en présence de l'un ou des plusieurs fils ou rubans (661, 662, 663),
les caractéristiques dimensionnelles et de matériau de l'un ou des plusieurs fils ou rubans (661, 662, 663) et le nombre d'enroulements par unité de longueur des entretoises s'étendant diagonalement et espacées circonférentiellement (670a, 670b, 671) étant choisies pour faire que la deuxième force radiale soit supérieure à la première force radiale d'un deuxième facteur de multiplication, le deuxième facteur de multiplication étant inférieur au premier facteur de multiplication.
